# EUROPEAN PATENT SPECIFICATION

(11) **EP 3 274 400 B1**
(45) Date of publication and mention of the grant of the patent: **01.05.2024**
(21) Application number: 16716966.3
(22) Date of filing: 17.03.2016
(51) Int. Cl.: C08J 7/06, B29C 71/02, B29C 71/04, C08J 7/12, C08K 5/00, C08L 23/04, A61L 27/16, A61L 27/50, C08J 3/20, C08J 3/28, C08K 5/529, C08K 5/1545, B29K 23/00, B29C 35/08

(54) **MELT-STABILIZED ULTRA HIGH MOLECULAR WEIGHT POLYETHYLENE**
SCHMELZSTABILISIERTES ULTRAHOCHMOLEKULARES POLYETHYLEN
POLYÉTHYLÈNE À ULTRA HAUT POIDS MOLÉCULAIRE STABILISÉ À L'ÉTAT FONDU

(30) Priority: 25.03.2015 US 201562138081 P
(43) Date of publication of application: 31.01.2018
(73) Proprietor: Zimmer, Inc., Warsaw, Indiana 46580 (US)
(72) Inventor: PLETCHER, Dirk, Walkerton, IN 46574 (US)
(74) Representative: Venner Shipley LLP
(86) International application number: PCT/US2016/022896
(87) International publication number: WO 2016/153925

(56) References cited:
- EP-A1- 2 578 248
- EP-A1- 3 227 360
- EP-A2- 2 395 048
- WO-A1-2012/061499
- WO-A2-2004/064618
- WO-A2-2005/074619
- WO-A2-2009/060043
- US-A1- 2008 215 142

## Description

### CROSS-REFERENCE TO RELATED APPLICATION

### BACKGROUND

Ultra high molecular weight polyethylene (UHMWPE) is a unique form of polyethylene of extremely high molecular weight, where the molecular weight of commercial grade materials are typically in the range of 2 to 7 million. The molecular weight of commodity polyethylene is typically in the range of 50,000 to 100,000, a factor of 25 or more times lower. UHMWPE is the most widely used material for orthopedic implants that articulate, such as for hip, knee, ankle, elbow and shoulder joint replacement due to osteoarthritis. First implemented in the early 1960's, a major concern for this material has been high wear rate with generation of microscopic wear particles over years of articulation. A known outcome of a high polyethylene particulate burden is a condition known as osteolysis, which results in implant loosening with subsequent need for revision surgery. This concern was addressed in the late 1990's with the introduction of highly crosslinked UHMWPE, which is crosslinked by the use of high energy irradiation such as gamma or electron beam. Crosslinking reduces the wear rate of UHMWPE significantly, but also leaves a high free radical burden in the polyethylene which, if not reduced, can cause oxidation in-vivo, with subsequent reduction in mechanical properties, increasing wear rates, and potential implant failure.

To address the free radical burden, highly crosslinked UHMWPE is most often heat stabilized by raising the material temperature above the melting point of the material. This allows the trapped free radicals that did not participate in crosslinking to promote further crosslinking in the material, or to re-combine, rendering them to an inert state that will not promote premature oxidative degradation. However, the melting process can cause the formation of a significant oxidized layer on the exterior of the material if the melting process is done in an oxygen-containing environment such as air, where sufficient oxygen is present to diffuse into the material in the molten state. This oxidized layer is removed during fabrication of the implant to prevent contamination of the implant with oxidatively-degraded UHMWPE.
WO2005074619A2 relates to methods for making highly crystalline cross-linked polymeric material, for example, highly crystalline cross-linked ultra-high molecular weight polyethylene (UHMWPE).
US2008215142A1 relates to methods for making cross-linked, oxidatively stable, and highly crystalline polymeric materials.
EP2578248A1 relates to an antioxidant combined with UHMWPE prior to subjecting the UHMWPE to crosslinking irradiation.
WO2012061499A1 relates to modified polymeric articles having modifying agents dispersed within and bonded to an interior region of the article and methods of modifying polymer materials used to form polymeric articles, and methods of making polymeric articles from polymer particles having modifying agents bonded thereto.
WO2004064618A2 relates to methods for making oxidation resistant medical devices that comprise polymeric materials, for example, ultra-high molecular weight polyethylene (UHMWPE).
EP2395048A2 is directed to polymer compositions including a protected antioxidant. The compositions can also include a deprotected antioxidant, an unprotected antioxidant or both. Methods of producing compositions including a protected antioxidant and articles including a protected antioxidant are also described.
WO2009060043A2 relates to a process for producing an (ultra) high molecular weight polyethylene (HMWPE) article comprising: - incorporating into the HMWPE resin a Hindered Amine Light Stabilizer (HALS) and - cross-link the (U)HMWPE during or after molding the (U)HMWPE resin.

### SUMMARY OF THE INVENTION

The invention is as defined in the claims. The present disclosure provides a method of melt-stabilizing ultra high molecular weight polyethylene (UHMWPE). The method includes coating a solid material including UHMWPE with an antioxidant, to provide an antioxidant-coated solid material. The method includes pre-irradiatively heating the antioxidant-coated solid material to diffuse the antioxidant therein, to provide an antioxidant-diffused solid material. The method includes irradiating the antioxidant-diffused solid material, to provide an irradiated solid material. The method includes post-irradiatively heating the irradiated solid material, the heating sufficient to melt at least part of the UHMWPE, to provide a heated material. The method includes solidifying the heated material, to provide a melt-stabilized material.

In various embodiments, the present invention provides a method of melt-stabilizing ultra high molecular weight polyethylene (UHMWPE). The method includes coating a solid material including UHMWPE with a protected vitamin E antioxidant, to provide an antioxidant-coated solid material. The protected vitamin E antioxidant is at least one of at least one of (a) and (b). Protected vitamin E antioxidant (a) is a protected tocopherol or tocotrienol having the structure: 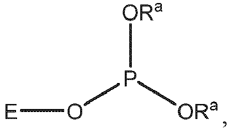 or a salt thereof, or 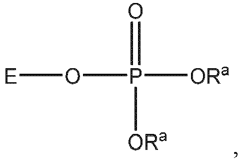 or a salt thereof.

At each occurrence, R^{a} is independently chosen from -H, -E, and substituted or unsubstituted (C₁-C₁₀)hydrocarbyl. The variable E has the structure: 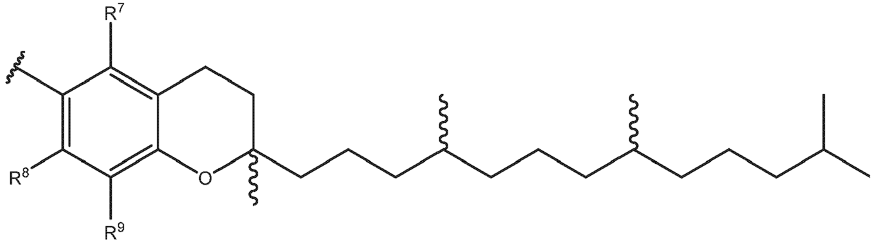 or

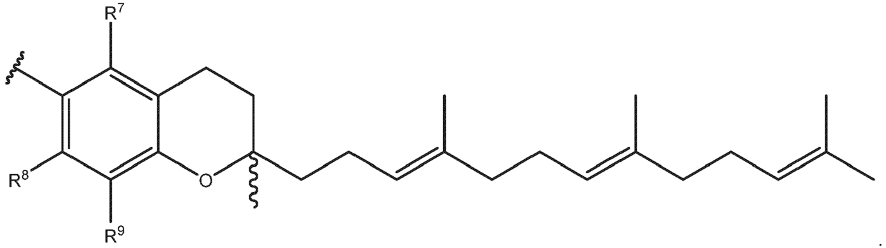

The variables R⁷, R⁸, and R⁹ are each independently chosen from -H, substituted or unsubstituted (C₁-C₁₀)alkyl, and substituted or unsubstituted (C₁-C₁₀)alkenyl. Protected vitamin E antioxidant (b) is a hindered amine stabilizer-protected tocopherol or tocotrienol of formula (1): 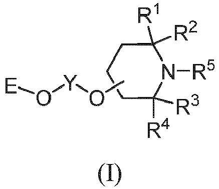 or a salt thereof. The variables R¹, R², R³, and R⁴ are each, independently, hydrogen or (Ci-C₁₀)alkyl. The variable R⁵ is chosen from hydrogen, (C₁-C₁₀)alkyl, -O·, and -OR¹¹ wherein R¹¹ is hydrogen or (C₁-C₁₀)alkyl. The variable Y represents the group:

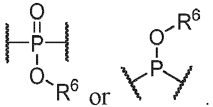

The variable R⁶ is hydrogen, (C₁-C₁₀)alkyl, -E, or a radical of the formula:

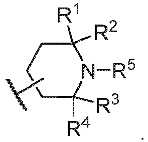

The method includes pre-irradiatively heating the antioxidant-coated solid material to diffuse the antioxidant therein, to provide an antioxidant-diffused solid material. The method includes irradiating the antioxidant-diffused solid material, to provide an irradiated solid material. The method includes post-irradiatively heating the irradiated solid material, the heating sufficient to melt at least part of the UHMWPE, to provide a heated material. The method also includes solidifying the heated material, to provide a melt-stabilized material.

A melt-stabilized ultra high molecular weight polyethylene (UHMWPE) material made by a method including coating a solid material including UHMWPE with an antioxidant, to provide an antioxidant-coated solid material is disclosed. The method includes pre-irradiatively heating the antioxidant-coated solid material to diffuse the antioxidant therein, to provide an antioxidant-diffused solid material. The method includes irradiating the antioxidant-diffused solid material, to provide an irradiated solid material. The method includes post-irradiatively heating the irradiated solid material, the heating sufficient to melt at least part of the UHMWPE, to provide a heated material. The method also includes solidifying the heated material, to provide the melt-stabilized material.

Various embodiments of the present invention provide certain advantages over other melt-stabilized ultra high molecular weight polyethylenes and methods of making the same. In some embodiments, the method can include forming a UHMWPE material having less or no formation of an oxidized layer on the surface of the UHMWPE. Medical-grade UHMWPE can represent a significant cost in the production of a medical implant including UHMWPE. Oxidation of the surface of UHMWPE during various steps, such as during melt-stabilization (e.g., melting after irradiation), results in the removal and discarding of the oxidized layer due to unsuitability for medical-implant preparation. In some embodiments, as compared to other techniques for preparing UHMWPE materials, the method can form a UHMWPE material that is ready to form into a medical implant with less or no removal of a surface layer. In various embodiments, by avoiding or decreasing removal of an oxidized surface layer of UHWMPE, the method provides cost savings over other methods by decreasing the amount of UHMWPE that is wasted. In some embodiments, the method can avoid formation of a surface oxidation layer even with melt-stabilization in an oxygen-containing atmosphere (e.g., air). In various embodiments, as compared to techniques using an oxygen-free or oxygen-depleted environment for melt-stabilization, the method provides costs savings by avoiding equipment, supplies, and time-consuming techniques needed for generating an oxygen-free or oxygen-depleted environment. In various embodiments, performing the method using a protected antioxidant provides a melt-stabilized crosslinked UHMWPE with a higher crosslinking density than other UHMWPEs with pre-irradiative addition of antioxidant, but having similar or greater post-irradiative resistance to oxidation and corresponding degradation.

In various embodiments, the addition and diffusion of the antioxidant can be more convenient and can reduce processing costs as well as reducing costs related to oxidation. For example, in various embodiments, diffusion of the antioxidant can be conducting during a consolidation process *in situ,* such as for semi-continuous processes such as ram extrusion or continuous processes such as extrusion. In various embodiments, the antioxidant can be applied to the material after consolidation of powder in an external layer.

In various embodiments, in addition to providing oxidative resistance to the implant, diffusion of the antioxidant in an external layer of material can retard or lower infusion of lipids *in vivo* that may promote oxidation of UHMWPE.

In various embodiments, the method can include targeting antioxidants that can retard crosslinking to regions of an implant that require higher mechanical property retention and that may not require the higher wear resistance provided by a higher crosslinking density. Articulation can be lower or minimal in a region not requiring high wear resistance; for example, the rim of a hip implant liner can be subjected to low wear rates but is subjected to oxidative stresses and can require high mechanical property retention.

### DETAILED DESCRIPTION OF THE INVENTION

Reference will now be made in detail to certain embodiments of the disclosed subject matter. While the disclosed subject matter will be described in conjunction with the enumerated claims, it will be understood that the exemplified subject matter is not intended to limit the claims to the disclosed subject matter.

Throughout this document, values expressed in a range format should be interpreted in a flexible manner to include not only the numerical values explicitly recited as the limits of the range, but also to include all the individual numerical values or sub-ranges encompassed within that range as if each numerical value and sub-range is explicitly recited. For example, a range of "about 0.1% to about 5%" or "about 0.1% to 5%" should be interpreted to include not just about 0.1% to about 5%, but also the individual values (e.g., 1%, 2%, 3%, and 4%) and the sub-ranges (e.g., 0.1% to 0.5%, 1.1% to 2.2%, 3.3% to 4.4%) within the indicated range. The statement "about X to Y" has the same meaning as "about X to about Y," unless indicated otherwise. Likewise, the statement "about X, Y, or about Z" has the same meaning as "about X, about Y, or about Z," unless indicated otherwise.

In this document, the terms "a," "an," or "the" are used to include one or more than one unless the context clearly dictates otherwise. The term "or" is used to refer to a nonexclusive "or" unless otherwise indicated. The statement "at least one of A and B" has the same meaning as "A, B, or A and B." In addition, it is to be understood that the phraseology or terminology employed herein, and not otherwise defined, is for the purpose of description only and not of limitation. Any use of section headings is intended to aid reading of the document and is not to be interpreted as limiting; information that is relevant to a section heading may occur within or outside of that particular section. A comma can be used as a delimiter or digit group separator to the left or right of a decimal mark; for example, "0.000,1" is equivalent to "0.0001."

In the methods described herein, the acts can be carried out in any order without departing from the principles of the invention, except when a temporal or operational sequence is explicitly recited. Furthermore, specified acts can be carried out concurrently unless explicit claim language recites that they be carried out separately. For example, a claimed act of doing X and a claimed act of doing Y can be conducted simultaneously within a single operation, and the resulting process will fall within the literal scope of the claimed process.

The term "about" as used herein can allow for a degree of variability in a value or range, for example, within 1 0%, within 5%, or within 1% of a stated value or of a stated limit of a range, and includes the exact stated value or range.

The term "substantially" as used herein refers to a majority of or mostly, as in at least about 50%, 60%, 70%, 80%, 90%, 95%, 96%, 97%, 98%, 99%, 99.5%, 99.9%, 99.99%, or at least about 99.999% or more, or 100%.

The term "organic group" as used herein refers to any carbon-containing functional group. For example, an oxygen-containing group such as an alkoxy group, aryloxy group, aralkyloxy group, oxo(carbonyl) group, a carboxyl group including a carboxylic acid, carboxylate, and a carboxylate ester; a sulfur-containing group such as an alkyl and aryl sulfide group; and other heteroatom-containing groups. Non-limiting examples of organic groups include OR, OOR, OC(O)N(R)₂, CN, CF₃, OCF₃, R, C(O), methylenedioxy, ethylenedioxy, N(R)₂, SR, SOR, SO₂R, SO₂N(R)₂, SO₃R, C(O)R, C(O)C(O)R, C(O)CH₂C(O)R, C(S)R, C(O)OR, OC(O)R, C(O)N(R)₂, OC(O)N(R)₂, C(S)N(R)₂, (CH₂)₀₋₂N(R)C(O)R, (CH₂)₀₋₂N(R)N(R)₂, N(R)N(R)C(O)R, N(R)N(R)C(O)OR, N(R)N(R)CON(R)₂, N(R)SO₂R, N(R)SO₂N(R)₂, N(R)C(O)OR, N(R)C(O)R, N(R)C(S)R, N(R)C(O)N(R)₂, N(R)C(S)N(R)₂, N(COR)COR, N(OR)R, C(=NH)N(R)₂, C(O)N(OR)R, C(=NOR)R, and substituted or unsubstituted (C₁-C₁₀₀)hydrocarbyl, wherein R can be hydrogen (in examples that include other carbon atoms) or a carbon-based moiety, and wherein the carbon-based moiety can be substituted or unsubstituted.

The term "substituted" as used herein in conjunction with a molecule or an organic group as defined herein refers to the state in which one or more hydrogen atoms contained therein are replaced by one or more non-hydrogen atoms. The term "functional group" or "substituent" as used herein refers to a group that can be or is substituted onto a molecule or onto an organic group. Examples of substituents or functional groups include, but are not limited to, a halogen (e.g., F, Cl, Br, and I); an oxygen atom in groups such as hydroxy groups, alkoxy groups, aryloxy groups, aralkyloxy groups, oxo(carbonyl) groups, carboxyl groups including carboxylic acids, carboxylates, and carboxylate esters; a sulfur atom in groups such as thiol groups, alkyl and aryl sulfide groups, sulfoxide groups, sulfone groups, sulfonyl groups, and sulfonamide groups; a nitrogen atom in groups such as amines, hydroxyamines, nitriles, nitro groups, N-oxides, hydrazides, azides, and enamines; and other heteroatoms in various other groups. Non-limiting examples of substituents that can be bonded to a substituted carbon (or other) atom include F, Cl, Br, I, OR, OC(O)N(R)₂, CN, NO, NO₂, ONO₂, azido, CF₃, OCF₃, R, O (oxo), S (thiono), C(O), S(O), methylenedioxy, ethylenedioxy, N(R)₂, SR, SOR, SO₂R, SO₂N(R)₂, SO₃R, C(O)R, C(O)C(O)R, C(O)CH₂C(O)R, C(S)R, C(O)OR, OC(O)R, C(O)N(R)₂, OC(O)N(R)₂, C(S)N(R)₂, (CH₂)₀₋₂N(R)C(O)R, (CH₂)₀₋₂N(R)N(R)₂, N(R)N(R)C(O)R, N(R)N(R)C(O)OR, N(R)N(R)CON(R)₂, N(R)SO₂R, N(R)SO₂N(R)₂, N(R)C(O)OR, N(R)C(O)R, N(R)C(S)R, N(R)C(O)N(R)₂, N(R)C(S)N(R)₂, N(COR)COR, N(OR)R, C(=NH)N(R)₂, C(O)N(OR)R, and C(=NOR)R, wherein R can be hydrogen or a carbon-based moiety; for example, R can be hydrogen, (C₁-C₁₀₀)hydrocarbyl, alkyl, acyl, cycloalkyl, aryl, aralkyl, heterocyclyl, heteroaryl, or heteroarylalkyl; or wherein two R groups bonded to a nitrogen atom or to adjacent nitrogen atoms can together with the nitrogen atom or atoms form a heterocyclyl.

The term "alkyl" as used herein refers to straight chain and branched alkyl groups and cycloalkyl groups having from 1 to 40 carbon atoms, 1 to about 20 carbon atoms, 1 to 12 carbons or, in some embodiments, from 1 to 8 carbon atoms. Examples of straight chain alkyl groups include those with from 1 to 8 carbon atoms such as methyl, ethyl, n-propyl, n-butyl, n-pentyl, n-hexyl, n-heptyl, and n-octyl groups. Examples of branched alkyl groups include, but are not limited to, isopropyl, iso-butyl, sec-butyl, t-butyl, neopentyl, isopentyl, and 2,2-dimethylpropyl groups. As used herein, the term "alkyl" encompasses n-alkyl, isoalkyl, and anteisoalkyl groups as well as other branched chain forms of alkyl. Representative substituted alkyl groups can be substituted one or more times with any of the groups listed herein, for example, amino, hydroxy, cyano, carboxy, nitro, thio, alkoxy, and halogen groups.

The term "alkenyl" as used herein refers to straight and branched chain and cyclic alkyl groups as defined herein, except that at least one double bond exists between two carbon atoms. Thus, alkenyl groups have from 2 to 40 carbon atoms, or 2 to about 20 carbon atoms, or 2 to 12 carbon atoms or, in some embodiments, from 2 to 8 carbon atoms. Examples include, but are not limited to vinyl, -CH=CH(CH₃), -CH=C(CH₃)₂, -C(CH₃)=CH₂, -C(CH₃)=CH(CH₃), - C(CH₂CH₃)=CH₂, cyclohexenyl, cyclopentenyl, cyclohexadienyl, butadienyl, pentadienyl, and hexadienyl among others.

The term "acyl" as used herein refers to a group containing a carbonyl moiety wherein the group is bonded via the carbonyl carbon atom. The carbonyl carbon atom is bonded to a hydrogen forming a "formyl" group or is bonded to another carbon atom, which can be part of an alkyl, aryl, aralkyl cycloalkyl, cycloalkylalkyl, heterocyclyl, heterocyclylalkyl, heteroaryl, heteroarylalkyl group or the like. An acyl group can include 0 to about 12, 0 to about 20, or 0 to about 40 additional carbon atoms bonded to the carbonyl group. An acyl group can include double or triple bonds within the meaning herein. An acryloyl group is an example of an acyl group. An acyl group can also include heteroatoms within the meaning herein. A nicotinoyl group (pyridyl-3-carbonyl) is an example of an acyl group within the meaning herein. Other examples include acetyl, benzoyl, phenylacetyl, pyridylacetyl, cinnamoyl, and acryloyl groups and the like. When the group containing the carbon atom that is bonded to the carbonyl carbon atom contains a halogen, the group is termed a "haloacyl" group. An example is a trifluoroacetyl group.

The term "aryl" as used herein refers to cyclic aromatic hydrocarbon groups that do not contain heteroatoms in the ring. Thus aryl groups include, but are not limited to, phenyl, azulenyl, heptalenyl, biphenyl, indacenyl, fluorenyl, phenanthrenyl, triphenylenyl, pyrenyl, naphthacenyl, chrysenyl, biphenylenyl, anthracenyl, and naphthyl groups. In some embodiments, aryl groups contain about 6 to about 14 carbons in the ring portions of the groups. Aryl groups can be unsubstituted or substituted, as defined herein. Representative substituted aryl groups can be mono-substituted or substituted more than once, such as, but not limited to, a phenyl group substituted at any one or more of 2-, 3-, 4-, 5-, or 6-positions of the phenyl ring, or a naphthyl group substituted at any one or more of 2- to 8-positions thereof.

The term "heterocyclyl" as used herein refers to aromatic and non-aromatic ring compounds containing three or more ring members, of which one or more is a heteroatom such as, but not limited to, N, O, and S.

The term "heteroaryl" as used herein refers to aromatic ring compounds containing 5 or more ring members, of which, one or more is a heteroatom such as, but not limited to, N, O, and S; for instance, heteroaryl rings can have 5 to about 8-12 ring members. A heteroaryl group is a variety of a heterocyclyl group that possesses an aromatic electronic structure. The term "heterocyclylalkyl" as used herein refers to alkyl groups as defined herein in which a hydrogen or carbon bond of an alkyl group as defined herein is replaced with a bond to a heterocyclyl group as defined herein. Representative heterocyclyl alkyl groups include, but are not limited to, furan-2-yl methyl, furan-3-yl methyl, pyridine-3-yl methyl, tetrahydrofuran-2-yl ethyl, and indol-2-yl propyl.

The term "alkoxy" as used herein refers to an oxygen atom connected to an alkyl group, including a cycloalkyl group, as are defined herein. Examples of linear alkoxy groups include but are not limited to methoxy, ethoxy, propoxy, butoxy, pentyloxy, hexyloxy, and the like. Examples of branched alkoxy include but are not limited to isopropoxy, sec-butoxy, tert-butoxy, isopentyloxy, isohexyloxy, and the like. Examples of cyclic alkoxy include but are not limited to cyclopropyloxy, cyclobutyloxy, cyclopentyloxy, cyclohexyloxy, and the like. An alkoxy group can include about 1 to about 12, about 1 to about 20, or about 1 to about 40 carbon atoms bonded to the oxygen atom, and can further include double or triple bonds, and can also include heteroatoms. For example, an allyloxy group or a methoxyethoxy group is also an alkoxy group within the meaning herein, as is a methylenedioxy group in a context where two adjacent atoms of a structure are substituted therewith.

The term "amine" as used herein refers to primary, secondary, and tertiary amines having, e.g., the formula N(group)₃ wherein each group can independently be H or non-H, such as alkyl, aryl, and the like. Amines include but are not limited to R-NH₂, for example, alkylamines, arylamines, alkylarylamines; R₂NH wherein each R is independently selected, such as dialkylamines, diarylamines, aralkylamines, heterocyclylamines and the like; and R₃N wherein each R is independently selected, such as trialkylamines, dialkylarylamines, alkyldiarylamines, triarylamines, and the like. The term "amine" also includes ammonium ions as used herein.

The terms "halo," "halogen," or "halide" group, as used herein, by themselves or as part of another substituent, mean, unless otherwise stated, a fluorine, chlorine, bromine, or iodine atom.

The term "hydrocarbon" or "hydrocarbyl" as used herein refers to a molecule or functional group, respectively, that includes carbon and hydrogen atoms. The term can also refer to molecule or functional group that normally includes both carbon and hydrogen atoms but wherein all the hydrogen atoms are substituted with other functional groups.

As used herein, the term "hydrocarbyl" refers to a functional group derived from a straight chain, branched, or cyclic hydrocarbon, and can be alkyl, alkenyl, alkynyl, aryl, cycloalkyl, acyl, or any combination thereof. Hydrocarbyl groups can be shown as (Cₐ-C_{b})hydrocarbyl, wherein a and b are integers and mean having any of a to b number of carbon atoms. For example, (C₁-C₄)hydrocarbyl means the hydrocarbyl group can be methyl (C₁), ethyl (C₂), propyl (C₃), or butyl (C₄), and (C₀-C_{b})hydrocarbyl means in certain embodiments there is no hydrocarbyl group.

The term "number-average molecular weight" (Mₙ) as used herein refers to the ordinary arithmetic mean of the molecular weight of individual molecules in a sample. It is defined as the total weight of all molecules in a sample divided by the total number of molecules in the sample. Experimentally, Mₙ is determined by analyzing a sample divided into molecular weight fractions of species i having nᵢ molecules of molecular weight Mᵢ through the formula Mₙ = ΣMᵢnᵢ / Σnᵢ. The Mₙ can be measured by a variety of well-known methods including gel permeation chromatography, spectroscopic end group analysis, and osmometry. If unspecified, molecular weights of polymers given herein are number-average molecular weights.

The term "solvent" as used herein refers to a liquid that can dissolve a solid, liquid, or gas. Non-limiting examples of solvents are silicones, organic compounds, water, alcohols, ionic liquids, and supercritical fluids.

The term "air" as used herein refers to a mixture of gases with a composition approximately identical to the native composition of gases taken from the atmosphere, generally at ground level. In some examples, air is taken from the ambient surroundings. Air has a composition that includes approximately 78% nitrogen, 21% oxygen, 1% argon, and 0.04% carbon dioxide, as well as small amounts of other gases.

The term "room temperature" as used herein refers to a temperature of about 15 °C to 28 °C.

The term "standard temperature and pressure" as used herein refers to 20 °C and 101 kPa.

The term "coating" as used herein refers to a continuous or discontinuous layer of material on the coated surface, wherein the layer of material can penetrate the surface and can fill areas such as pores, wherein the layer of material can have any three-dimensional shape, including a flat or curved plane. In one example, a coating can be formed on one or more surfaces, any of which may be porous or nonporous, by immersion in a bath of coating material.

The term "surface" as used herein refers to a boundary or side of an object, wherein the boundary or side can have any perimeter shape and can have any three-dimensional shape, including flat, curved, or angular, wherein the boundary or side can be continuous or discontinuous. While the term surface generally refers to the outermost boundary of an object with no implied depth, when the term 'pores' is used in reference to a surface, it refers to both the surface opening and the depth to which the pores extend beneath the surface into the substrate.

As used herein, the term "polymer" refers to a molecule having at least one repeating unit and can include copolymers.

In various embodiments, salts having a positively charged counterion can include any suitable positively charged counterion. For example, the counterion can be ammonium(NH4⁺), or an alkali metal such as sodium (Na⁺), potassium (K⁺), or lithium (Li⁺). In some embodiments, the counterion can have a positive charge greater than +1, which can in some embodiments complex to multiple ionized groups, such as Zn²⁺, Al³⁺, or alkaline earth metals such as Ca²⁺ or Mg²⁺.

In various embodiments, salts having a negatively charged counterion can include any suitable negatively charged counterion. For example, the counterion can be a halide, such as fluoride, chloride, iodide, or bromide. In other examples, the counterion can be nitrate, hydrogen sulfate, dihydrogen phosphate, bicarbonate, nitrite, perchlorate, iodate, chlorate, bromate, chlorite, hypochlorite, hypobromite, cyanide, amide, cyanate, hydroxide, permanganate. The counterion can be a conjugate base of any carboxylic acid, such as acetate or formate. In some embodiments, a counterion can have a negative charge greater than -1, which can in some embodiments complex to multiple ionized groups, such as oxide, sulfide, nitride, arsenate, phosphate, arsenite, hydrogen phosphate, sulfate, thiosulfate, sulfite, carbonate, chromate, dichromate, peroxide, or oxalate.

The polymers described herein can terminate in any suitable way. In some embodiments, the polymers can terminate with an end group that is independently chosen from a suitable polymerization initiator, -H, -OH, a substituted or unsubstituted (C₁-C₂₀)hydrocarbyl (e.g., (C₁-C₁₀)alkyl or (C₆-C₂₀)aryl) interrupted with 0, 1, 2, or 3 groups independently selected from -O-, substituted or unsubstituted -NH-, and -S-, a poly(substituted or unsubstituted (Ci-C₂₀)hydrocarbyloxy), and a poly(substituted or unsubstituted (C₁-C₂₀)hydrocarbylamino).

### Method of melt-stabilizing ultra high molecular weight polyethylene (UHMWPE).

Oxidation of polyethylene can occur through a free radical pathway, as shown in the following sequence:

RH + IN → R· Initiation

R· + O₂ → ROO·

ROO- + RH → ROOH + R· Propagation

ROOH > RO· + HO·

RO· + RH → ROH + R· Chain Branching

HO· + RH → HOH + R·

ROO· (RO· etc.) → Inert Products Termination

ROO· + AH → ROOH + A·

RO- + AH → ROH + A- Inhibition (stabilization)

HO· + AH > HOH + A·

wherein
RH = polymer (e.g., polyethylene, UHMWPE)
IN = initiator (e.g., irradiation)
AH = inhibitor (e.g., free-radical scavenging antioxidant)

In various embodiments, the present invention provides a method of melt-stabilizing ultra high molecular weight polyethylene (UHMWPE). The method can include coating a solid material including UHMWPE with an antioxidant, to provide an antioxidant-coated solid material. The method can include pre-irradiatively heating the antioxidant-coated solid material to diffuse the antioxidant therein, to provide an antioxidant-diffused solid material. The method can include irradiating the antioxidant-diffused solid material, to provide an irradiated solid material. The method can include post-irradiatively heating the irradiated solid material, the heating sufficient to melt at least part of the UHMWPE, to provide a heated material. The method can include solidifying the heated material, to provide a melt-stabilized material.

In certain examples, one or more agents, e.g., bioactive agents, can be added to the material including UHMWPE. Such addition can be accomplished during any stage of preparation but may be desirable after any heat treatments are performed to reduce the likelihood of deactivation of the bioactive agent. Illustrative agents include, but are not limited to, an antibiotic, a steroid, a drug, a growth factor such as bone morphogenic protein, an osteocyte, an osteoclast or other cells, a vitamin, a chondroitin, a glucosamine, a glycosoaminglycan, high energy phosphates such as phosphoenolpyruvate, ATP, 5'-AMP, and other small molecule biologics or other chemical or biological agents. In some examples, the material including UHMWPE can be loaded with stem cells, and the material can act as a scaffold to permit growth and differentiation of bone or cartilage within the polymer framework. The presence of an antioxidant in the material including UHMWPE can act to prevent degradation of the scaffold in its use environment and may also provide some oxidative protection to the bioactive agent or stem cells loaded into the scaffold.

In certain examples, the method of melt-stabilizing UHMWPE can include any suitable physical manipulation before, between, or after any suitable steps of the method (e.g., coating, pre-irradiatively heating, preheating, irradiating, post-irradiatively heating, or solidifying), such as molding, compressing, consolidating, removing material from, or otherwise processing to provide a desired shape, part size, or other physical attributes to render the part suitable for its intended use.

In certain embodiments, additional components may be combined with the material including UHMWPE before, between, or after any suitable steps of the method (e.g., any of coating, pre-irradiatively heating, preheating, irradiating, post-irradiatively heating, or solidifying). In one embodiment, tribological components such as metal and/or ceramic articulating components and/or preassembled bipolar components may be joined with the material including UHMWPE. In other embodiments, metal backing (e.g., plates or shields) may be added. In further embodiments, surface components such a trabecular metal, fiber metal, Sulmesh^{™} coating, meshes, cancellous titanium, and/or metal or polymer coatings may be added to or joined with the material including UHMWPE. Radiomarkers or radiopacifiers such as tantalum, steel and/or titanium balls, wires, bolts or pegs may be added. Locking features such as rings, bolts, pegs, snaps and/or cements/adhesives can be added. These additional components may be used to form sandwich implant designs, radiomarked implants, metal-backed implants to prevent direct bone contact, functional growth surfaces, and/or implants with locking features.

### Material including UHMWPE.

The material including the UHMWPE is a solid monolithic material, such as a single solid mass, a non-particulate form, a non-powder, a bar, or a form. Any suitable proportion of the solid material including UHMWPE can be the UHMWPE, such as about 1 wt% to about 100 wt% of the solid material, about 90 wt% to about 100 wt%, or about 1 wt% or less, or about 2, 3, 4, 5, 10, 15, 20, 25, 30, 35, 40, 45, 50, 55, 60, 65, 70, 75, 80, 85, 90, 95, 96, 97, 98, 99, or about 99.9 wt% or more. The UHMWPE can form a homogeneous or heterogeneous mixture with other components in the solid material.

UHWMPE is a semi crystalline, linear homopolymer of ethylene, which in some embodiments can be produced by stereospecific polymerization with a Ziegler-Natta catalyst at low pressure (6-8 bar) and low temperature (66-80°C). The synthesis of UHMWPE can result in a fine granular powder. The molecular weight and its distribution can be controlled by process parameters such as temperature, time and pressure. UHMWPE generally has a molecular weight of at least about 2,000,000 g/mol. Suitable UHMWPE materials for use as raw materials may be in the form of a powder or mixture of powders. Examples of suitable UHMWPE materials include GUR^{®} 1020 and GUR^{®} 1050 available from Ticona Engineering Polymers. UHMWPE powder can be processed and consolidated to form a solid monolithic material.

In addition to UHMWPE, the solid material including UHMWPE can include any other suitable component. In certain embodiments, the UHWMPE can be combined with another crosslinkable polymer. The crosslinkable polymer can be any polymer that is crosslinkable using radiation, a chemical crosslinking agent or that can be physically cross-linked under suitable conditions. In some examples, the polymer can be a thermoplastic polymer such as, for example, an acrylonitrile butadiene styrene (ABS) polymer, an acrylic polymer, a celluloid polymer, a cellulose acetate polymer, a cycloolefin copolymer (COC), an ethylenevinyl acetate (EVA) polymer, an ethylene vinyl alcohol (EVOH) polymer, a fluoroplastic, an ionomer, an acrylic/PVC alloy, a liquid crystal polymer (LCP), a polyacetal polymer (POM or acetal), a polyacrylate polymer, a polyacrylonitrile polymer (PAN or acrylonitrile), a polyamide polymer (PA or nylon), a polyamide-imide polymer (PAI), a polyaryletherketone polymer (PAEK or ketone), a polybutadiene polymer (PBD), a polybutylene polymer (PB), a polybutylene terephthalate polymer (PBT), a polycaprolactone polymer (PCL), a polychlorotrifluoroethylene polymer (PCTFE), a polyethylene terephthalate polymer (PET), a polycyclohexylene dimethylene terephthalate polymer (PCT), a polycarbonate polymer, a polyhydroxyalkanoate polymer (PHA), a polyketone polymer (PK), a polyester polymer, a polyethylene polymer (PE), a polyetheretherketone polymer (PEEK), a polyetherketoneketone polymer (PEKK), a polyetherimide polymer (PEI), a polyethersulfone polymer (PES), a polyethylenechlorinate polymer (PEC), a polyimide polymer (PI), a polylactic acid polymer (PLA), a polymethylpentene polymer (PMP), a polyphenylene oxide polymer (PPO), a polyphenylene sulfide polymer (PPS), a polyphthalamide polymer (PPA), a polypropylene polymer, a polystyrene polymer (PS), a polysulfone polymer (PSU), a polytrimethylene terephthalate polymer (PTT), a polyurethane polymer (PU), a polyvinyl acetate polymer (PVA), a polyvinyl chloride polymer (PVC), a polyvinylidene chloride polymer (PVDC), and a styreneacrylonitrile polymer (SAN). Illustrative types of polyethylene in addition to the UHMWPE include, for example, ultra low molecular weight polyethylene (ULMWPE), high molecular weight polyethylene (HMWPE), high density polyethylene (HDPE), high density cross-linked polyethylene (HDXLPE), cross-linked polyethylene (PEX or XLPE), medium density polyethylene (MDPE), low density polyethylene (LDPE), linear low density polyethylene (LLDPE) and very low density polyethylene (VLDPE). In some examples, a polypropylene can be used. A polypropylene may be particularly desirable where the final product is a mesh, stent, breast implant material, suture material or other medical device. In one alternative, a polypropylene (or other polymer) may be used as one layer in a multi-layered medical device. Illustrative polypropylenes include, but are not limited to, a homopolymeric polypropylene, a block copolymeric polypropylene, and a random copolymeric polypropylene. In certain examples, the polymers used in the compositions described herein can be copolymerized with one or more monomers or polymers. The solid material including UHMWPE is a consolidated mixture of UHMWPE and any other suitable component.

In certain examples, the solid material including UHMWPE can include one or more suitable additives that impart a desired physical or chemical property. Illustrative suitable additives include, but are not limited to, radiopaque materials, antimicrobial materials such as silver ions, antibiotics, and microparticles and/or nanoparticles serving various functions. Preservatives, colorants and other conventional additives may also be used.

In certain embodiments, the solid material including UHMWPE can be prepared by a method including blending a UHMWPE powder with other suitable materials, such as a blend with another polymer or a blend with an antioxidant. Such processes include physical mixing, mixing with the aid of a solvent, mixing with the aid of a solvent (e.g., CO₂) under supercritical temperature and pressure conditions, and ultrasonic mixing.

In some embodiments, the solid material including UHMWPE can include an antioxidant, such as any one or more suitable antioxidants described herein. The one or more antioxidants can be present in any suitable concentration, such as any concentration described herein. The one or more antioxidant can be present in any type of distribution in the solid material including UHMWPE, such as a substantially homogeneous distribution. In other embodiments, the solid material including UHMWPE can be substantially free of antioxidants.

### Coating.

The method includes coating the solid material including UHMWPE with an antioxidant, to provide an antioxidant-coated solid material. The antioxidant can be applied neat or as part of a liquid composition including the antioxidant. The coating can be any suitable coating method that applies the antioxidant sufficiently such that the antioxidant can penetrate a surface layer of the solid material including UHMWPE. The coating can be performed using any suitable coating process, such as one or more of brushing, dipping, soaking, immersion with agitation or stirring, spraying, and the like.

In the present invention, the coating is performed such that the antioxidant does not penetrate past a depth of 0 mm of the surface of application, to provide an antioxidant-coated solid material.

According to the disclosure, the coating can be sufficient for the antioxidant to infuse (e.g., penetrate) into a surface layer of the solid material including UHMWPE that includes any suitable depth from the surface of the solid material including UHMWPE where the coating is applied, such as about 0 mm (e.g., only present on or very near the surface of application), about 0 mm to about 1 mm, about 0 mm to about 10 mm deep, about 1 mm to about 10 mm deep, about 0 mm to about 20 mm deep, about 1 mm or less, or about 1.5 mm, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, or about 20 mm or more. According to the disclosure, the coating can be performed such that the antioxidant does not penetrate past a certain depth of the solid material including UHMWPE. For example, the coating penetrates the solid material including UHMWPE no deeper than about 1 mm or less, or about 1.5 mm, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19 mm, or about 20 mm or more. According to the disclosure, the coating does not penetrate past the surface layer, wherein the non-surface layer portions of the solid material including UHMWPE are substantially free of the antioxidant. According to the disclosure, the coating penetrates the solid material including UHMWPE such that in at least one of the antioxidant-coated solid material the antioxidant is present to a depth of about 0 mm to about 1 mm, about 0 mm to about 10 mm deep, about 1 mm to about 10 mm deep, about 0 mm to about 20 mm deep, about 1 mm or less, or about 1.5 mm, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, or about 20 mm or more. Subsequent to penetration, the antioxidant can diffuse through the UHMWPE material, such as during pre-irradiative heating.

In some embodiments, the antioxidant added via the coating can protect the UHMWPE in the solid material including UHMWPE from oxidation by oxygen in the air during a subsequent melt-stabilization. For example, the coating can allow the antioxidant to penetrate and subsequently diffuse into the UHMWPE on the surface of the solid material including UHMWPE and protect the UHMWPE therein from oxidation by oxygen in the air, as described herein.

The coating can include coating any suitable proportion of the total surface area of the solid material including UHMWPE. The coating can include selective coating or uniform coating of the solid material including UHMWPE. The coating can be sufficient to contact the antioxidant with at least some of the UHMWPE in the solid material including UHMWPE, wherein the UHMWPE can be on the surface or proximate to the surface (e.g., within 1 mm to about 10 mm). In an embodiment wherein the solid material only has exposed UHMWPE on a portion of the surface, or only has UHMWPE within about 1-10 mm of only a portion of the surface, the method can optionally include only coating the part of the surface of the solid material that includes the UHMWPE or that is proximate to UHMWPE. For example, the coating can include coating about 1% to about 100% of the total surface area of the solid material, about 50% to about 100%, about 90% to about 100%, or about 1% or less, or about 2%, 3, 4, 5, 10, 15, 20, 25, 30, 35, 40, 45, 50, 55, 60, 65, 70, 75, 80, 85, 90, 91, 92, 93, 94, 95, 96, 97, 98, 99, 99.5, 99.9, 99.99, or about 99.999% or more.

The coating can be sufficient to provide any suitable weight gain to the solid material including UHM WPE, such that the antioxidant is suitably applied to the solid material including UHMWPE. For example, the coating can be sufficient to provide a weight gain of about 0.000,01 g per cm² surface area of the solid material to about 50 g/cm² surface area, about 0.000,1 g/cm² surface area to about 1 g/cm² surface area, about 0.000,01 g/cm² surface area or less, or about 0.000,1 g/cm² surface area, 0.000,2, 0.000,5, 0.000,8, 0.001, 0.005, 0.01, 0.05, 0.1, 0.5, 1, 1.5, 2, 2.5, 3, 3.5, 4, 4.5, 5, 6, 7, 8, 9, 10, 12, 14, 16, 18, 20, 25, 30, 35, 40, 45, or about 50 g/cm² surface area or more.

### Liquid composition including the antioxidant.

The coating of the solid material including UHMWPE with an antioxidant can include coating the solid material with neat antioxidant or coating the solid material with a liquid composition including the antioxidant. The liquid composition can be a solution of the one or more antioxidants in one or more suitable solvents (e.g., carrier liquids). The neat antioxidant can be applied if it is a liquid with low enough viscosity, or it can be dissolved in a suitable carrier fluid, such as if it is a viscous liquid or solid. The concentration of the antioxidant can be varied to control the amount of antioxidant infused and diffused in the solid material including UHMWPE. The antioxidant or the multiple antioxidants can be any suitable wt% of the liquid composition, such as about 0.01 wt% to about 100 wt% of the liquid composition, about 1 wt% to about 100 wt%, about 5 wt% to about 100 wt%, about 0.01 wt% or less, or about 0.1 wt%, 1, 2, 3, 4, 5, 6, 7, 8, 9, 10, 15, 20, 25, 30, 35, 40, 45, 50, 55, 60, 65, 70, 75, 80, 85, 90, 91, 92, 93, 94, 95, 96, 97, 98, 99, 99.5, 99.9, 99.99, or about 99.999 wt% of the liquid composition or more.

The carrier liquid can be any suitable carrier liquid. The carrier liquid can be water (e.g., di-ionized water), or an aqueous solution (e.g., saline). The carrier liquid can be an organic solvent, such as any suitable organic solvent, such as acetone, methanol, ethanol, or propanol (e.g., isopropanol or normal propanol). The carrier liquid can be any suitable proportion of the liquid composition including the antioxidant, such as about 1 wt% to about 99 wt%, 5 wt% to about 95 wt%, or about 1 wt% or less, or about 2 wt%, 3, 4, 5, 10, 15, 20, 25, 30, 35, 40, 45, 50, 55, 60, 65, 70, 75, 80, 85, 90, 91, 92, 93, 94, 95, 96, 97, 98, or about 99 wt% or more. In embodiments of the method wherein the liquid composition includes one or more solvents, the method can include heating the material including UHMWPE in subsequent steps sufficiently such that one or more of the one or more solvents is substantially completely evaporated from the material including UHMWPE, for example, such that only the one or more antioxidants are left behind.

The liquid composition can include any suitable material in addition to the one or more antioxidants and the one or more optional carrier fluids. For example, in some embodiments, the liquid composition includes one or more organic peroxides. In some embodiments, the one or more organic peroxides can provide crosslinking, reducing or eliminating a subsequent irradiation crosslinking step.

### Antioxidant.

The antioxidant can be any suitable antioxidant. The antioxidant can be a free-radical scavenger, such that the antioxidant can neutralize a free-radical before the free-radical can react with oxygen to form an oxidized species. The antioxidant can be any suitable antioxidant that allows the method to effectively produce materials including UHMWPE that can resist oxidation, such as melt-stabilized materials including UHMWPE having less or no oxidized layer when melt-stabilized in an oxygen-containing environment. One antioxidant can be used, or multiple antioxidants can be used.

The one or more antioxidants can form any suitable wt% of the material including the UHMWPE, such as the antioxidant-coated solid material, the antioxidant-diffused solid material, the irradiated solid material, the heated material, or the melt-stabilized material, such as about 0.01 wt% to about 20 wt%, about 0.1 wt% to about 5 wt%, about 0.01 wt% or less, or about 0.05 wt%, 0.1, 0.2, 0.3, 0.4, 0.5, 0.6, 0.7, 0.8, 0.9, 1, 1.2, 1.4, 1.6, 1.8, 2, 2.2, 2.4, 2.6, 2.8, 3, 3.5, 4, 4.5, 5, 6, 7, 8, 9, 10, 15, or about 20 wt% or more.

The antioxidant can have a molecular weight that allows the antioxidant to diffuse effectively during the pre-irradiative heating. For example, the antioxidant can have a molecular weight of less than 10,000 g/mol, or less than 5,000, about 100 to about 5,000, about 100 to about 2,000, about 100 or less, or about 200, 300, 400, 500, 600, 700, 800, 900, 1,000, 1,200, 1,400, 1,600, 1,800, 2,000, 2,500, 3,000, 3,500, 4,000, 4,500, 5,000, 6,000, 7,000, 8,000, 9,000, or about 10,000 g/mol or more.

The antioxidant can be at least one of a tocopherol, a tocopherol phosphite, a tocotrienol, vitamin E, vitamin E acetate, a protected vitamin E, a rosemary oil, pentaerythritol tetrakis(3-(3,5-di-tert-butyl-4-hydroxyphenyl)propionate), butanedioic acid dimethyl ester/4-hydroxy-2,2,6,6-tetramethyl-1-piperidine ethanol copolymer, tannic acid, bilberry extract, vitamin C, a carotene, a flavonoid, an isoflavonoid, a neoflavonoid, a lignin, quinine, ubiquinone, vitamin K1, a metal, glutathione, propyl gallate, octyl gallate, lauryl gallate, resveratrol, rosmarinic acid, rutin, 5-aminosalicylic acid, butylated hydroxy anisole, butylated hydroxy toluene, a phenolic compound, and a monomeric or polymeric hindered amine stabilizer. The antioxidant can be at least one of vitamin E, vitamin E acetate, pentaerythritol tetrakis(3-(3,5-di-tert-butyl-4-hydroxyphenyl)propionate), butanedioic acid dimethyl ester/4-hydroxy-2,2,6,6-tetramethyl-1-piperidine ethanol copolymer, tannic acid, and bilberry extract. The antioxidant can be at least one of racemic alpha-tocopherol, RRR-alpha-tocopherol, SRR-alpha-tocopherol, SSR-alpha-tocopherol, SRS-alpha-tocopherol, SSS-alpha-tocopherol, RSR-alpha-tocopherol, RRS-alpha-tocopherol, RSS-alpha-tocopherol, racemic beta-tocopherol, RRR-beta-tocopherol, SRR-beta-tocopherol, SSR-beta-tocopherol, SRS-beta-tocopherol, SSS-beta-tocopherol, RSR-beta-tocopherol, RRS-beta-tocopherol, RSS-beta-tocopherol, racemic gamma-tocopherol, RRR-gamma-tocopherol, SRR-gamma-tocopherol, SSR-gamma-tocopherol, SRS-gamma-tocopherol, SSS-gamma-tocopherol, RSR-gamma-tocopherol, RRS-gamma-tocopherol, RSS-gamma-tocopherol, racemic delta-tocopherol, RRR-delta-tocopherol, SRR-delta-tocopherol, SSR-delta-tocopherol, SRS-delta-tocopherol, SSS-delta-tocopherol, RSR-delta-tocopherol, RRS-delta-tocopherol, and RSS-delta-tocopherol.

A tocopherol can have the structure:

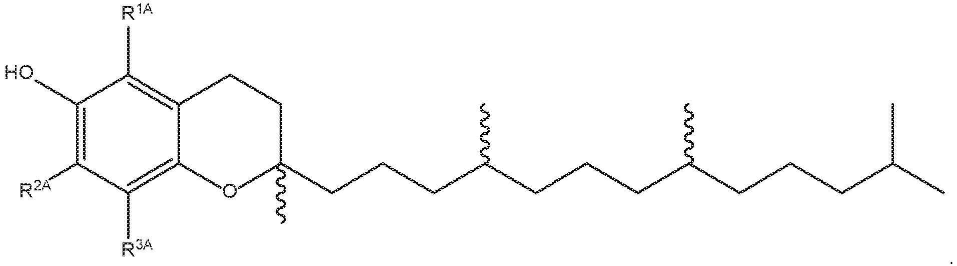

The variables R^{1A}, R^{2A}, and R^{3A} are each independently selected from hydrogen, substituted or unsubstituted (C₁-C₁₀)alkyl, and substituted or unsubstituted (C₁-C₁₀)alkenyl. The stereochemistry of the tocopherol can be racemic or at least one of RRR, SRR, SSR, SRS, RSR, RRS, RSS, and SSS. In some embodiments, R^{1A}, R^{2A}, and R^{3A} are each (C₁-C₁₀)alkyl, such as methyl (e.g., alpha-tocopherol). In some embodiments, R^{1A} and R^{3A} are each (C₁-C₁₀)alkyl, such as methyl, and R^{2A} is hydrogen (beta-tocopherol). In some embodiments, R^{2A} and R^{3A} are each (C₁-C₁₀)alkyl, such as methyl, and R^{1A} is hydrogen (gamma-tocopherol). In some embodiments, R^{1A} and R^{2A} are each hydrogen and R^{3A} is (C₁-C₁₀)alkyl, such as methyl (delta-tocopherol).

A tocotrienol can have the structure:

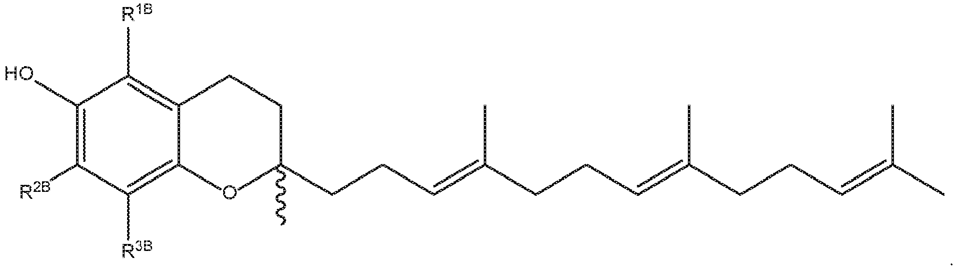

The variables R^{1B}, R^{2B}, and R^{3B} are each independently selected from hydrogen, substituted or unsubstituted (C₁-C₁₀)alkyl, and substituted or unsubstituted (C₁-C₁₀)alkenyl. The stereochemistry of the tocotrienol can be racemic or at least one of R and S. In some embodiments, R^{1B}, R^{2B}, and R^{3B} are each (C₁-C₁₀)alkyl, such as methyl (e.g., alpha-tocotrienol). In some embodiments, R^{1B} and R^{3B} are each (C₁-C₁₀)alkyl, such as methyl, and R^{2B} is hydrogen (beta-tocotrienol). In some embodiments, R^{2B} and R^{3B} are each (C₁-C₁₀)alkyl, such as methyl, and R^{1B} is hydrogen (gamma-tocotrienol). In some embodiments, R^{1B} and R^{2B} are each hydrogen and R^{3B} is (C₁-C₁₀)alkyl, such as methyl (delta-tocotrienol). A tocopherol or tocotrienol can be naturally occurring or synthetic.

In various embodiments, the antioxidant can be at least one of a hindered amine stabilizer or a hindered phenol stabilizer. For example, the antioxidant can be at least one of 4-Allyloxy-2-hydroxybenzophenone, 1-aza-3,7-dioxabicyclo[3.3.0]octane-5-methanol, 2-(2H-benzotriazol-2-yl)-4,6-bis(1-methyl-1-phenylethyl)phenol, 2-(2H-benzotriazol-2-yl)-4,6-di-tert-pentylphenol, 2-(2H-benzotriazol-2-yl)-6-dodecyl-4-methylphenol, 2-[3-(2H-benzotriazol-2-yl)-4-hydroxyphenyl]ethyl methacrylate, 2-(2H-benzotriazol-2-yl)-4-methyl-6-(2-propenyl)phenol, 2-(2H-benzotriazol-2-yl)-4-(1,1,3,3-tetramethylbutyl)phenol, 2-(4-benzoyl-3-hydroxyphenoxy)ethyl acrylate, 3 ,9-bis(2,4-dicumylphenoxy)-2,4,8, 1 0-tetraoxa-3 ,9-diphosphaspiro[5.5]undecane, bis(octadecyl)hydroxylamine, 3,9-bis(octadecyloxy)-2,4,8,10-tetraoxa-3,9-diphosphaspiro[5.5]undecane, bis(1-octyloxy-2,2,6,6-tetramethyl-4-piperidyl) sebacate, bis(2,2,6,6-tetramethyl-4-piperidyl) sebacate, 2-tert-butyl-6-(5-chloro-2H-benzotriazol-2-yl)-4-methylphenol, 2-tert-butyl-4-ethylphenol, 5-chloro-2-hydroxybenzophenone, 5-chloro-2-hydroxy-4-methylbenzophenone, 2,4-di-tert-butyl-6-(5-chloro-2H-benzotriazol-2-yl)phenol, 2,6-di-tert-butyl-4-(dimethylaminomethyl)phenol, 3',5'-dichloro-2'-hydroxyacetophenone, didodecyl 3,3'-thiodipropionate, 2,4-dihydroxybenzophenone, 2,2'-dihydroxy-4,4'-dimethoxybenzophenone, 2,2'-dihydroxy-4-methoxybenzophenone, 2',4'-dihydroxy-3'-propyl acetophenone, 2,3-dimethylhydroquinone, 2-(4,6-diphenyl-1,3,5-triazin-2-yl)-5-[(hexyl)oxy]-phenol, 5-ethyl-1-aza-3,7-dioxabicyclo[3.3.0]octane, ethyl 2-cyano-3,3-diphenylacrylate, 2-ethylhexyl 2-cyano-3,3-diphenylacrylate, 2-ethylhexyl trans-4-methoxycinnamate, 2-ethylhexyl salicylate, 2,2'-ethylidene-bis(4,6-di-tert-butylphenol), 2-hydroxy-4-(octyloxy)benzophenone, menthyl anthranilate, 2-methoxyhydroquinone, methyl-p-benzoquinone, 2,2'-methylenebis[6-(2H-benzotriazol-2-yl)-4-(1,1,3,3-tetramethylbutyl)phenol], 2,2'-methylenebis(6-tert-butyl-4-ethylphenol), 2,2'-methylenebis(6-tert-butyl-4-methylphenol), 5,5'-methylenebis(2-hydroxy-4-methoxybenzophenone), methylhydroquinone, 4-nitrophenol sodium salt hydrate, octadecyl 3-(3,5-di-tert-butyl-4-hydroxyphenyl)propionate, pentaerythritol tetrakis(3,5-di-tert-butyl-4-hydroxyhydrocinnamate), 2-phenyl-5-benzimidazolesulfonic acid, poly[[6-[(1,1,3,3-tetramethylbutyl)amino]-s-triazine-2,4-diyl]-[(2,2,6,6-tetramethyl-4-piperidyl)imino]-hexamethylene-[(2,2,6,6-tetramethyl-4-piperidyl)imino], sodium D-isoascorbate monohydrate, tetrachloro- 1,4-benzoquinone, triisodecyl phosphite, 1,3,5-trimethyl-2,4,6-tris(3,5-di-tert-butyl-4-hydroxybenzyl)benzene, tris(4-tert-butyl-3-hydroxy-2,6-dimethylbenzyl) isocyanurate, tris(2,4-di-tert-butylphenyl) phosphite, 1,3,5-tris(2-hydroxyethyl)isocyanurate, and 2-[(2E,6E,10E,14E,18E,22E,26E,30E,34E)-3,7,11,15,19,23,27,31,35,39-decamethyltetraconta-2,6,10,14,18,22,26,30,34,38-decaenyl]-5,6-dimethoxy-3-methyl-benzene-1,4-diol (e.g., Ubiquinol). In some embodiments, the hindered amine stabilizer is at least one of a 2,2,6,6-tetra((C₁-C₅₀)hydrocarbyl)-4-piperidyl diester of HOC(O)-(C₁-C₅₀)hydrocarbyl-C(O)OH, a 2,2,6,6-tetramethyl-4-piperidyl diester of HOC(O)-(C₁-C₅₀)hydrocarbyl-C(O)OH, bis(2,2,6,6-tetramethyl-4-piperidyl) sebacate, 1,2,2,6,6-penta((C₁-C₅₀)hydrocarbyl)-4-piperidyl diester of HOC(O)-(C₁-C₅₀)hydrocarbyl-C(O)OH, a 1,2,2,6,6-pentamethyl-4-piperidyl diester of HOC(O)-(C₁-C₅₀)hydrocarbyl-C(O)OH, 2,2,6,6-tetramethylpiperidine, wherein each (C₁-C₅₀)hydrocarbyl is independently selected, is substituted or unsubstituted, and is interrupted by 0, 1, 2, or 3 -O- groups.

The antioxidant can be a protected antioxidant. A protected antioxidant can be advantageous because it can avoid or reduce acting as an antioxidant during irradiative crosslinking, but after irradiative crosslinking (and deprotection) can act as an antioxidant to reduce oxidation and corresponding degradation of the UHMWPE. By avoiding or reducing antioxidant activity during crosslinking, a higher crosslinking density can be achieved.

In some embodiments, the antioxidant is a protected tocopherol or tocotrienol having the structure: 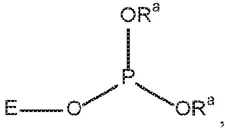 or a salt thereof, or 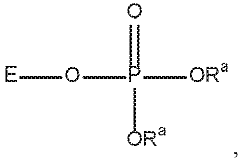 or a salt thereof.

At each occurrence, R^{a} can be independently chosen from -H, -E, and substituted or unsubstituted (C₁-C₁₀)hydrocarbyl. The variable E can have the structure: 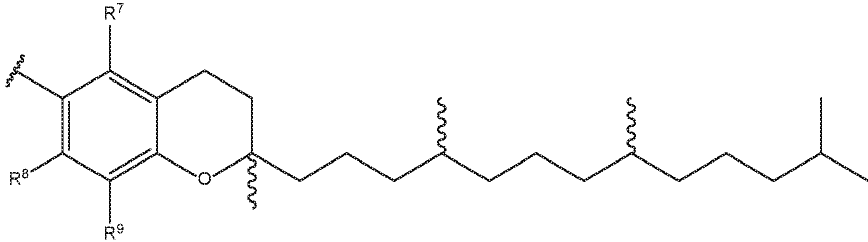 or

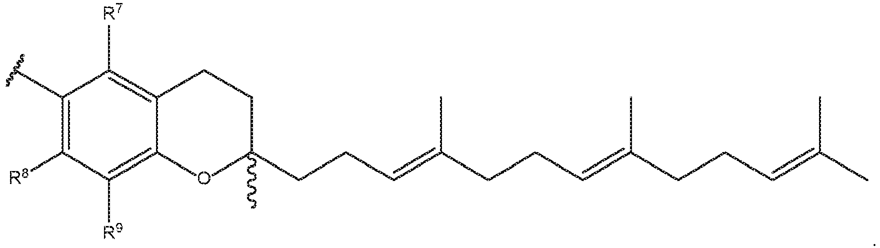

The variables R⁷, R⁸, and R⁹ are each independently chosen from -H, substituted or unsubstituted (C₁-C₁₀)alkyl, and substituted or unsubstituted (C₁-C₁₀)alkenyl. The method can include converting at least some of protected tocopherol or tocotrienol to a compound of the formula E: OH, such as after the irradiative crosslinking.

The antioxidant can be a hindered amine stabilizer-protected tocopherol or tocotrienol of formula (I): 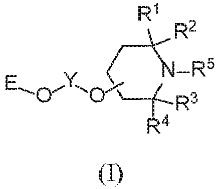 or a salt thereof. The variables R¹, R², R³, and R⁴ can be each, independently, hydrogen or (C₁-C₁₀)alkyl. The variable R⁵ can be chosen from hydrogen, (C₁-C₁₀)alkyl, -O·, and -OR¹¹ wherein R¹¹ can be hydrogen or (C₁-C₁₀)alkyl. The variable E can have the structure: 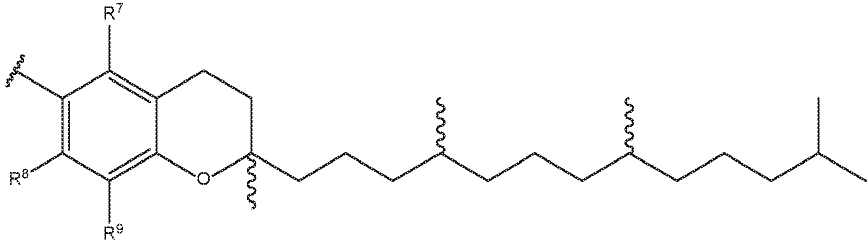 or

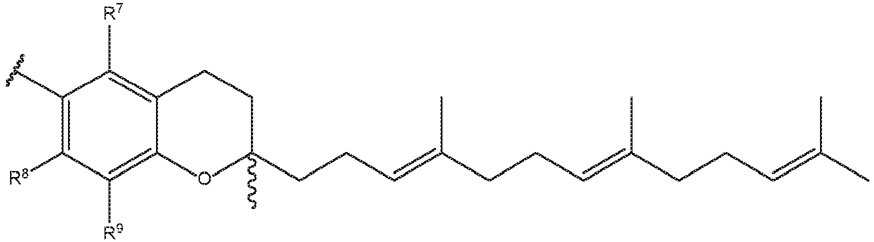

The variables R⁷, R⁸, and R⁹ can be each independently chosen from -H, substituted or unsubstituted (C₁-C₁₀)alkyl, and substituted or unsubstituted (C₁-C₁₀)alkenyl. The variable Y can represent the group:

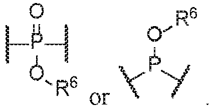

The variable R⁶ can be hydrogen, (C₁-C₁₀)alkyl, -E, or a radical of the formula:

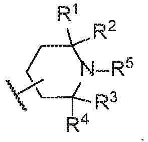

The one or more compounds of the formula (1) can be substantially uniformly distributed throughout the ultrahigh molecular weight polyethylene. The method include converting at least some of the compound of the formula (I) to a compound of the formula E-OH, after the irradiating step.

The group E-O- can be a vitamin E radical. The group R⁶-O- can be a vitamin E radical. The variable R⁶ can be a radical of the formula:

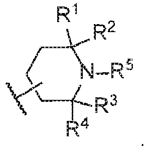

The variables R¹, R², R³, R⁴, and R⁵ can be each, independently, (C₁-C₁₀)alkyl. The variables R¹, R², R³, R⁴, and R⁵ can be each methyl. The variable Y can represent the group:

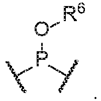

### Pre-irradiatively heating.

The method can include pre-irradiatively heating the antioxidant-coated solid material. The pre-irradiative heating diffuses the antioxidant in the solid material. The pre-irradiative heating provides an antioxidant-diffused solid material. The pre-irradiative heating can be sufficient to at least partially diffuse the one or more antioxidants in the antioxidant-coated solid material. The pre-irradiative heating can drive the antioxidant into the interior of the material. The distance of the diffusion can be dependent on characteristics of the specific antioxidant such as molecular weight and similarity of solubility parameter with respect to UHMWPE. Migration distance and rate can also depend on the physical state of the antioxidant; for example, the diffusion distance can be greater when the melting point of the antioxidant is below the temperature achieved during the pre-irradiative heating.

The pre-irradiative heating can melt any suitable amount of the antioxidant-diffused solid material, or of the UHMWPE in the antioxidant-diffused solid material, such as about 0 vol% to about 100 vol%, or about 1 vol% or less, or about 2 vol%, 3, 4, 5, 6, 7, 8, 9, 10, 15, 20, 25, 30, 35, 40, 45, 50, 55, 60, 65, 70, 75, 80, 85, 90, 95, 96, 97, 98, or about 99 vol% or more. The heating is sufficient to at least partially diffuse the one or more antioxidants in the antioxidant-coated solid material.

The method can include pre-irradiatively heating the antioxidant-diffused solid material in an environment including oxygen, the heating sufficient to melt at least part of the UHMWPE, to provide a heated material. In some embodiments, the method includes heating the antioxidant-diffused solid material in an environment substantially free of oxygen. Various embodiments of the present invention provide a means to reduce the oxidized layer that forms during pre-irradiative heating of a material including UHMWPE in an oxygen-containing environment such as air. During the pre-irradiative heating, the antioxidant can scavenge the free radicals present in the outer layer that would normally be oxidized. The pre-irradiative heating can occur in an environment including any suitable amount of oxygen. For example, the heating can occur in an environment including ambient air, having about 20-21 vol% oxygen. The heating can occur in an environment having about 1 vol% to about 50 vol% oxygen, about 10 vol% to about 30 vol% oxygen, about 1 vol% oxygen or less, or about 2 vol%, 3, 4, 5, 6, 8, 10, 12, 14, 16, 18, 20, 22, 24, 26, 28, 30, 32, 34, 36, 38, 40, 42, 44, 46, 48, or about 50 vol% oxygen or more.

The pre-irradiative heating heats the antioxidant-diffused solid material to any suitable temperature, such as about 50 °C to about 300 °C, about 80 °C to about 250 °C, about 130 °C to about 160 °C, about 50 °C or less, or about 60 °C, 70, 80, 90, 100, 110, 120, 130, 140, 150, 160, 170, 180, 190, 200, 220, 240, 260, 280, or about 300 °C or more. The temperature achieved can be a temperature below the melting point (e.g., about 138 °C), or above the melting point. The antioxidant-diffused solid material can be heated for any suitable duration, such as about 1 minute to about 7 days, or about 1 hour to about 48 hours, or about 1 minute or less, or about 2 minutes, 3, 4, 5, 10, 15, 20, 25, 30, 35, 40, 45, 50, 55 minutes, 1 hour, 1.5, 2, 2.5, 3, 3.5, 4, 4.5, 5, 6, 7, 8, 9, 10, 11, 12, 13, 14, 15, 16, 17, 18, 19, 20, 21, 22, 23 hours, 1 day, 1.5, 2, 2.5, 3, 3.5, 4, 4.5, 5, 5.5, 6, 6.5 days, or about 7 days or more.

The pre-irradiative heating can be sufficient to form a substantially homogenous distribution of the antioxidant in a surface layer of the antioxidant-diffused solid material. The surface layer can be a layer of any suitable depth as measured from the outside of the material, such as about 0 mm to about 1 mm deep, about 0 mm to about 10 mm deep, about 1 mm to about 10 mm deep, about 0 mm to about 20 mm deep, about 1 mm or less, or about 1.5 mm, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, or about 20 mm deep or more. The pre-irradiative heating can be sufficient to allow the antioxidant (e.g., at least some of the antioxidant) to penetrate to a depth of at least about 1 mm from a surface (e.g., a surface where antioxidant was coated) of the antioxidant-diffused solid material, or about 2 mm, 3, 4, 5, 6, 7, 8, 9, 10, 11, 12, 13, 14, 15, 16, 17, 18, 19, or about 20 mm from a surface.

The pre-irradiative heating can optionally include cooling prior to the irradiation (e.g., prior to an optional preheating step, or directly prior to the irradiation). The cooling can be to any temperature below the melting point of the heated material, such as to room temperature. The cooling can occur in ambient conditions, or the cooling can occur in a chilled environment. The cooling can occur in any medium, such as in a gas (e.g., air) or in a liquid (e.g., water).

### Preheating.

The method can include preheating prior to irradiation (e.g., warm-irradiating). In other embodiments, the method can be free of preheating prior to irradiation. The preheating can be the same as the pre-irradiative heating, or the preheating and the pre-irradiative heating can be different heating steps. In some embodiments, the pre-irradiative heating can be preheating to or above a preheat temperature, to provide a preheated antioxidant-diffused solid material, wherein the irradiating includes irradiating the preheated antioxidant diffused solid material. In some embodiments, the pre-irradiative heating and preheating can be separate steps, wherein after the pre-irradiative heating the antioxidant-diffused solid material that was pre-irradiatively heated is preheated to at or above a preheat temperature to provide a preheated antioxidant-diffused solid material, wherein the irradiating includes irradiating the preheated antioxidant-diffused solid material.

In some embodiments, the preheating can include heating to a temperature above room temperature and below or above the melting point of the UHMWPE or mixture of UHMWPE and other components, such as about 50 °C to about 300 °C, about 80 °C to about 250 °C, about 130 °C to about 160 °C, about 50 °C or less, or about 55 °C, 60, 65, 70, 75, 80, 85, 90, 95, 100, 105, 110, 115, 120, 125, 130, 140, 145, 150, 160, 170, 180, 190, 200, 220, 240, 260, 280, or about 300 °C or more, such that at the time of irradiation onset the material has a preheated temperature. The preheating can be performed for any suitable amount of time, such as about 1 minute to about 7 days, or about 1 hour to about 48 hours, or about 1 minute or less, or about 2 minutes, 3, 4, 5, 10, 15, 20, 25, 30, 35, 40, 45, 50, 55 minutes, 1 hour, 1.5, 2, 2.5, 3, 3.5, 4, 4.5, 5, 6, 7, 8, 9, 10, 11, 12, 13, 14, 15, 16, 17, 18, 19, 20, 21, 22, 23 hours, 1 day, 1.5, 2, 2.5, 3, 3.5, 4, 4.5, 5, 5.5, 6, 6.5 days, or about 7 days or more.

### Irradiating.

The method includes irradiating the antioxidant-diffused material with an electron beam, to provide an irradiated solid material. The antioxidant-diffused material can be preheated, or no preheating occurs prior to irradiation (e.g., the antioxidant-diffused material can be approximately ambient temperature or room temperature when irradiation begins). The irradiating can crosslink the UHMWPE in the antioxidant-diffused solid material.

Other types of irradiation can be visible light radiation, infrared radiation, ultraviolet radiation, electron beam radiation, gamma radiation, or X-ray radiation. Where ionizing radiation is employed to effect the crosslinking reaction, the radiation can be obtained from any suitable source such as an atomic pile, a resonant transformer accelerator, a Van de Graaff electron accelerator, a Linac electron accelerator, a betatron, a synchrotron, a cyclotron, or the like. Radiation from these sources will produce ionizing radiation such as electrons, protons, neutrons, deuterons, gamma rays, X-rays, alpha particles, or beta particles. Where ionizing radiation is used, a sufficient radiation dose rate and absorbed dose can be used to induce crosslinking and/or control the degree of crosslinking. In some embodiments, during the irradiation, the temperature of the UHMWPE or mixture of UHMWPE and other components can be maintained below the melting point of the same. In some embodiments, during the irradiation, the temperature of the UHMWPE or mixture of UHMWPE and other components can be allowed to rise above the melting point of the same. In various embodiments, during irradiation, the temperature can be allowed to rise to, or the temperature can be maintained at, about 60 °C, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 250, 275, or about 300 °C or more. In various embodiments, the UHMWPE or mixture of UHMWPE and other components can be preheated to a temperature below the melting point of the same, then subsequently irradiated while maintaining the temperature of the preheated UHMWPE or mixture of UHMWPE and other components below the melting point of the same.

In various embodiments, the irradiating, such as electron-beam irradiation, uses a total dose of about 1 kGy to about 100,000 kGy, 10 kGy to about 1000 kGy, about 50 kGy to about 500 kGy, 50 kGy to 300 kGy, or about 1 kGy or less, or about 5, 10, 15, 20, 25, 50, 75, 100, 125, 150, 175, 200, 250, 300, 350, 400, 500, 750, 1,000, 1,250, 1,500, 1,750, 2,000, 2,500, 3,000, 4,000, 5,000, 7,500, 10,000, 15,000, 20,000, 25,000, 50,000, 75,000, or about 100,000 kGy or more. In various embodiments, the irradiating includes using a dose rate of about 0.001 mGy/h to about 500 MGy/h, about 1 mGy/h to about 50 MGy/h, or about 0.001 mGy/h or less, or about 0.005 mGy/h, 0.01, 0.05, 0.1, 0.5, 1, 1.5, 2, 2.5, 3, 3.5, 4, 4.5, 5, 6, 7, 8, 9, 10, 11, 12, 13, 14, 15, 16, 17, 18, 19, 20, 22, 24, 26, 28, 30, 35, 40, 45, 50, 60, 70, 80, 90, 100, 150, 200, 250, 300, 400, or about 500 MGy/h or more.

In certain examples, irradiative crosslinking can be performed in the presence of an additive that can promote or deter crosslinking, depending on the desired level of crosslinking. Illustrative crosslinking promoters include, but are not limited to, trimethylolpropane triacrylate, trimethylolpropane trimethacrylate, pentaerythritol tetraacrylate, and pentaerythritol tetramethacrylate.

### Melt-stabilizing.

The method can include post-irradiatively heating the irradiated solid material. The post-irradiative heating is sufficient to melt at least part of the UHMWPE. The post-irradiative heating provides a heated material. The method can include solidifying the heated material, to provide a melt-stabilized material.

The post-irradiative heating can melt any suitable amount of the irradiated solid material, or of the UHMWPE in the irradiated solid material, such as about 1 vol% to about 100 vol%, or about 1 vol% or less, or about 2 vol%, 3, 4, 5, 6, 7, 8, 9, 10, 15, 20, 25, 30, 35, 40, 45, 50, 55, 60, 65, 70, 75, 80, 85, 90, 95, 96, 97, 98, or about 99 vol% or more. The heating is sufficient to melt-stabilize the irradiated solid material, such that at least some of the free radicals (e.g., free radicals in the UHMWPE, which can be generated during irradiation) can recombine or otherwise be neutralized.

The method includes post-irradiatively heating the irradiated solid material in an environment including oxygen, the heating sufficient to melt at least part of the UHMWPE, to provide a heated material. In some embodiments, the method includes heating the irradiated solid material in an environment substantially free of oxygen. Various embodiments of the present invention provide a means to reduce the oxidized layer that forms during melt-stabilization of a material including UHMWPE in an oxygen-containing environment such as air. During the melt-stabilization, the antioxidant can scavenge the free radicals present in the outer layer that would normally be oxidized. The heating can occur in an environment including any suitable amount of oxygen. For example, the heating can occur in an environment including ambient air, having about 20-21 vol% oxygen. The heating can occur in an environment having about 1 vol% to about 50 vol% oxygen, about 10 vol% to about 30 vol% oxygen, about 1 vol% oxygen or less, or about 2 vol%, 3, 4, 5, 6, 8, 10, 12, 14, 16, 18, 20, 22, 24, 26, 28, 30, 32, 34, 36, 38, 40, 42, 44, 46, 48, or about 50 vol% oxygen or more.

The post-irradiative heating heats the irradiated solid material to any suitable temperature, such as about 50 °C to about 300 °C, about 80 °C to about 250 °C, about 130 °C to about 160 °C, about 50 °C or less, or about 60 °C, 70, 80, 90, 100, 110, 120, 130, 140, 150, 160, 170, 180, 190, 200, 220, 240, 260, 280, or about 300 °C or more. The irradiated solid material can be heated for any suitable duration, such as about 1 minute to about 7 days, or about 1 hour to about 48 hours, or about 1 minute or less, or about 2 minutes, 3, 4, 5, 10, 15, 20, 25, 30, 35, 40, 45, 50, 55 minutes, 1 hour, 1.5, 2, 2.5, 3, 3.5, 4, 4.5, 5, 6, 7, 8, 9, 10, 11, 12, 13, 14, 15, 16, 17, 18, 19, 20, 21, 22, 23 hours, 1 day, 1.5, 2, 2.5, 3, 3.5, 4, 4.5, 5, 5.5, 6, 6.5 days, or about 7 days or more.

The solidifying can be any suitable solidifying, such that the melted material is allowed to solidify. The solidifying can include allowing the post-irradiatively heated material to cool to a temperature below the melting point of the heated material, such as to room temperature. The solidifying can occur in ambient conditions, or the solidifying can occur in a chilled environment. The solidifying can occur in any medium, such as in a gas (e.g., air) or in a liquid (e.g., water).

The method can be effective to generate a melt-stabilized material including UHMWPE, melt-stabilized in an environment including oxygen, that has decreased or no oxidation in a surface layer of the material, as compared to other methods for melt-stabilization in an oxygen-containing environment. The surface layer including decreased or no oxidation can be a surface layer that corresponds to the entire outer surface of the material, such as for a material including UHMWPE on the entire surface of the material (e.g., the material can be 100% UHMWPE or can have UHMWPE distributed evenly throughout). The surface layer can be a portion of the outer surface that corresponds to a portion of the outer surface of the material, such as for a material including UHMWPE on only a portion of the surface of the material, or such as for a material that was only partially coated with the liquid composition including the antioxidant. The surface layer can be a layer of any suitable depth as measured from the outside of the material, such as about 0 mm to about 1 mm deep, about 0 mm to about 10 mm deep, about 1 mm to about 10 mm deep, about 0 mm to about 20 mm deep, about 1 mm or less, or about 1.5 mm, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, or about 20 mm deep or more.

The irradiated solid material can have a first concentration of free-radicals. The first concentration of free-radicals can be any suitable concentration, such as about 1 × 10¹⁵ spins/gram to about 1 × 10²⁰ spins/g, 1 × 10¹⁶ spins/g to 1 × 10¹⁸ spins/g, or about 1 × 10¹⁵ spin/g or less, or about 1 × 10¹⁶ spins/g, 1 × 10¹⁷, 1 × 10¹⁸, 1 × 10¹⁹, 1 × 10²⁰, 1 × 10²¹, 1 × 10²², 1 × 10²³, 1 × 10²⁴, 1 × 10²⁵, 1 × 10²⁶, 1 × 10²⁷, 1 × 10²⁸, 1 × 10²⁹, or about 1 × 10³⁰ spins/g or more. The number of spins per gram of the material can be measured in any suitable fashion, such as by electron spin resonance (ESR). The first concentration of free-radicals can be a concentration in the UHMWPE or a concentration in the irradiated solid including the UHMWPE. The first concentration of free-radicals can be a concentration in a part or localized area of the material, or can be a concentration throughout the entire material including the UHMWPE. In some embodiments, the first concentration of free-radicals can be generated by and consistent with an amount of irradiation applied to the antioxidant-diffused solid material to crosslink the UHMWPE or to crosslink other components in the antioxidant-diffused solid material.

The method can include solidifying the post-irradiatively heated material, to provide a melt-stabilized material including UHMWPE including a second concentration of free-radicals, wherein the second concentration of free-radicals is less than the first concentration of free-radicals. The melt-stabilization can reduce the concentration of free-radicals. The concentration of free-radicals in the UHWMPE can be reduced. The concentration of free-radicals in other materials can also optionally be reduced, for materials including other materials in addition to UHMWPE, such as other polyethylenes or other polymers. The second concentration of free-radicals in the melt-stabilized material can be any suitable concentration that is lower than the first concentration of free radicals, such as about 1 × 10⁵ spins/g to about 1 × 10¹⁵ spins/g, or about 1 × 10² spins/g or less, or about 1 × 10³ spins/g, 1 × 10⁴, 1 × 10⁵, 1 × 10⁶, 1 × 10⁷, 1 × 10⁸, 1 × 10⁹, 1 × 10¹⁰, 1 × 10¹¹, 1 × 10¹², 1 × 10¹³, 1 × 10¹⁴ spins/g, 1 × 10¹⁵ spins/g or more. The number of spins per gram of the material can be measured in any suitable fashion, such as by electron spin resonance (ESR). The second concentration of free-radicals can be a concentration in the UHMWPE or a concentration in all the materials the melt-stabilized material including the UHMWPE, corresponding to the part or localized area where the first concentration of free-radicals is determined. The second concentration of free-radicals can be a concentration in a part or localized area of the material (e.g., corresponding to a part or localized area where the first concentration of free-radicals is measured), or can be a concentration throughout the melt-stabilized material including the UHMWPE. The second concentration of free-radicals can be any suitable proportion of the first concentration of free-radicals. For example, the second concentration of free-radical s can be about 1% to about 0.000,1% of the first concentration of free-radicals, about 0.1% to about 0.001%, or about 1% or more, or about 0.5 %, 0.1, 0.05, 0.01, 0.005, 0.001, 0.000,5, or about 0.000,1% or less.

As used herein, "oxidation index" refers to an area ratio of fourier transform infrared (FTIR) peaks at 1765-1680 cm⁻¹ (e.g. carbonyl peaks) to FTIR peaks 1392-1330 cm⁻¹ (e.g., methyl peaks), wherein the area of the carbonyl absorptions centered near 1720 cm⁻¹ is related to the amount of chemically bound oxygen present in the material, and the intensity (area) of the C-H absorption centered near 1370 cm⁻¹ is used to normalize for the sample's thickness. A surface layer (e.g., the entire surface, or only part of the surface, of any suitable depth) of the melt-stabilized material can have an oxidation index that does not exceed 1 (e.g., the average oxidation index of the surface layer does not exceed an oxidation index of 1 or any portion of the surface layer does not exceed an oxidation index of 1). For example, in some embodiments, the surface layer of the melt-stabilized material has an oxidation index that does not exceed 0.5, or that is about 0.001 to about 1, 0.01 to about 0.5, or about 0.001 or less, or that is equal to or less than about 0.002, 0.003, 0.004, 0.005,0.006, 0.008, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.15, 0.2, 0.25, 0.3, 0.35, 0.4, 0.45, 0.5, 0.6, 0.7, 0.8, 0.9, or about 1 or more. The surface layer can be a layer of any suitable depth on the material, such as about 0 mm deep (e.g., the top surface most exposed to oxygen), or a layer about 0 mm deep to about 1 mm deep, about 0 mm deep to about 10 mm deep, or about 1 mm deep or less, or about 2 mm, 3, 4, 5, 6, 7, 8, 9, 10, 11, 12, 13, 14, 15, 16, 17, 18, 19, or about 20 mm deep or more.

The melt-stabilized material can have any suitable concentration of antioxidant at various depths from the surface of the material. For example, the coating, pre-irradiative heating, irradiating, and melt-stabilizing (e.g., post-irradiative heating and solidifying) can be sufficient such that the melt-stabilized material has a vitamin E index (VEI, the FTIR ratio of the peak areas between 1275 and 1245 cm⁻¹ to the peak areas between 1985 and 1850 cm⁻¹) in a surface layer of about -0.1 to about 0.5, about -0.05 to about 0.25, about 0.01 to about 0.25, about 0.05 to about 0.25, about 0.1 to about 0.25, or about -0.1 or less, or about -0.08, -0.06, - 0.04, -0.02, -0.01, 0, 0.01, 0.02, 0.04, 0.06, 0.08, 0.1, 0.12, 0.14, 0.16, 0.18, 0.2, 0.22, 0.24, 0.26, 0.28, 0.3, 0.35, 0.4, 0.45, or about 0.5 or more. The surface layer can be a layer of any suitable depth on the material, such as about 0 mm deep (e.g., the top surface most exposed to oxygen), or a layer about 0 mm deep to about 1 mm deep, about 0 mm deep to about 10 mm deep, or about 1 mm deep or less, or about 2 mm, 3, 4, 5, 6, 7, 8, 9, 10, 11, 12, 13, 14, 15, 16, 17, 18, 19, or about 20 mm deep or more. In some embodiments, the VEI can be a gradient that is highest at a depth of 0 mm and that becomes lower at deeper depths. In some embodiments, the VEI can be substantially similar throughout the surface layer or throughout the melt-stabilized material.

The melt-stabilized material can have any suitable concentration of the coated and diffused antioxidant at various depths from the surface of the material, such as an antioxidant (e.g., vitamin E), or such as another component. For example, the coating, pre-irradiative heating, irradiating, and melt-stabilizing (e.g., post-irradiative heating and solidifying) can be sufficient such that the melt-stabilized material has a concentration of an antioxidant such as vitamin E in a surface layer of about 0.001 wt% to about 10 wt%, about 0.01 wt% to about 5 wt%, about 0.1 wt% to about 2.5 wt%, about 0.1 wt% to about 1 wt%, or about 0.001 wt% or less, or about 0.01, 0.1, 0.2, 0.4, 0.6, 0.8, 1, 1.2, 1.4, 1.6, 1.8, 2, 2.4, 2.6, 2.8, 3, 3.5, 4, 4.5, 5, 6, 7, 8, 9, or about 10 wt% or more. The surface layer can be a layer of any suitable depth on the material, such as about 0 mm deep (e.g., the top surface most exposed to oxygen), or a layer about 0 mm deep to about 1 mm deep, about 0 mm deep to about 10 mm deep, or about 1 mm deep or less, or about 2 mm, 3, 4, 5, 6, 7, 8, 9, 10, 11, 12, 13, 14, 15, 16, 17, 18, 19, or about 20 mm deep or more. In some embodiments, the concentration of the antioxidant can be a gradient (linear or non-linear) that is highest at a depth of 0 mm and that becomes lower at deeper depths. In some embodiments, the concentration of the component can be substantially similar throughout the surface layer or throughout the melt-stabilized material.

### UHMWPE material and medical implant including the same.

In various embodiments, the present invention provides a melt-stabilized material made by any suitable embodiment of a method described herein. For example, in various embodiments, the present invention provides an oxygen-containing-environment-melt-stabilized material including UHMWPE and an antioxidant, the antioxidant introduced prior to an irradiation step, the melt-stabilized material being free of post-melt-stabilization oxidized surface layer removal greater than about 1 mm depth, 1.5, 2, 2.5, 3, 3.5, 4, 4.5, 5, 5.5, 6, 6.5, 7, 7.5, 8, 8.5, 9, 9.5, or greater than about 10 mm depth, wherein the UHMWPE in a surface layer (e.g., about 0 mm deep, or a layer about 0 mm deep to about 1 mm deep, about 0 mm deep to about 10 mm deep, or about 1 mm deep or less, or about 2 mm, 3, 4, 5, 6, 7, 8, 9, 10, 11, 12, 13, 14, 15, 16, 17, 18, 19, or about 20 mm deep or more) of the melt-stabilized material (e.g., the material after any surface layer removal) has an oxidation index that does not exceed 1.

In various embodiments, the present invention provides a medical implant including any suitable melt-stabilized material including UHMWPE that can be produced by an embodiment of the method described herein. The method of melt-stabilizing UHMWPE can include generating a medical implant from the resulting material, such that the method is a method of making a medical implant. In some embodiments, various amounts of the surface of the melt-stabilized material can be removed during processing and machining the material into the desired shape for the implant, such as about 0 mm to about 1 mm, about 0 mm to about 5 mm, about 0 mm to about 10 mm, about 0.1 mm or less, or about 0.5 mm, 1, 1.5, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, or about 20 mm or more. In some embodiments, the medical implant can be an orthopedic implant. In various embodiments, the medical implant can form or be part of an artificial hip, hip liner, knee, knee liner, disk replacement, shoulder, elbow, foot, ankle, finger, mandible, or bearings in an artificial heart.

### Examples

Various embodiments of the present invention can be better understood by reference to the following Examples which are offered by way of illustration. The present invention is not limited to the Examples given herein.

Oxidation levels were determined through the blocks at center from top to bottom and side to side of each block, bottom denoting the surface the block was setting on during the melt stabilization process. The FTIR Oxidation Index (OI) was determined per ASTM F2102-06. Following the ASTM F2102-06 protocol, 100-200 micron thick films were microtomed from the block of material, with the top indicative of the initial incident irradiation face. The film was scanned with an FTIR spectrophotometer using an indexing microscopic attachment to obtain infrared spectra at 200 micron intervals across the entire length of the film. The oxidation index at various locations scanned was then calculated using the ratio of the oxidation peak (1765-1680 cm⁻¹, centered at 1720 cm⁻¹) to a control peak that does not change with irradiation (1392-1330 cm⁻¹, centered at 1370 cm⁻¹).

The trans-vinylene index throughout the Examples is determined as the area of the infrared absorption peak centered near 965 cm⁻¹ to the area of the of the C-H absorption peak centered near 1370 cm⁻¹. The area of the trans-vinylene absorptions (-C=C-) centered near 965 cm⁻¹ is related to the amount of crosslinking experienced by the material when exposed to ionizing radiation. Polymer main chain unsaturation in the form of trans-vinyl groups are a side reaction during crosslinking via ionizing radiation such as gamma, x-ray and electron beam. The correlation between TVI and actual received radiation dose can depend on the nature of the irradiation conditions, for example, radiation source (gamma or electron beam), temperature, dose rate, and oxygen level. The amount of unsaturation formation can be directly correlated with the amount of irradiation (e.g., dose), and can be used as a dosimeter for a given material and irradiation method combination.

The vitamin E-phosphite was synthesized as follows. The equipment used included (1) three neck schlenk style round bottom 100 ml flask; (1) gas inlet, schlenk; (1) addition funnel, schlenk; (1) reflux condenser, schlenk; (1) filter funnel, 25-50 µm glass frit disc, schlenk; (1) single neck collection flask, 100 ml, schlenk; (1) distillation adapter, schlenk; (1) water chilled condenser, schlenk; (1) single neck collection flask, 50 ml, schlenk; (1) magnetic stir bar to fit 100 ml round bottom flask; (1) pipet, 5 ml; (1) pipet, 1 ml; (1) pipet bulb; (1) lab scale, resolution ≥ 0.001 g; (1) hot plate with magnetic stir capability; and (1) glass jar with large stir bar for use as heating bath. The materials also included all racemic d,l-α-tocopherol; HPLC grade dichloromethane; triethylamine, assay ≥ 99%; Aldrich grade reagent-plus PCl₃. assay ≥ 99%; molecular sieves, type 3A, 8-12 mesh; dry nitrogen gas; and heat transfer oil. Activated molecular sieves, type 3A, 8-12 mesh were dried 4 hours @ 220 C, cooled 1 hour @ 110 C, and returned to dried glass container. Fisher HPLC grade dichloromethane and Sigma-Aldrich triethylamine, assay ≥ 99%,were dried over activated molecular sieves prior to use. All glassware was dried before use by gently warming with a hot air gun or oven, taking care not to overheat the schlenk connections to cause distortion. A 100 ml three neck Schlenk-style flask was set up containing a magnetic stir bar and fitted with a gas inlet and addition funnel. The flask was charged with 3.79 × 10⁻² mole (16.3424 g) of d,l-α-tocopherol. The tocopherol was dissolved under a dry nitrogen purge with 15 ml dry dichloromethane. Triethylamine (5.5 ml) was pipeted into the flask (about 3.95 × 10 ⁻² mole) under dry purge with stirring. Dichloromethane (10 ml) was added to the addition funnel under dry nitrogen purge. PCl₃ was added (1.198 × 10 ⁻² mole, 1.05 ml) under dry purge into the addition funnel containing the 10 ml of dichloromethane (95% of calculated stoichiometry based on amount of tocopherol). The diluted PCl₃ solution was added drop-wise to the reaction flask with stirring under dry purge (a precipitate will form). The Schlenk-style addition funnel used for the PCl₃ addition was rinsed into the reaction flask with three additional 5 ml aliquots of dichloromethane to insure complete transfer of the PCl₃. The reaction flask was stirred for one hour at ambient temperature under dry nitrogen purge. The reaction flask was slowly heated with stirring to 60 °C with a reflux condenser attached to the flask maintaining the dry nitrogen purge and maintain for one hour at 60 °C. Precipitate was filtered off using dry nitrogen purge/vacuum with 25-50 µm glass frit disc filter, and 100 ml collection flask, Schlenk. The single neck 100 ml collection flask used to collect the product was fitted with a distillation adapter, water chilled condenser, vacuum adapter and 50 ml collection flask. To remove volatiles, the 100 ml collection flask was heated to 100 °C overnight under dry nitrogen purge with stirring, then a vacuum was applied for one hour maintaining the 100 °C temperature with stirring. The remaining product was hot filtered using dry nitrogen purge/vacuum with 25-50 µm glass frit disc filter, and 100 ml tared collection flask, Schlenk. The filtrate was cooled to ambient under dry nitrogen purge. The product weight was 7.1 g (clear, amber liquid). The product was diluted quantitatively to a 10% by weight solution in dry dichloromethane. The diluted product was stored under dry nitrogen in a sealed glass container, in a dessicator. Remaining VE-Phosphite in glassware was recovered, after sitting several weeks in air and light, by dilution with dry dicholormethane and subsequent filtering. The recovered product weight was 6.3 g (clear, amber liquid).

### Example 1

Four sections of a GUR 1050 compression molded bar were treated as follows:
Sample 1.1.1: No coating.
Sample 1.1.2: Coated with a solution of 10% vitamin E (VE) dissolved in isopropanol.
Sample 1.1.3: Coated with a solution of 10% vitamin E-phosphite (VEP) dissolved in dichloromethane.
Sample 1.1.4: Coated with a solution of 10% recovered vitamin E-phosphite (VEP-R) dissolved in dichloromethane.

The recovered VEP was obtained by a secondary cleaning of the glassware used for synthesis of the VEP, which had some increased exposure to oxygen during recovery as compared to the VEP which was maintained under an inert environment until application to the UHMWPE section.

All sections were individually placed in a metallized mylar bag under nitrogen purge to prevent oxygen infiltration. The bagged sections were then heated to 120 °C for 14 hours, irradiated warm (120 °C) with 75 kGy using a 10 MeV electron beam. After cooling in the mylar bags purged with nitrogen, the sections were removed from the inert atmosphere and were melted at 150 °C for 14 hours in air, followed by slow cooling (16 hours) to ambient temperature, in air.

The Samples were sectioned, then films were prepared with a microtome from top to bottom of each sectioned form, and from side to side of each sectioned form, and analyzed for trans-vinyl index and oxidation index per ASTM F2381-10 and ASTM F2102-13, respectively.

Table 1 illustrates oxidation index to a depth of 5 mm as measured from the top, bottom, and both sides of Samples 1.1.1-1.1.4.

**Table 1. Oxidation index for Samples 1.1.1-1.1.4.**

| **Sample** | **Dept h, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 1.1.1 | 0 | 5.343 | 0.305 | 6.224 | 7.026 | 0.037 | 0.015 | 0.057 | 0.049 | None |
| | 200 | 1.736 | 0.214 | 3.039 | 2.588 | 0.026 | 0.025 | 0.026 | 0.025 | |
| | 400 | 0.551 | 0.140 | 0.880 | 0.717 | 0.031 | 0.017 | 0.026 | 0.039 | |
| | 600 | 0.294 | 0.128 | 0.358 | 0.210 | 0.034 | 0.014 | 0.028 | 0.048 | |
| | 800 | 0.151 | 0.128 | 0.149 | 0.124 | 0.036 | 0.013 | 0.031 | 0.048 | |
| | 1000 | 0.117 | 0.115 | 0.117 | 0.110 | 0.034 | 0.014 | 0.033 | 0.048 | |
| | 1200 | 0.118 | 0.105 | 0.114 | 0.102 | 0.036 | 0.015 | 0.031 | 0.047 | |
| | 1400 | 0.099 | 0.092 | 0.102 | 0.096 | 0.034 | 0.016 | 0.033 | 0.045 | |
| | 1600 | 0.118 | 0.086 | 0.097 | 0.092 | 0.034 | 0.015 | 0.034 | 0.044 | |
| | 1800 | 0.095 | 0.078 | 0.092 | 0.081 | 0.033 | 0.016 | 0.033 | 0.043 | |
| | 2000 | 0.082 | 0.076 | 0.081 | 0.077 | 0.033 | 0.016 | 0.033 | 0.044 | |
| | 2200 | 0.083 | 0.065 | 0.076 | 0.071 | 0.034 | 0.016 | 0.033 | 0.042 | |
| | 2400 | 0.072 | 0.057 | 0.067 | 0.071 | 0.033 | 0.017 | 0.033 | 0.041 | |
| | 2600 | 0.062 | 0.058 | 0.068 | 0.064 | 0.032 | 0.019 | 0.034 | 0.040 | |
| | 2800 | 0.062 | 0.055 | 0.057 | 0.058 | 0.033 | 0.018 | 0.034 | 0.041 | |
| | 3000 | 0.057 | 0.048 | 0.054 | 0.052 | 0.034 | 0.018 | 0.034 | 0.039 | |
| | 3200 | 0.054 | 0.049 | 0.050 | 0.042 | 0.034 | 0.019 | 0.034 | 0.038 | |
| | 3400 | 0.048 | 0.046 | 0.047 | 0.043 | 0.034 | 0.019 | 0.035 | 0.038 | |
| | 3600 | 0.041 | 0.04.4 | 0.040 | 0.039 | 0.036 | 0.019 | 0.034 | 0.038 | |
| | 3800 | 0.038 | 0.039 | 0.034 | 0.029 | 0.034 | 0.020 | 0.035 | 0.036 | |
| | 4000 | 0.033 | 0.036 | 0.032 | 0.031 | 0.035 | 0.020 | 0.035 | 0.037 | |
| | 4200 | 0.033 | 0.034 | 0.028 | 0.025 | 0.035 | 0.020 | 0.036 | 0.036 | |
| | 4400 | 0.027 | 0.018 | 0.030 | 0.025 | 0.035 | 0.020 | 0.035 | 0.035 | |
| | 4600 | 0.027 | 0.025 | 0.026 | 0.022 | 0.035 | 0.021 | 0.036 | 0.035 | |
| | 4800 | 0.024 | 0.026 | 0.023 | 0.015 | 0.036 | 0.021 | 0.036 | 0.035 | |
| | 5000 | 0.019 | 0.027 | 0.020 | 0.014 | 0.035 | 0.022 | 0.035 | 0.035 | |

| **Sample** | **Dept h, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 1.1.2 | 0 | 0.045 | 0.036 | 0.027 | 0.026 | 0.035 | 0.011 | 0.043 | 0.053 | Vitamin E |
| | 200 | 0.054 | 0.034 | 0.019 | 0.022 | 0.035 | 0.011 | 0.034 | 0.034 | |
| | 400 | 0.047 | 0.032 | 0.021 | 0.024 | 0.035 | 0.011 | 0.033 | 0.033 | |
| | 600 | 0.046 | 0.028 | 0.021 | 0.024 | 0.035 | 0.011 | 0.033 | 0.032 | |
| | 800 | 0.042 | 0.029 | 0.025 | 0.024 | 0.035 | 0.011 | 0.034 | 0.032 | |
| | 1000 | 0.043 | 0.029 | 0.028 | 0.023 | 0.036 | 0.012 | 0.033 | 0.032 | |
| | 1200 | 0.042 | 0.033 | 0.028 | 0.019 | 0.037 | 0.012 | 0.033 | 0.032 | |
| | 1400 | 0.039 | 0.032 | 0.025 | 0.017 | 0.037 | 0.014 | 0.033 | 0.032 | |
| | 1600 | 0.035 | 0.026 | 0.020 | 0.010 | 0.036 | 0.013 | 0.033 | 0.031 | |
| | 1800 | 0.033 | 0.025 | 0.018 | 0.007 | 0.037 | 0.014 | 0.033 | 0.031 | |
| | 2000 | 0.038 | 0.017 | 0.017 | 0.006 | 0.036 | 0.013 | 0.033 | 0.031 | |
| | 2200 | 0.071 | 0.025 | 0.016 | 0.007 | 0.035 | 0.014 | 0.033 | 0.033 | |
| | 2400 | 0.116 | 0.020 | 0.021 | 0.006 | 0.034 | 0.014 | 0.034 | 0.033 | |
| | 2600 | 0.098 | 0.017 | 0.023 | 0.008 | 0.035 | 0.015 | 0.035 | 0.033 | |
| | 2800 | 0.061 | 0.017 | 0.032 | 0.007 | 0.036 | 0.016 | 0.034 | 0.033 | |
| | 3000 | 0.109 | 0.018 | 0.042 | 0.020 | 0.038 | 0.016 | 0.034 | 0.033 | |
| | 3200 | 0.155 | 0.017 | 0.064 | 0.048 | 0.039 | 0.016 | 0.034 | 0.033 | |
| | 3400 | 0.154 | 0.019 | 0.083 | 0.107 | 0.039 | 0.016 | 0.034 | 0.034 | |
| | 3600 | 0.027 | 0.017 | 0.125 | 0.152 | 0.036 | 0.017 | 0.035 | 0.035 | |
| | 3800 | 0.012 | 0.018 | 0.132 | 0.120 | 0.035 | 0.018 | 0.038 | 0.035 | |
| | 4000 | 0.013 | 0.019 | 0.102 | 0.050 | 0.036 | 0.017 | 0.037 | 0.035 | |
| | 4200 | 0.012 | 0.019 | 0.033 | -0.001 | 0.035 | 0.019 | 0.036 | 0.035 | |
| | 4400 | 0.009 | 0.019 | 0.010 | -0.007 | 0.035 | 0.019 | 0.036 | 0.035 | |
| | 4600 | 0.009 | 0.018 | 0.009 | -0.006 | 0.036 | 0.020 | 0.035 | 0.035 | |
| | 4800 | 0.001 | 0.015 | 0.005 | -0.007 | 0.036 | 0.019 | 0.036 | 0.035 | |
| | 5000 | 0.002 | 0.021 | 0.002 | -0.007 | 0.035 | 0.020 | 0.036 | 0.036 | |

| **Sample** | **Dept** | **Top** | **Btm OI** | **Side 1** | **Side 2** | **Top** | **Btm** | **Side** | **Side 2** | **AO** |
|---|---|---|---|---|---|---|---|---|---|---|
| | **h, µ** | **OI** | | **OI** | **OI** | **TVI** | **TVI** | **1 TVI** | **TVI** | **Treatment** |
| 1.1.3 | 0 | -0.009 | 0.052 | 0.009 | 0.022 | 0.049 | 0.029 | 0.052 | 0.044 | VE-Phosphite |
| | 200 | 0.002 | 0.057 | 0.007 | 0.035 | 0.046 | 0.029 | 0.047 | 0.042 | |
| | 400 | 0.009 | 0.055 | 0.016 | 0.035 | 0.046 | 0.027 | 0.046 | 0.042 | |
| | 600 | 0.008 | 0.040 | 0.022 | 0.034 | 0.046 | 0.025 | 0.046 | 0.041 | |
| | 800 | 0.006 | 0.038 | 0.019 | 0.027 | 0.044 | 0.026 | 0.045 | 0.039 | |
| | 1000 | 0.002 | 0.037 | 0.015 | 0.026 | 0.043 | 0.024 | 0.043 | 0.038 | |
| | 1200 | -0.002 | 0.038 | 0.011 | 0.026 | 0.041 | 0.023 | 0.041 | 0.037 | |
| | 1400 | -0.006 | 0.040 | 0.007 | 0.032 | 0.041 | 0.023 | 0.039 | 0.036 | |
| | 1600 | -0.007 | 0.035 | 0.004 | 0.040 | 0.039 | 0.023 | 0.037 | 0.035 | |
| | 1800 | -0.009 | 0.031 | 0.007 | 0.040 | 0.039 | 0.021 | 0.038 | 0.035 | |
| | 2000 | -0.009 | 0.025 | 0.009 | 0.044 | 0.038 | 0.021 | 0.037 | 0.035 | |
| | 2200 | -0.006 | 0.022 | 0.012 | 0.062 | 0.037 | 0.020 | 0.036 | 0.034 | |
| | 2400 | -0.007 | 0.031 | 0.015 | 0.037 | 0.038 | 0.019 | 0.035 | 0.035 | |
| | 2600 | -0.027 | 0.023 | 0.016 | 0.033 | 0.037 | 0.018 | 0.035 | 0.034 | |
| | 2800 | -0.006 | 0.032 | 0.018 | 0.031 | 0.036 | 0.017 | 0.035 | 0.036 | |
| | 3000 | -0.008 | 0.052 | 0.027 | 0.042 | 0.037 | 0.018 | 0.035 | 0.036 | |
| | 3200 | 0.000 | 0.052 | 0.032 | 0.055 | 0.036 | 0.020 | 0.035 | 0.037 | |
| | 3400 | 0.013 | 0.046 | 0.046 | 0.133 | 0.037 | 0.018 | 0.037 | 0.039 | |
| | 3600 | 0.030 | 0.068 | 0.079 | 0.085 | 0.037 | 0.021 | 0.038 | 0.038 | |
| | 3800 | 0.058 | 0.094 | 0.133 | 0.014 | 0.037 | 0.022 | 0.039 | 0.035 | |
| | 4000 | 0.074 | 0.067 | 0.024 | 0.006 | 0.039 | 0.022 | 0.037 | 0.035 | |
| | 4200 | 0.057 | 0.030 | 0.001 | 0.006 | 0.039 | 0.019 | 0.035 | 0.035 | |
| | 4400 | -0.009 | 0.038 | -0.002 | 0.005 | 0.037 | 0.019 | 0.035 | 0.036 | |
| | 4600 | -0.014 | 0.030 | 0.000 | 0.003 | 0.036 | 0.019 | 0.035 | 0.035 | |
| | 4800 | -0.015 | 0.022 | -0.001 | 0.003 | 0.017 | 0.018 | 0.036 | 0.036 | |
| | 5000 | -0.016 | 0.022 | 0.000 | 0.002 | 0.036 | 0.020 | 0.036 | 0.036 | |

| **Sample** | **Dept h, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 1.1.4 | 0 | 0.022 | 0.041 | 0.019 | 0.036 | 0.068 | 0.030 | 0.052 | 0.056 | VE - Phosphite-R |
| | 200 | 0.027 | 0.042 | 0.022 | 0.036 | 0.056 | 0.030 | 0.049 | 0.049 | |
| | 400 | 0.030 | 0.043 | 0.030 | 0.043 | 0.055 | 0.030 | 0.049 | 0.048 | |
| | 600 | 0.031 | 0.045 | 0.028 | 0.039 | 0.056 | 0.027 | 0.049 | 0.045 | |
| | 800 | 0.028 | 0.050 | 0.026 | 0.037 | 0.053 | 0.027 | 0.047 | 0.044 | |
| | 1000 | 0.022 | 0.046 | 0.027 | 0.030 | 0.051 | 0.027 | 0.045 | 0.042 | |
| | 1200 | 0.016 | 0.047 | 0.021 | 0.028 | 0.049 | 0.026 | 0.044 | 0.041 | |
| | 1400 | 0.012 | 0.040 | 0.018 | 0.023 | 0.046 | 0.024 | 0.042 | 0.038 | |
| | 1600 | 0.011 | 0.042 | 0.015 | 0.022 | 0.044 | 0.023 | 0.040 | 0.038 | |
| | 1800 | 0.008 | 0.041 | 0.012 | 0.022 | 0.043 | 0.022 | 0.039 | 0.037 | |
| | 2000 | 0.012 | 0.039 | 0.016 | 0.021 | 0.041 | 0.020 | 0.038 | 0.036 | |
| | 2200 | 0.010 | 0.039 | 0.012 | 0.019 | 0.039 | 0.017 | 0.037 | 0.035 | |
| | 2400 | 0.011 | 0.038 | 0.012 | 0.018 | 0.038 | 0.019 | 0.036 | 0.036 | |
| | 2600 | 0.009 | 0.041 | 0.009 | 0.019 | 0.038 | 0.018 | 0.037 | 0.035 | |
| | 2800 | 0.007 | 0.041 | 0.009 | 0.020 | 0.037 | 0.017 | 0.036 | 0.035 | |
| | 3000 | 0.006 | 0.062 | 0.010 | 0.022 | 0.036 | 0.018 | 0.036 | 0.035 | |
| | 3200 | 0.006 | 0.086 | 0.014 | 0.024 | 0.037 | 0.019 | 0.037 | 0.034 | |
| | 3400 | 0.007 | 0.104 | 0.021 | 0.022 | 0.036 | 0.022 | 0.037 | 0.036 | |
| | 3600 | 0.005 | 0.279 | 0.017 | 0.022 | 0.037 | 0.026 | 0.037 | 0.034 | |
| | 3800 | 0.005 | 0.055 | 0.030 | 0.037 | 0.036 | 0.020 | 0.038 | 0.036 | |
| | 4000 | 0.011 | 0.025 | 0.086 | 0.257 | 0.037 | 0.017 | 0.039 | 0.037 | |
| | 4200 | 0.013 | 0.023 | 0.240 | 0.199 | 0.036 | 0.018 | 0.039 | 0.038 | |
| | 4400 | 0.017 | 0.026 | 0.027 | 0.026 | 0.037 | 0.018 | 0.038 | 0.036 | |
| | 4600 | 0.069 | 0.028 | 0.002 | 0.018 | 0.038 | 0.019 | 0.037 | 0.035 | |
| | 4800 | 0.079 | 0.029 | 0.004 | 0.020 | 0.017 | 0.019 | 0.037 | 0.035 | |
| | 5000 | 0.054 | 0.022 | 0.000 | 0.024 | 0.038 | 0.019 | 0.037 | 0.036 | |

### Example 2.

Four sections of a GUR 1050 compression molded bar were treated as follows:
Sample 2.1.1: No coating.
Sample 2.1.2: Coated with a solution of 10% vitamin E (VE) dissolved in isopropanol, coated with a solution of 10% vitamin E-phosphite (VEP) dissolved in dichloromethane.
Sample 2.1.3: Coated with a solution of 10% vitamin E-phosphite (VEP)dissolved in dichloromethane.
Sample 2.1.4: Coated with a solution of 10% recovered vitamin E-phosphite (VEP-R) dissolved in dichloromethane.

The recovered VEP was obtained by a secondary cleaning of the glassware used for synthesis of the VEP, which had some increased exposure to oxygen during recovery as compared to the VEP which was maintained under an inert environment until application to the UHMWPE section.

All sections were then heated to 120 °C for 14 hours in air, irradiated warm (120 °C) in air with 75 kGy using a 10 MeV electron beam. After cooling in air, the sections were melted at 150 °C for 14 hours in air, followed by slow cooling (16 hours) to ambient temperature, in air.

The Samples were sectioned, then films were prepared with a microtome from top to bottom of each sectioned form, and from side to side of each sectioned form, and analyzed for trans-vinyl index and oxidation index per ASTM F2381-10 and ASTM F2102-13, respectively.

Table 2 illustrates oxidation index to a depth of 5 mm as measured from the top, bottom, and both sides of Samples 2.1.1-2.1.4.

**Table 2. Oxidation index for Samples 2.1.1-2.1.4.**

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 2.1.1 | 0 | 5.186 | 5.623 | 4.650 | 7.287 | 0.035 | 0.056 | 0.031 | 0.049 | None |
| | 200 | 1.380 | 2.811 | 1.244 | 3.662 | 0.025 | 0.025 | 0.024 | 0.027 | |
| | 400 | 0.520 | 0.899 | 0.463 | 1.099 | 0.029 | 0.019 | 0.029 | 0.024 | |
| | 600 | 0.244 | 0.437 | 0.167 | 0.430 | 0.032 | 0.016 | 0.030 | 0.029 | |
| | 800 | 0.132 | 0.270 | 0.126 | 0.225 | 0.032 | 0.015 | 0.030 | 0.030 | |
| | 1000 | 0.115 | 0.157 | 0.115 | 0.189 | 0.032 | 0.014 | 0.030 | 0.030 | |
| | 1200 | 0.105 | 0.099 | 0.103 | 0.152 | 0.032 | 0.012 | 0.030 | 0.029 | |
| | 1400 | 0.105 | 0.070 | 0.101 | 0.140 | 0.031 | 0.012 | 0.030 | 0.029 | |
| | 1600 | 0.089 | 0.054 | 0.097 | 0.126 | 0.031 | 0.012 | 0.029 | 0.030 | |
| | 1800 | 0.088 | 0.044 | 0.087 | 0.127 | 0.032 | 0.012 | 0.030 | 0.029 | |
| | 2000 | 0.071 | 0.036 | 0.082 | 0.107 | 0.032 | 0.013 | 0.031 | 0.030 | |
| | 2200 | 0.068 | 0.029 | 0.078 | 0.096 | 0.032 | 0.013 | 0.030 | 0.030 | |
| | 2400 | 0.064 | 0.026 | 0.072 | 0.086 | 0.033 | 0.014 | 0.031 | 0.030 | |
| | 2600 | 0.052 | 0.026 | 0.060 | 0.078 | 0.033 | 0.014 | 0.031 | 0.030 | |
| | 2800 | 0.049 | 0.022 | 0.058 | 0.075 | 0.033 | 0.014 | 0.031 | 0.030 | |
| | 3000 | 0.043 | 0.021 | 0.052 | 0.062 | 0.033 | 0.015 | 0.032 | 0.031 | |
| | 3200 | 0.036 | 0.018 | 0.045 | 0.061 | 0.033 | 0.016 | 0.032 | 0.031 | |
| | 3400 | 0.034 | 0.017 | 0.038 | 0.051 | 0.034 | 0.015 | 0.032 | 0.031 | |
| | 3600 | 0.027 | 0.019 | 0.037 | 0.045 | 0.033 | 0.016 | 0.033 | 0.032 | |
| | 3800 | 0.023 | 0.017 | 0.031 | 0.045 | 0.033 | 0.017 | 0.032 | 0.032 | |
| | 4000 | 0.022 | 0.016 | 0.036 | 0.039 | 0.032 | 0.017 | 0.034 | 0.032 | |
| | 4200 | 0.025 | 0.015 | 0.029 | 0.038 | 0.034 | 0.017 | 0.032 | 0.033 | |
| | 4400 | 0.015 | 0.020 | 0.030 | 0.029 | 0.035 | 0.017 | 0.033 | 0.033 | |
| | 4600 | 0.013 | 0.013 | 0.018 | 0.029 | 0.034 | 0.018 | 0.037 | 0.033 | |
| | 4800 | 0.008 | 0.006 | 0.021 | 0.030 | 0.034 | 0.019 | 0.034 | 0.033 | |
| | 5000 | 0.008 | 0.005 | 0.017 | 0.026 | 0.034 | 0.019 | 0.034 | 0.034 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 2.1.2 | 0 | 0.035 | 0.005 | 0.021 | 0.016 | 0.031 | 0.011 | 0.039 | 0.035 | Vitamin E |
| | 200 | 0.040 | 0.002 | 0.018 | 0.014 | 0.032 | 0.011 | 0.034 | 0.032 | |
| | 400 | 0.033 | -0.005 | 0.025 | 0.016 | 0.032 | 0.012 | 0.032 | 0.030 | |
| | 600 | 0.030 | -0.002 | 0.019 | 0.022 | 0.034 | 0.011 | 0.032 | 0.029 | |
| | 800 | 0.031 | 0.003 | 0.022 | 0.022 | 0.034 | 0.012 | 0.031 | 0.031 | |
| | 1000 | 0.026 | 0.004 | 0.027 | 0.023 | 0.034 | 0.012 | 0.031 | 0.031 | |
| | 1200 | 0.013 | 0.006 | 0.020 | 0.015 | 0.034 | 0.012 | 0.031 | 0.030 | |
| | 1400 | 0.021 | 0.010 | 0.015 | 0.014 | 0.034 | 0.014 | 0.032 | 0.031 | |
| | 1600 | 0.015 | 0.010 | 0.011 | 0.011 | 0.034 | 0.014 | 0.031 | 0.030 | |
| | 1800 | 0.015 | 0.009 | 0.009 | 0.009 | 0.034 | 0.015 | 0.031 | 0.030 | |
| | 2000 | 0.012 | 0.005 | 0.006 | 0.009 | 0.034 | 0.015 | 0.031 | 0.030 | |
| | 2200 | 0.017 | 0.004 | 0.010 | 0.010 | 0.035 | 0.015 | 0.033 | 0.031 | |
| | 2400 | 0.024 | 0.000 | 0.011 | 0.008 | 0.035 | 0.015 | 0.030 | 0.031 | |
| | 2600 | 0.035 | 0.001 | 0.008 | 0.012 | 0.035 | 0.016 | 0.032 | 0.031 | |
| | 2800 | 0.045 | 0.003 | 0.009 | 0.014 | 0.036 | 0.016 | 0.032 | 0.032 | |
| | 3000 | 0.055 | 0.001 | 0.016 | 0.014 | 0.035 | 0.016 | 0.033 | 0.032 | |
| | 3200 | 0.043 | -0.002 | 0.011 | 0.021 | 0.036 | 0.017 | 0.034 | 0.033 | |
| | 3400 | 0.053 | -0.002 | 0.017 | 0.026 | 0.036 | 0.017 | 0.034 | 0.033 | |
| | 3600 | 0.105 | 0.002 | 0.051 | 0.084 | 0.038 | 0.018 | 0.034 | 0.034 | |
| | 3800 | 0.066 | -0.001 | 0.195 | 0.160 | 0.037 | 0.018 | 0.035 | 0.036 | |
| | 4000 | 0.014 | 0.001 | 0.284 | 0.066 | 0.035 | 0.019 | 0.034 | 0.037 | |
| | 4200 | 0.008 | 0.000 | 0.016 | 0.010 | 0.034 | 0.019 | 0.034 | 0.033 | |
| | 4400 | 0.004 | -0.001 | 0.003 | 0.005 | 0.034 | 0.020 | 0.034 | 0.033 | |
| | 4600 | 0.003 | 0.000 | -0.001 | 0.005 | 0.034 | 0.021 | 0.034 | 0.033 | |
| | 4800 | 0.007 | 0.001 | -0.001 | 0.000 | 0.035 | 0.021 | 0.034 | 0.034 | |
| | 5000 | 0.005 | 0.003 | -0.007 | 0.001 | 0.035 | 0.023 | 0.034 | 0.033 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 2.1.3 | 0 | 0.019 | 0.015 | 0.077 | 0.069 | 0.064 | 0.021 | 0.044 | 0.064 | VE-Phosphite |
| | 200 | 0.056 | 0.008 | 0.065 | 0.262 | 0.054 | 0.019 | 0.043 | 0.058 | |
| | 400 | 0.052 | 0.011 | 0.042 | 0.702 | 0.053 | 0.020 | 0.043 | 0.049 | |
| | 600 | 0.042 | 0.014 | 0.031 | 0.540 | 0.051 | 0.020 | 0.042 | 0.051 | |
| | 800 | 0.033 | 0.017 | 0.015 | 0.316 | 0.049 | 0.020 | 0.041 | 0.050 | |
| | 1000 | 0.021 | 0.019 | 0.007 | 0.123 | 0.046 | 0.018 | 0.041 | 0.051 | |
| | 1200 | 0.017 | 0.018 | 0.006 | 0.082 | 0.043 | 0.021 | 0.038 | 0.048 | |
| | 1400 | 0.013 | 0.017 | 0.004 | 0.066 | 0.041 | 0.018 | 0.037 | 0.047 | |
| | 1600 | 0.013 | 0.013 | 0.005 | 0.069 | 0.039 | 0.017 | 0.045 | 0.046 | |
| | 1800 | 0.011 | 0.009 | 0.009 | 0.056 | 0.038 | 0.016 | 0.036 | 0.044 | |
| | 2000 | 0.014 | 0.009 | 0.012 | 0.044 | 0.036 | 0.015 | 0.037 | 0.040 | |
| | 2200 | 0.012 | 0.009 | 0.025 | 0.053 | 0.035 | 0.015 | 0.036 | 0.042 | |
| | 2400 | 0.010 | 0.011 | 0.026 | 0.050 | 0.035 | 0.017 | 0.034 | 0.039 | |
| | 2600 | 0.013 | 0.043 | 0.026 | 0.062 | 0.034 | 0.016 | 0.035 | 0.040 | |
| | 2800 | 0.012 | 0.048 | 0.038 | 0.082 | 0.034 | 0.017 | 0.039 | 0.040 | |
| | 3000 | 0.013 | 0.041 | 0.034 | 0.107 | 0.034 | 0.018 | 0.036 | 0.040 | |
| | 3200 | 0.013 | 0.037 | 0.034 | 0.121 | 0.033 | 0.018 | 0.037 | 0.039 | |
| | 3400 | 0.023 | 0.056 | 0.071 | 0.059 | 0.034 | 0.020 | 0.038 | 0.036 | |
| | 3600 | 0.044 | 0.075 | 0.129 | 0.042 | 0.035 | 0.021 | 0.039 | 0.034 | |
| | 3800 | 0.074 | 0.062 | 0.012 | 0.028 | 0.035 | 0.022 | 0.038 | 0.033 | |
| | 4000 | 0.070 | 0.008 | -0.002 | 0.031 | 0.036 | 0.017 | 0.037 | 0.034 | |
| | 4200 | 0.105 | -0.002 | 0.000 | 0.026 | 0.037 | 0.017 | 0.035 | 0.033 | |
| | 4400 | 0.016 | -0.002 | 0.000 | 0.019 | 0.035 | 0.018 | 0.035 | 0.033 | |
| | 4600 | 0.002 | 0.001 | -0.003 | 0.021 | 0.033 | 0.017 | 0.036 | 0.032 | |
| | 4800 | 0.000 | -0.004 | 0.002 | 0.024 | 0.035 | 0.018 | 0.036 | 0.034 | |
| | 5000 | -0.001 | 0.002 | 0.004 | 0.023 | 0.034 | 0.017 | 0.035 | 0.034 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 2.1.4. | 0 | 0.060 | 0.016 | 0.026 | 0.062 | 0.044 | 0.027 | 0.038 | 0.058 | VE-Phosphite-R |
| | 200 | 0.054 | 0.016 | 0.033 | 0.084 | 0.044 | 0.025 | 0.039 | 0.058 | |
| | 400 | 0.033 | 0.014 | 0.032 | 0.060 | 0.044 | 0.024 | 0.039 | 0.058 | |
| | 600 | 0.033 | 0.018 | 0.077 | 0.045 | 0.042 | 0.024 | 0.039 | 0.054 | |
| | 800 | 0.020 | 0.027 | 0.150 | 0.038 | 0.019 | 0.024 | 0.036 | 0.052 | |
| | 1000 | 0.017 | 0.023 | 0.140 | 0.027 | 0.038 | 0.023 | 0.036 | 0.049 | |
| | 1200 | 0.013 | 0.023 | 0.113 | 0.020 | 0.037 | 0.022 | 0.036 | 0.047 | |
| | 1400 | 0.012 | 0.020 | 0.045 | 0.026 | 0.037 | 0.020 | 0.038 | 0.046 | |
| | 1600 | 0.013 | 0.015 | 0.030 | 0.038 | 0.035 | 0.020 | 0.038 | 0.044 | |
| | 1800 | 0.013 | 0.010 | 0.025 | 0.042 | 0.035 | 0.017 | 0.037 | 0.042 | |
| | 2000 | 0.013 | 0.009 | 0.016 | 0.049 | 0.034 | 0.017 | 0.038 | 0.041 | |
| | 2200 | 0.015 | 0.010 | 0.019 | 0.037 | 0.034 | 0.016 | 0.036 | 0.038 | |
| | 2400 | 0.015 | 0.008 | 0.015 | 0.037 | 0.034 | 0.016 | 0.037 | 0.039 | |
| | 2600 | 0.011 | 0.008 | 0.025 | 0.021 | 0.034 | 0.016 | 0.036 | 0.033 | |
| | 2800 | 0.016 | 0.006 | 0.022 | 0.017 | 0.034 | 0.017 | 0.037 | 0.035 | |
| | 3000 | 0.020 | 0.008 | 0.033 | 0.032 | 0.034 | 0.016 | 0.037 | 0.037 | |
| | 3200 | 0.033 | 0.008 | 0.071 | 0.032 | 0.034 | 0.016 | 0.038 | 0.035 | |
| | 3400 | 0.068 | 0.008 | 0.029 | 0.058 | 0.034 | 0.016 | 0.038 | 0.037 | |
| | 3600 | 0.051 | 0.015 | -0.003 | 0.096 | 0.035 | 0.017 | 0.035 | 0.037 | |
| | 3800 | 0.090 | 0.052 | -0.011 | 0.108 | 0.036 | 0.018 | 0.037 | 0.037 | |
| | 4000 | 0.118 | 0.065 | -0.012 | 0.021 | 0.037 | 0.020 | 0.035 | 0.036 | |
| | 4200 | 0.019 | 0.060 | -0.013 | 0.011 | 0.035 | 0.021 | 0.036 | 0.036 | |
| | 4400 | 0.006 | 0.075 | -0.008 | 0.010 | 0.033 | 0.022 | 0.035 | 0.035 | |
| | 4600 | 0.005 | 0.018 | -0.010 | 0.007 | 0.033 | 0.022 | 0.036 | 0.034 | |
| | 4800 | 0.002 | 0.000 | -0.017 | 0.011 | 0.033 | 0.018 | 0.035 | 0.033 | |
| | 5000 | 0.007 | -0.001 | -0.014 | 0.005 | 0.034 | 0.018 | 0.036 | 0.032 | |

### Example 3.

Four sections of a GUR 1050 compression molded bar were treated as follows:
Sample 3.1.1: No coating.
Sample 3.1.2: Coated with a solution of 10% vitamin E (VE) dissolved in isopropanol.
Sample 3.1.3: Coated with a solution of 10% vitamin E-phosphite (VEP) dissolved in dichloromethane.
Sample 3.1.4: Coated with a solution of 10% recovered vitamin E-phosphite (VEP-R) dissolved in dichloromethane.

The recovered VEP was obtained by a secondary cleaning of the glassware used for synthesis of the VEP, which had some increased exposure to oxygen during recovery as compared to the VEP which was maintained under an inert environment until application to the UHMWPE section.

All sections were melted at 150 °C for 14 hours in air, followed by slow cooling (16 hours) to ambient temperature, in air. Following the initial melting treatment, the sections were heated to 120 °C for 14 hours in air, irradiated warm (120 °C) in air with 75 kGy using a 10 MeV electron beam. After cooling in air, the sections were melted at 150 °C for 14 hours in air, followed by slow cooling (16 hours) to ambient temperature, in air.

The Samples were sectioned, then films were prepared with a microtome from top to bottom of each sectioned form, and from side to side of each sectioned form, and analyzed for trans-vinyl index and oxidation index per ASTM F2381-10 and ASTM F2102-13, respectively.

Table 3 illustrates oxidation index to a depth of 5 mm as measured from the top, bottom, and both sides of Samples 3.1.1-3.1.4.

**Table 3. Oxidation index for Samples 3.1.1-3.1.4.**

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 3.1.1 | 0 | 12.338 | 7.652 | 2.421 | 6.425 | 0.046 | 0.048 | 0.081 | 0.044 | None |
| | 200 | 3.431 | 2.954 | 7.004 | 6.990 | 0.030 | 0.019 | 0.047 | 0.040 | |
| | 400 | 1.083 | 1.150 | 3.302 | 2.178 | 0.031 | 0.010 | 0.032 | 0.030 | |
| | 600 | 0.437 | 0.640 | 1.172 | 0.869 | 0.030 | 0.008 | 0.028 | 0.027 | |
| | 800 | 0.258 | 0.404 | 0.463 | 0.506 | 0.028 | 0.006 | 0.027 | 0.028 | |
| | 1000 | 0.214 | 0.312 | 0.308 | 0.376 | 0.029 | 0.005 | 0.028 | 0.028 | |
| | 1200 | 0.180 | 0.240 | 0.253 | 0.271 | 0.035 | 0.005 | 0.028 | 0.027 | |
| | 1400 | 0.157 | 0.207 | 0.246 | 0.201 | 0.030 | 0.004 | 0.028 | 0.027 | |
| | 1600 | 0.150 | 0.178 | 0.254 | 0.161 | 0.029 | 0.004 | 0.028 | 0.027 | |
| | 1800 | 0.155 | 0.163 | 0.215 | 0.139 | 0.030 | 0.004 | 0.028 | 0.027 | |
| | 2000 | 0.132 | 0.143 | 0.190 | 0.117 | 0.030 | 0.005 | 0.029 | 0.027 | |
| | 2200 | 0.126 | 0.135 | 0.185 | 0.102 | 0.030 | 0.004 | 0.029 | 0.026 | |
| | 2400 | 0.121 | 0.134 | 0.170 | 0.097 | 0.030 | 0.005 | 0.030 | 0.028 | |
| | 2600 | 0.105 | 0.124 | 0.158 | 0.087 | 0.031 | 0.005 | 0.029 | 0.029 | |
| | 2800 | 0.111 | 0.115 | 0.151 | 0.082 | 0.031 | 0.007 | 0.030 | 0.030 | |
| | 3000 | 0.099 | 0.106 | 0.140 | 0.073 | 0.028 | 0.003 | 0.030 | 0.029 | |
| | 3200 | 0.104 | 0.103 | 0.132 | 0.066 | 0.032 | 0.006 | 0.032 | 0.030 | |
| | 3400 | 0.095 | 0.099 | 0.123 | 0.059 | 0.031 | 0.006 | 0.031 | 0.030 | |
| | 3600 | 0.088 | 0.085 | 0.125 | 0.043 | 0.032 | 0.007 | 0.031 | 0.030 | |
| | 3800 | 0.087 | 0.083 | 0.118 | 0.043 | 0.032 | 0.009 | 0.032 | 0.031 | |
| | 4000 | 0.076 | 0.074 | 0.122 | 0.035 | 0.032 | 0.008 | 0.032 | 0.031 | |
| | 4200 | 0.068 | 0.073 | 0.106 | 0.020 | 0.032 | 0.008 | 0.032 | 0.031 | |
| | 4400 | 0.063 | 0.072 | 0.098 | 0.022 | 0.032 | 0.006 | 0.033 | 0.032 | |
| | 4600 | 0.051 | 0.069 | 0.096 | 0.017 | 0.032 | 0.009 | 0.032 | 0.031 | |
| | 4800 | 0.047 | 0.069 | 0.087 | 0.014 | 0.032 | 0.011 | 0.032 | 0.032 | |
| | 5000 | 0.047 | 0.062 | 0.091 | 0.005 | 0.033 | 0.009 | 0.034 | 0.032 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 3.1.2 | 0 | 5.981 | 0.760 | 3.147 | 2.090 | 0.046 | 0.009 | 0.085 | 0.020 | Vitamin E |
| | 200 | 1.817 | 0.024 | 4.505 | 0.201 | 0.021 | 0.007 | 0.030 | 0.028 | |
| | 400 | 0.316 | 0.023 | 0.633 | 0.086 | 0.032 | 0.004 | 0.031 | 0.028 | |
| | 600 | 0.126 | 0.026 | 0.174 | 0.058 | 0.034 | 0.007 | 0.039 | 0.030 | |
| | 800 | 0.065 | 0.029 | 0.129 | 0.058 | 0.034 | 0.005 | 0.036 | 0.028 | |
| | 1000 | 0.050 | 0.024 | 0.116 | 0.031 | 0.032 | 0.006 | 0.036 | 0.027 | |
| | 1200 | 0.048 | 0.026 | 0.078 | 0.044 | 0.033 | 0.006 | 0.034 | 0.028 | |
| | 1400 | 0.039 | 0.024 | 0.073 | 0.035 | 0.033 | 0.006 | 0.035 | 0.029 | |
| | 1600 | 0.037 | 0.014 | 0.059 | 0.032 | 0.033 | 0.005 | 0.034 | 0.036 | |
| | 1800 | 0.025 | 0.013 | 0.053 | 0.031 | 0.033 | 0.007 | 0.033 | 0.030 | |
| | 2000 | 0.033 | 0.017 | 0.050 | 0.029 | 0.031 | 0.008 | 0.035 | 0.030 | |
| | 2200 | 0.018 | 0.019 | 0.041 | 0.032 | 0.034 | 0.007 | 0.034 | 0.032 | |
| | 2400 | 0.015 | 0.014 | 0.043 | 0.029 | 0.033 | 0.008 | 0.034 | 0.032 | |
| | 2600 | 0.016 | 0.019 | 0.039 | 0.026 | 0.035 | 0.007 | 0.032 | 0.031 | |
| | 2800 | 0.004 | 0.015 | 0.033 | 0.022 | 0.032 | 0.008 | 0.033 | 0.031 | |
| | 3000 | 0.006 | 0.024 | 0.032 | 0.020 | 0.032 | 0.009 | 0.033 | 0.032 | |
| | 3200 | 0.012 | -0.002 | 0.031 | 0.021 | 0.033 | 0.009 | 0.032 | 0.031 | |
| | 3400 | 0.013 | 0.019 | 0.024 | 0.017 | 0.034 | 0.012 | 0.032 | 0.032 | |
| | 3600 | 0.013 | 0.007 | 0.025 | 0.020 | 0.033 | 0.010 | 0.032 | 0.032 | |
| | 3800 | 0.016 | 0.018 | 0.025 | 0.019 | 0.033 | 0.011 | 0.032 | 0.033 | |
| | 4000 | 0.008 | 0.019 | 0.023 | 0.018 | 0.033 | 0.021 | 0.032 | 0.033 | |
| | 4200 | 0.000 | 0.026 | 0.022 | 0.022 | 0.033 | 0.011 | 0.032 | 0.033 | |
| | 4400 | 0.009 | 0.024 | 0.026 | 0.023 | 0.036 | 0.012 | 0.032 | 0.033 | |
| | 4600 | 0.009 | 0.023 | 0.026 | 0.018 | 0.034 | 0.012 | 0.035 | 0.033 | |
| | 4800 | 0.007 | 0.017 | 0.034 | 0.025 | 0.033 | 0.013 | 0.032 | 0.034 | |
| | 5000 | 0.017 | 0.017 | 0.055 | 0.022 | 0.033 | 0.012 | 0.033 | 0.033 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 3.1.3 | 0 | 0.044 | 6.389 | 10.174 | -0.019 | 0.032 | 0.048 | 0.052 | 0.029 | VE-Phosphite |
| | 200 | 0.035 | 4.050 | 3.421 | -0.024 | 0.033 | 0.044 | 0.038 | 0.028 | |
| | 400 | 0.030 | 1.381 | 1.282 | -0.025 | 0.032 | 0.030 | 0.044 | 0.028 | |
| | 600 | 0.023 | 0.618 | 0.700 | -0.017 | 0.032 | 0.021 | 0.045 | 0.029 | |
| | 800 | 0.021 | 0.346 | 0.907 | -0.005 | 0.035 | 0.015 | 0.046 | 0.030 | |
| | 1000 | 0.022 | 0.275 | 0.736 | 0.011 | 0.033 | 0.014 | 0.046 | 0.029 | |
| | 1200 | 0.018 | 0.259 | 0.440 | 2.696 | 0.033 | 0.014 | 0.043 | 0.037 | |
| | 1400 | 0.017 | 0.262 | 0.324 | 1.661 | 0.033 | 0.014 | 0.041 | 0.043 | |
| | 1600 | 0.020 | 0.288 | 0.267 | 0.393 | 0.032 | 0.015 | 0.040 | 0.035 | |
| | 1800 | 0.019 | 0.299 | 0.221 | 0.211 | 0.033 | 0.016 | 0.039 | 0.032 | |
| | 2000 | 0.023 | 0.295 | 0.228 | 0.210 | 0.033 | 0.016 | 0.037 | 0.032 | |
| | 2200 | 0.023 | 0.259 | 0.238 | 0.122 | 0.032 | 0.015 | 0.038 | 0.031 | |
| | 2400 | 0.037 | 0.187 | 0.255 | 0.172 | 0.032 | 0.013 | 0.036 | 0.033 | |
| | 2600 | 0.040 | 0.156 | 0.219 | 0.011 | 0.032 | 0.011 | 0.036 | 0.030 | |
| | 2800 | 0.126 | 0.134 | 0.255 | -0.030 | 0.032 | 0.011 | 0.034 | 0.029 | |
| | 3000 | 1.000 | 0.118 | 0.256 | -0.035 | 0.035 | 0.010 | 0.034 | 0.029 | |
| | 3200 | 0.402 | 0.114 | 0.241 | -0.044 | 0.036 | 0.011 | 0.033 | 0.029 | |
| | 3400 | 0.238 | 0.102 | 0.246 | -0.040 | 0.036 | 0.010 | 0.032 | 0.029 | |
| | 3600 | 0.060 | 0.096 | 0.232 | -0.046 | 0.034 | 0.011 | 0.032 | 0.030 | |
| | 3800 | 0.042 | 0.095 | 0.244 | -0.045 | 0.035 | 0.011 | 0.031 | 0.031 | |
| | 4000 | 0.027 | 0.087 | 0.237 | -0.044 | 0.035 | 0.012 | 0.031 | 0.031 | |
| | 4200 | 0.017 | 0.087 | 0.222 | -0.045 | 0.034 | 0.011 | 0.031 | 0.032 | |
| | 4400 | 0.010 | 0.078 | 0.250 | -0.041 | 0.034 | 0.011 | 0.031 | 0.032 | |
| | 4600 | 0.005 | 0.069 | 0.241 | -0.039 | 0.034 | 0.013 | 0.030 | 0.031 | |
| | 4800 | 0.005 | 0.073 | 0.230 | -0.044 | 0.034 | 0.011 | 0.030 | 0.031 | |
| | 5000 | 0.000 | 0.061 | 0.200 | -0.042 | 0.034 | 0.012 | 0.031 | 0.032 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 3.1.4 | 0 | 0.079 | 0.078 | 0.091 | 0.059 | 0.047 | 0.015 | 0.032 | 0.055 | VE-Phosphite-R |
| | 200 | 0.057 | 0.067 | 0.053 | 0.060 | 0.035 | 0.015 | 0.030 | 0.053 | |
| | 400 | 0.049 | 0.060 | 0.051 | 0.055 | 0.034 | 0.016 | 0.030 | 0.051 | |
| | 600 | 0.051 | 0.053 | 0.047 | 0.049 | 0.034 | 0.016 | 0.032 | 0.049 | |
| | 800 | 0.052 | 0.050 | 0.044 | 0.050 | 0.035 | 0.015 | 0.031 | 0.048 | |
| | 1000 | 0.048 | 0.046 | 0.040 | 0.048 | 0.036 | 0.014 | 0.032 | 0.045 | |
| | 1200 | 0.047 | 0.045 | 0.046 | 0.046 | 0.035 | 0.014 | 0.032 | 0.043 | |
| | 1400 | 0.045 | 0.049 | 0.043 | 0.047 | 0.034 | 0.013 | 0.032 | 0.042 | |
| | 1600 | 0.044 | 0.059 | 0.039 | 0.064 | 0.034 | 0.013 | 0.032 | 0.042 | |
| | 1800 | 0.044 | 0.204 | 0.042 | 0.114 | 0.036 | 0.017 | 0.033 | 0.041 | |
| | 2000 | 0.039 | 1.610 | 0.052 | 0.389 | 0.036 | 0.046 | 0.034 | 0.041 | |
| | 2200 | 0.037 | 0.572 | 0.079 | 0.411 | 0.034 | 0.031 | 0.034 | 0.039 | |
| | 2400 | 0.041 | 0.341 | 0.665 | 0.240 | 0.034 | 0.023 | 0.039 | 0.037 | |
| | 2600 | 0.045 | 0.306 | 0.410 | 0.207 | 0.034 | 0.026 | 0.036 | 0.037 | |
| | 2800 | 0.041 | 0.229 | 0.258 | 0.289 | 0.035 | 0.019 | 0.035 | 0.039 | |
| | 3000 | 0.046 | 0.065 | 0.286 | 0.142 | 0.036 | 0.012 | 0.036 | 0.037 | |
| | 3200 | 0.043 | 0.032 | 0.202 | 0.052 | 0.033 | 0.009 | 0.037 | 0.035 | |
| | 3400 | 0.047 | 0.018 | 0.041 | 0.037 | 0.035 | 0.010 | 0.034 | 0.035 | |
| | 3600 | 0.569 | 0.023 | 0.027 | 0.035 | 0.037 | 0.009 | 0.034 | 0.036 | |
| | 3800 | 0.461 | 0.018 | 0.041 | 0.029 | 0.038 | 0.009 | 0.035 | 0.035 | |
| | 4000 | 0.097 | 0.021 | 0.085 | 0.033 | 0.034 | 0.009 | 0.033 | 0.035 | |
| | 4200 | 0.030 | 0.020 | 0.068 | 0.084 | 0.033 | 0.009 | 0.035 | 0.034 | |
| | 4400 | 0.022 | 0.018 | 0.082 | 0.155 | 0.037 | 0.011 | 0.038 | 0.034 | |
| | 4600 | 0.031 | 0.011 | 0.031 | 0.094 | 0.037 | 0.011 | 0.037 | 0.035 | |
| | 4800 | 0.138 | 0.015 | 0.010 | 0.033 | 0.035 | 0.011 | 0.036 | 0.035 | |
| | 5000 | 0.075 | 0.015 | 0.004 | 0.016 | 0.035 | 0.011 | 0.036 | 0.036 | |

### Example 4.

Four sections of a GUR 1050 compression molded bar were treated as follows:
Sample 4.1.1: No coating.
Sample 4.1.2: Coated with a solution of 10% vitamin E (VE) dissolved in isopropanol.
Sample 4.1.3: Coated with a solution of 10% vitamin E-phosphite (VEP) dissolved in dichloromethane.
Sample 4.1.4: Coated with a solution of 10% recovered vitamin E-phosphite (VEP-R) dissolved in dichloromethane.

The recovered VEP was obtained by a secondary cleaning of the glassware used for synthesis of the VEP, which had some increased exposure to oxygen during recovery as compared to the VEP which was maintained under an inert environment until application to the UHMWPE section.

All sections were individually placed in a metallized mylar bag under nitrogen purge to prevent oxygen infiltration. The bagged sections were then melted at 150 °C for 14 hours in air, followed by slow cooling (16 hours) to ambient temperature. Following the initial melting treatment, the bagged sections were heated to 120 °C for 14 hours, irradiated warm (120 °C) with 75 kGy using a 10 MeV electron beam. After cooling in the mylar bags purged with nitrogen, the sections were removed from the inert atmosphere and were melted at 150 °C for 14 hours in air, followed by slow cooling (16 hours) to ambient temperature, in air.

The Samples were sectioned, then films were prepared with a microtome from top to bottom of each sectioned form, and from side to side of each sectioned form, and analyzed for trans-vinyl index and oxidation index per ASTM F2381-10 and ASTM F2102-13, respectively.

Table 4 illustrates oxidation index to a depth of 5 mm as measured from the top, bottom, and both sides of Samples 4.1.1-4.1.4.

**Table 4. Oxidation index for Samples 4.1.1-4.1.4.**

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 4.1.1 | 0 | 4.065 | 1.585 | 4.438 | 5.816 | 0.064 | 0.023 | 0.054 | 0.054 | None |
| | 200 | 2.891 | 0.806 | 3.125 | 2.755 | 0.023 | 0.016 | 0.026 | 0.022 | |
| | 400 | 0.863 | 0.551 | 1.150 | 0.885 | 0.025 | 0.014 | 0.023 | 0.024 | |
| | 600 | 0.346 | 0.496 | 0.482 | 0.438 | 0.029 | 0.011 | 0.026 | 0.028 | |
| | 800 | 0.136 | 0.235 | 0.257 | 0.207 | 0.032 | 0.010 | 0.029 | 0.029 | |
| | 1000 | 0.082 | 0.154 | 0.154 | 0.127 | 0.032 | 0.009 | 0.030 | 0.029 | |
| | 1200 | 0.047 | 0.098 | 0.130 | 0.106 | 0.032 | 0.009 | 0.030 | 0.029 | |
| | 1400 | 0.044 | 0.076 | 0.115 | 0.104 | 0.033 | 0.008 | 0.029 | 0.030 | |
| | 1600 | 0.047 | 0.050 | 0.095 | 0.085 | 0.032 | 0.008 | 0.030 | 0.027 | |
| | 1800 | 0.042 | 0.045 | 0.094 | 0.083 | 0.032 | 0.008 | 0.030 | 0.027 | |
| | 2000 | 0.040 | 0.041 | 0.100 | 0.059 | 0.032 | 0.008 | 0.031 | 0.027 | |
| | 2200 | 0.033 | 0.045 | 0.093 | 0.048 | 0.033 | 0.008 | 0.031 | 0.029 | |
| | 2400 | 0.033 | 0.040 | 0.072 | 0.051 | 0.033 | 0.009 | 0.031 | 0.027 | |
| | 2600 | 0.026 | 0.038 | 0.071 | 0.029 | 0.032 | 0.009 | 0.030 | 0.027 | |
| | 2800 | 0.023 | 0.035 | 0.061 | 0.032 | 0.032 | 0.009 | 0.032 | 0.027 | |
| | 3000 | 0.027 | 0.038 | 0.047 | 0.028 | 0.033 | 0.009 | 0.032 | 0.028 | |
| | 3200 | 0.023 | 0.029 | 0.047 | 0.000 | 0.034 | 0.009 | 0.032 | 0.030 | |
| | 3400 | 0.016 | 0.025 | 0.038 | 0.004 | 0.033 | 0.010 | 0.033 | 0.027 | |
| | 3600 | 0.016 | 0.028 | 0.047 | 0.005 | 0.033 | 0.010 | 0.033 | 0.028 | |
| | 3800 | 0.018 | 0.025 | 0.035 | 0.010 | 0.033 | 0.010 | 0.034 | 0.029 | |
| | 4000 | 0.013 | 0.027 | 0.030 | -0.013 | 0.033 | 0.010 | 0.033 | 0.030 | |
| | 4200 | 0.013 | 0.026 | 0.017 | 0.002 | 0.033 | 0.011 | 0.033 | 0.028 | |
| | 4400 | 0.016 | 0.023 | 0.001 | 0.005 | 0.033 | 0.011 | 0.033 | 0.029 | |
| | 4600 | 0.014 | 0.021 | 0.023 | 0.008 | 0.034 | 0.012 | 0.032 | 0.029 | |
| | 4800 | 0.005 | 0.025 | 0.026 | 0.000 | 0.033 | 0.012 | 0.033 | 0.029 | |
| | 5000 | 0.013 | 0.022 | 0.018 | 0.001 | 0.033 | 0.012 | 0.033 | 0.030 | |
| 4.1.2 | 0 | -0.017 | 0.043 | 0.006 | -0.080 | 0.028 | 0.003 | 0.030 | 0.031 | Vitamin E |
| | 200 | -0.030 | 0.034 | 0.018 | -0.079 | 0.035 | 0.004 | 0.025 | 0.033 | |
| | 400 | -0.029 | 0.031 | 0.025 | -0.075 | 0.036 | 0.004 | 0.028 | 0.033 | |
| | 600 | -0.028 | 0.035 | 0.016 | -0.077 | 0.032 | 0.005 | 0.027 | 0.032 | |
| | 800 | -0.034 | 0.030 | 0.017 | -0.070 | 0.030 | 0.005 | 0.029 | 0.031 | |
| | 1000 | -0.016 | 0.021 | -0.006 | -0.062 | 0.034 | 0.004 | 0.025 | 0.033 | |
| | 1200 | -0.028 | 0.033 | -0.010 | -0.065 | 0.033 | 0.005 | 0.025 | 0.032 | |
| | 1400 | -0.019 | 0.027 | -0.025 | -0.062 | 0.034 | 0.005 | 0.027 | 0.034 | |
| | 1600 | -0.040 | 0.021 | -0.031 | -0.059 | 0.031 | 0.006 | 0.027 | 0.032 | |
| | 1800 | -0.052 | 0.021 | -0.023 | -0.046 | 0.031 | 0.006 | 0.027 | 0.032 | |
| | 2000 | -0.063 | 0.017 | -0.027 | -0.055 | 0.032 | 0.005 | 0.026 | 0.032 | |
| | 2200 | -0.051 | 0.023 | -0.038 | -0.045 | 0.031 | 0.006 | 0.026 | 0.032 | |
| | 2400 | -0.043 | 0.019 | -0.027 | -0.054 | 0.034 | 0.006 | 0.028 | 0.032 | |
| | 2600 | -0.037 | 0.021 | -0.022 | -0.070 | 0.037 | 0.006 | 0.028 | 0.032 | |
| | 2800 | -0.047 | 0.022 | -0.024 | -0.065 | 0.034 | 0.008 | 0.028 | 0.032 | |
| | 3000 | -0.028 | 0.021 | -0.034 | -0.068 | 0.035 | 0.007 | 0.032 | 0.033 | |
| | 3200 | -0.025 | 0.022 | -0.039 | -0.059 | 0.033 | 0.007 | 0.030 | 0.032 | |
| | 3400 | -0.032 | 0.019 | -0.038 | -0.078 | 0.034 | 0.008 | 0.029 | 0.032 | |
| | 1600 | -0.032 | 0.027 | -0.037 | -0.062 | 0.033 | 0.008 | 0.029 | 0.032 | |
| | 3800 | -0.033 | 0.025 | -0.023 | -0.045 | 0.036 | 0.008 | 0.028 | 0.033 | |
| | 4000 | -0.053 | 0.021 | -0.027 | -0.069 | 0.034 | 0.008 | 0.029 | 0.031 | |
| | 4200 | -0.042 | 0.021 | -0.036 | -0.051 | 0.034 | 0.009 | 0.030 | 0.031 | |
| | 4400 | -0.051 | 0.023 | -0.026 | -0.079 | 0.036 | 0.010 | 0.030 | 0.031 | |
| | 4600 | -0.039 | 0.020 | -0.037 | -0.082 | 0.035 | 0.010 | 0.030 | 0.032 | |
| | 4800 | -0.034 | 0.018 | -0.024 | -0.087 | 0.035 | 0.010 | 0.030 | 0.031 | |
| | 5000 | -0.032 | 0.020 | -0.034 | -0.087 | 0.035 | 0.010 | 0.028 | 0.032 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 4.1.3 | 0 | 0.013 | -0.039 | 0.058 | -0.002 | 0.037 | 0.019 | 0.031 | 0.039 | VE - Phosphite |
| | 200 | 0.004 | -0.047 | 0.042 | -0.010 | 0.037 | 0.020 | 0.029 | 0.030 | |
| | 400 | 0.002 | -0.029 | 0.047 | -0.005 | 0.036 | 0.020 | 0.030 | 0.031 | |
| | 600 | 0.009 | -0.004 | 0.041 | 0.001 | 0.040 | 0.022 | 0.030 | 0.032 | |
| | 800 | 0.011 | 0.010 | 0.042 | 0.001 | 0.036 | 0.021 | 0.029 | 0.030 | |
| | 1000 | 0.008 | 0.010 | 0.039 | -0.001 | 0.035 | 0.021 | 0.029 | 0.031 | |
| | 1200 | 0.004 | 0.010 | 0.035 | 0.006 | 0.037 | 0.019 | 0.030 | 0.031 | |
| | 1400 | 0.009 | 0.006 | 0.030 | 0.007 | 0.036 | 0.018 | 0.030 | 0.031 | |
| | 1600 | 0.002 | 0.006 | 0.027 | 0.008 | 0.036 | 0.017 | 0.033 | 0.031 | |
| | 1800 | 0.005 | 0.000 | 0.026 | 0.007 | 0.036 | 0.015 | 0.032 | 0.030 | |
| | 2000 | 0.004 | -0.002 | 0.025 | 0.009 | 0.035 | 0.014 | 0.031 | 0.030 | |
| | 2200 | -0.003 | -0.003 | 0.022 | 0.011 | 0.035 | 0.013 | 0.032 | 0.031 | |
| | 2400 | 0.000 | -0.008 | 0.023 | 0.014 | 0.035 | 0.012 | 0.032 | 0.031 | |
| | 2600 | -0.001 | 0.004 | 0.031 | 0.011 | 0.035 | 0.014 | 0.033 | 0.030 | |
| | 2800 | 0.003 | -0.011 | 0.033 | 0.008 | 0.035 | 0.011 | 0.033 | 0.031 | |
| | 3000 | 0.005 | 0.007 | 0.023 | 0.008 | 0.035 | 0.011 | 0.034 | 0.030 | |
| | 3200 | 0.004 | 0.013 | 0.018 | 0.002 | 0.035 | 0.012 | 0.032 | 0.032 | |
| | 3400 | -0.002 | 0.056 | 0.020 | 0.006 | 0.035 | 0.014 | 0.033 | 0.031 | |
| | 1600 | 0.001 | 0.057 | 0.025 | 0.002 | 0.035 | 0.015 | 0.034 | 0.032 | |
| | 3800 | 0.004 | 0.054 | 0.028 | 0.001 | 0.035 | 0.016 | 0.034 | 0.032 | |
| | 4000 | 0.008 | 0.061 | 0.031 | 0.002 | 0.035 | 0.017 | 0.033 | 0.032 | |
| | 4200 | 0.007 | 0.020 | 0.030 | 0.006 | 0.036 | 0.015 | 0.034 | 0.033 | |
| | 4400 | 0.014 | 0.010 | 0.024 | 0.030 | 0.036 | 0.013 | 0.035 | 0.033 | |
| | 4600 | 0.115 | 0.008 | 0.030 | 0.331 | 0.036 | 0.013 | 0.035 | 0.031 | |
| | 4800 | 0.064 | -0.001 | 0.043 | 0.233 | 0.037 | 0.013 | 0.036 | 0.033 | |
| | 5000 | 0.001 | -0.002 | 0.149 | 0.021 | 0.036 | 0.013 | 0.034 | 0.034 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 4.1.4 | 0 | 0.024 | -0.007 | 0.013 | 0.014 | 0.034 | 0.029 | 0.035 | 0.041 | VE - Phosphite-R |
| | 200 | 0.029 | 0.012 | 0.005 | 0.015 | 0.036 | 0.030 | 0.033 | 0.038 | |
| | 400 | 0.023 | 0.011 | 0.006 | 0.003 | 0.036 | 0.030 | 0.031 | 0.035 | |
| | 600 | 0.016 | 0.012 | -0.005 | 0.007 | 0.038 | 0.028 | 0.029 | 0.035 | |
| | 800 | 0.005 | 0.004 | -0.008 | 0.003 | 0.037 | 0.027 | 0.030 | 0.033 | |
| | 1000 | 0.021 | -0.007 | -0.007 | 0.010 | 0.037 | 0.025 | 0.030 | 0.033 | |
| | 1200 | 0.022 | -0.016 | 0.000 | 0.012 | 0.035 | 0.023 | 0.030 | 0.033 | |
| | 1400 | 0.016 | -0.001 | -0.002 | 0.008 | 0.035 | 0.024 | 0.030 | 0.033 | |
| | 1600 | 0.011 | -0.002 | -0.001 | 0.007 | 0.034 | 0.023 | 0.030 | 0.032 | |
| | 1800 | 0.009 | 0.007 | -0.004 | 0.009 | 0.034 | 0.021 | 0.031 | 0.032 | |
| | 2000 | 0.005 | -0.003 | -0.006 | 0.004 | 0.033 | 0.019 | 0.030 | 0.032 | |
| | 2200 | 0.008 | 0.000 | -0.009 | 0.006 | 0.034 | 0.018 | 0.032 | 0.033 | |
| | 2400 | 0.008 | 0.001 | -0.002 | 0.005 | 0.035 | 0.017 | 0.031 | 0.032 | |
| | 2600 | 0.023 | 0.001 | -0.002 | 0.007 | 0.037 | 0.016 | 0.032 | 0.032 | |
| | 2800 | 0.069 | 0.006 | -0.001 | 0.004 | 0.035 | 0.015 | 0.032 | 0.032 | |
| | 3000 | 0.180 | 0.005 | -0.005 | 0.010 | 0.034 | 0.014 | 0.032 | 0.033 | |
| | 3200 | 0.077 | 0.003 | -0.005 | 0.007 | 0.038 | 0.013 | 0.032 | 0.032 | |
| | 3400 | 0.093 | 0.001 | -0.003 | 0.005 | 0.038 | 0.012 | 0.033 | 0.032 | |
| | 3600 | 0.041 | 0.006 | -0.004 | 0.002 | 0.037 | 0.011 | 0.033 | 0.033 | |
| | 3800 | 0.014 | 0.002 | -0.006 | 0.002 | 0.036 | 0.011 | 0.034 | 0.033 | |
| | 4000 | -0.004 | -0.002 | -0.006 | -0.001 | 0.037 | 0.012 | 0.034 | 0.032 | |
| | 4200 | -0.006 | -0.008 | 0.000 | 0.000 | 0.035 | 0.012 | 0.034 | 0.033 | |
| | 4400 | -0.008 | -0.004 | -0.001 | 0.006 | 0.035 | 0.012 | 0.034 | 0.033 | |
| | 4600 | -0.012 | -0.001 | 0.001 | 0.031 | 0.036 | 0.013 | 0.035 | 0.036 | |
| | 4800 | -0.013 | -0.004 | 0.005 | 0.117 | 0.034 | 0.012 | 0.036 | 0.034 | |
| | 5000 | -0.014 | -0.004 | 0.012 | 0.137 | 0.036 | 0.013 | 0.037 | 0.036 | |

### Example 5.

Four sections of a GUR 1050 compression molded bar were treated as follows:
Sample 5.1.1: No coating.
Sample 5.1.2: Coated with a solution of 10% vitamin E (VE) dissolved in isopropanol.
Sample 5.1.3: Coated with a solution of 10% vitamin E-phosphite (VEP) dissolved in dichloromethane.
Sample 5.1.4: Coated with a solution of 10% recovered vitamin E-phosphite (VEP-R) dissolved in dichloromethane.

The recovered VEP was obtained by a secondary cleaning of the glassware used for synthesis of the VEP, which had some increased exposure to oxygen during recovery as compared to the VEP which was maintained under an inert environment until application to the UHMWPE section.

All sections were melted at 150 °C for 14 hours in air, followed by slow cooling (16 hours) to ambient temperature, in air. Following the initial melting treatment, the sections were irradiated in air with 100 kGy using a 10 MeV electron beam. After cooling in air, the sections were melted at 150 °C for 14 hours in air, followed by slow cooling (16 hours) to ambient temperature, in air.

The Samples were sectioned, then films were prepared with a microtome from top to bottom of each sectioned form, and from side to side of each sectioned form, and analyzed for trans-vinyl index and oxidation index per ASTM F2381-10 and ASTM F2102-13, respectively.

Table 5 illustrates oxidation index to a depth of 5 mm as measured from the top, bottom, and both sides of Samples 5.1.1-5.1.4.

**Table 5. Oxidation index for Samples 5.1.1-5.1.4.**

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 5.1.1 | 0 | 8.399 | 4.524 | 9.204 | 7.441 | 0.047 | 0.043 | 0.047 | 0.049 | None |
| | 200 | 3.758 | 2.601 | 3.853 | 5.347 | 0.032 | 0.034 | 0.035 | 0.040 | |
| | 400 | 1.027 | 1.207 | 1.090 | 1.632 | 0.033 | 0.035 | 0.035 | 0.042 | |
| | 600 | 0.512 | 0.624 | 0.547 | 0.789 | 0.034 | 0.037 | 0.036 | 0.046 | |
| | 800 | 0.313 | 0.36:s | 0.331 | 0.496 | 0.034 | 0.036 | 0.036 | 0.046 | |
| | 1000 | 0.318 | 0.236 | 0.331 | 0.293 | 0.037 | 0.036 | 0.036 | 0.046 | |
| | 1200 | 0.276 | 0.172 | 0.303 | 0.245 | 0.036 | 0.038 | 0.036 | 0.045 | |
| | 1400 | 0.181 | 0.137 | 0.218 | 0.202 | 0.036 | 0.037 | 0.037 | 0.045 | |
| | 1600 | 0.151 | 0.119 | 0.186 | 0.190 | 0.035 | 0.038 | 0.035 | 0.044 | |
| | 1800 | 0.137 | 0.103 | 0.162 | 0.178 | 0.036 | 0.038 | 0.034 | 0.043 | |
| | 2000 | 0.124 | 0.084 | 0.142 | 0.165 | 0.036 | 0.037 | 0.035 | 0.043 | |
| | 2200 | 0.112 | 0.074 | 0.123 | 0.152 | 0.036 | 0.038 | 0.034 | 0.042 | |
| | 2400 | 0.114 | 0.063 | 0.111 | 0.141 | 0.036 | 0.038 | 0.033 | 0.041 | |
| | 2600 | 0.092 | 0.055 | 0.118 | 0.146 | 0.036 | 0.040 | 0.033 | 0.040 | |
| | 2800 | 0.087 | 0.039 | 0.110 | 0.133 | 0.035 | 0.039 | 0.032 | 0.040 | |
| | 3000 | 0.077 | 0.037 | 0.105 | 0.126 | 0.036 | 0.038 | 0.033 | 0.039 | |
| | 3200 | 0.079 | 0.029 | 0.097 | 0.109 | 0.036 | 0.038 | 0.032 | 0.038 | |
| | 3400 | 0.052 | 0.021 | 0.091 | 0.113 | 0.036 | 0.038 | 0.033 | 0.038 | |
| | 3600 | 0.063 | 0.009 | 0.092 | 0.097 | 0.035 | 0.038 | 0.032 | 0.038 | |
| | 3800 | 0.064 | -0.004 | 0.088 | 0.089 | 0.037 | 0.039 | 0.033 | 0.037 | |
| | 4000 | 0.052 | -0.001 | 0.079 | 0.087 | 0.036 | 0.040 | 0.032 | 0.037 | |
| | 4200 | 0.055 | -0.010 | 0.072 | 0.079 | 0.035 | 0.039 | 0.033 | 0.037 | |
| | 4400 | 0.053 | -0.010 | 0.066 | 0.075 | 0.036 | 0.040 | 0.033 | 0.037 | |
| | 4600 | 0.046 | -0.004 | 0.062 | 0.075 | 0.037 | 0.039 | 0.033 | 0.036 | |
| | 4800 | 0.043 | -0.002 | 0.062 | 0.075 | 0.037 | 0.040 | 0.032 | 0.036 | |
| | 5000 | 0.042 | -0.010 | 0.060 | 0.071 | 0.036 | 0.040 | 0.033 | 0.036 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 5.1.2 | 0 | 8.242 | 3.529 | 4.698 | 7.104 | 0.053 | 0.080 | 0.030 | 0.053 | Vitamin E |
| | 200 | 1.660 | 3.127 | 0.694 | 2.071 | 0.025 | 0.027 | 0.032 | 0.023 | |
| | 400 | 0.370 | 0.521 | 0.235 | 0.402 | 0.035 | 0.031 | 0.041 | 0.033 | |
| | 600 | 0.204 | 0.230 | 0.179 | 0.212 | 0.035 | 0.036 | 0.036 | 0.037 | |
| | 800 | 0.166 | 0.150 | 0.162 | 0.128 | 0.036 | 0.038 | 0.051 | 0.037 | |
| | 1000 | 0.136 | 0.121 | 0.167 | 0.120 | 0.036 | 0.038 | 0.036 | 0.038 | |
| | 1200 | 0.119 | 0.108 | 0.161 | 0.102 | 0.036 | 0.038 | 0.036 | 0.042 | |
| | 1400 | 0.106 | 0.095 | 0.121 | 0.079 | 0.036 | 0.039 | 0.036 | 0.037 | |
| | 1600 | 0.095 | 0.088 | 0.103 | 0.075 | 0.037 | 0.038 | 0.035 | 0.037 | |
| | 1800 | 0.087 | 0.071 | 0.108 | 0.069 | 0.070 | 0.037 | 0.036 | 0.038 | |
| | 2000 | 0.075 | 0.066 | 0.106 | 0.060 | 0.037 | 0.038 | 0.037 | 0.037 | |
| | 2200 | 0.071 | 0.062 | 0.090 | 0.055 | 0.036 | 0.037 | 0.037 | 0.039 | |
| | 2400 | 0.060 | 0.054 | 0.078 | 0.048 | 0.037 | 0.037 | 0.036 | 0.038 | |
| | 2600 | 0.054 | 0.048 | 0.077 | 0.043 | 0.037 | 0.038 | 0.036 | 0.038 | |
| | 2800 | 0.046 | 0.038 | 0.070 | 0.037 | 0.036 | 0.037 | 0.036 | 0.038 | |
| | 3000 | 0.041 | 0.033 | 0.068 | 0.030 | 0.037 | 0.041 | 0.036 | 0.038 | |
| | 3200 | 0.040 | 0.027 | 0.066 | 0.018 | 0.038 | 0.038 | 0.038 | 0.038 | |
| | 3400 | 0.041 | 0.019 | 0.065 | 0.019 | 0.037 | 0.038 | 0.035 | 0.038 | |
| | 3600 | 0.038 | 0.014 | 0.068 | 0.016 | 0.038 | 0.038 | 0.037 | 0.039 | |
| | 3800 | 0.040 | 0.009 | 0.071 | 0.012 | 0.038 | 0.038 | 0.037 | 0.039 | |
| | 4000 | 0.043 | 0.008 | 0.065 | 0.007 | 0.038 | 0.040 | 0.039 | 0.040 | |
| | 4200 | 0.051 | 0.011 | 0.069 | 0.003 | 0.037 | 0.038 | 0.037 | 0.038 | |
| | 4400 | 0.055 | 0.011 | 0.073 | 0.004 | 0.037 | 0.037 | 0.038 | 0.038 | |
| | 4600 | 0.047 | 0.000 | 0.065 | 0.003 | 0.038 | 0.039 | 0.038 | 0.038 | |
| | 4800 | 0.045 | -0.009 | 0.062 | 0.000 | 0.038 | 0.039 | 0.038 | 0.038 | |
| | 5000 | 0.042 | 0.006 | 0.058 | -0.004 | 0.038 | 0.039 | 0.038 | 0.038 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 5.1.3 | 0 | 5.334 | -0.009 | 0.175 | -0.015 | 0.047 | 0.031 | 0.067 | 0.043 | VE-Phosphite |
| | 200 | 2.343 | -0.013 | 0.037 | -0.019 | 0.032 | 0.031 | 0.054 | 0.045 | |
| | 400 | 0.752 | -0.013 | 0.027 | -0.016 | 0.041 | 0.030 | 0.054 | 0.043 | |
| | 600 | 0.493 | -0.018 | 0.024 | -0.020 | 0.043 | 0.029 | 0.053 | 0.036 | |
| | 800 | 0.385 | -0.013 | 0.011 | -0.019 | 0.044 | 0.029 | 0.051 | 0.036 | |
| | 1000 | 0.392 | -0.021 | 0.005 | -0.024 | 0.044 | 0.029 | 0.047 | 0.034 | |
| | 1200 | 0.477 | -0.024 | 0.012 | -0.024 | 0.043 | 0.033 | 0.048 | 0.033 | |
| | 1400 | 0.550 | -0.025 | 0.006 | -0.033 | 0.049 | 0.031 | 0.050 | 0.035 | |
| | 1600 | 0.433 | -0.030 | 0.016 | -0.037 | 0.044 | 0.029 | 0.047 | 0.034 | |
| | 1800 | 0.248 | -0.024 | 0.015 | -0.010 | 0.042 | 0.032 | 0.047 | 0.035 | |
| | 2000 | 0.221 | -0.029 | 0.032 | -0.003 | 0.042 | 0.032 | 0.048 | 0.034 | |
| | 2200 | 0.192 | -0.035 | 0.042 | -0.001 | 0.042 | 0.032 | 0.048 | 0.036 | |
| | 2400 | 0.249 | -0.030 | 0.540 | 0.167 | 0.044 | 0.032 | 0.048 | 0.033 | |
| | 2600 | 0.098 | -0.041 | 0.499 | 0.325 | 0.043 | 0.034 | 0.046 | 0.035 | |
| | 2800 | 0.029 | -0.033 | 0.233 | 0.291 | 0.043 | 0.032 | 0.046 | 0.036 | |
| | 3000 | 0.030 | -0.036 | 0.217 | 0.160 | 0.046 | 0.030 | 0.045 | 0.036 | |
| | 3200 | 0.025 | -0.034 | 0.290 | 0.011 | 0.042 | 0.032 | 0.048 | 0.033 | |
| | 3400 | 0.025 | -0.035 | 0.064 | -0.001 | 0.042 | 0.031 | 0.044 | 0.034 | |
| | 3600 | 0.028 | -0.041 | 0.415 | -0.015 | 0.041 | 0.031 | 0.039 | 0.032 | |
| | 3800 | 0.045 | -0.033 | 0.127 | -0.018 | 0.042 | 0.030 | 0.039 | 0.031 | |
| | 4000 | 0.021 | -0.034 | 0.072 | 0.036 | 0.043 | 0.032 | 0.041 | 0.032 | |
| | 4200 | 0.021 | -0.043 | 0.073 | 0.093 | 0.043 | 0.033 | 0.042 | 0.033 | |
| | 4400 | 0.019 | -0.041 | 0.053 | 0.023 | 0.043 | 0.032 | 0.041 | 0.034 | |
| | 4600 | 0.027 | -0.035 | 0.004 | -0.025 | 0.043 | 0.032 | 0.040 | 0.032 | |
| | 4800 | 0.015 | -0.034 | -0.009 | -0.029 | 0.040 | 0.031 | 0.041 | 0.033 | |
| | 5000 | 0.018 | -0.034 | -0.010 | -0.034 | 0.044 | 0.030 | 0.040 | 0.032 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 5.1.4 | 0 | 0.053 | -0.026 | 0.147 | 0.065 | 0.040 | 0.028 | 0.062 | 0.051 | VE-Phosphite-R |
| | 200 | 0.041 | -0.054 | 0.002 | 0.008 | 0.036 | 0.023 | 0.047 | 0.049 | |
| | 400 | 0.034 | -0.043 | 0.002 | 0.000 | 0.036 | 0.024 | 0.047 | 0.047 | |
| | 600 | 0.017 | -0.033 | -0.005 | -0.017 | 0.037 | 0.027 | 0.046 | 0.046 | |
| | 800 | 0.016 | -0.031 | -0.022 | -0.029 | 0.036 | 0.028 | 0.044 | 0.045 | |
| | 1000 | 0.021 | -0.030 | -0.027 | -0.035 | 0.037 | 0.027 | 0.043 | 0.044 | |
| | 1200 | 0.017 | -0.032 | -0.026 | -0.040 | 0.037 | 0.028 | 0.042 | 0.042 | |
| | 1400 | 0.013 | -0.033 | -0.029 | -0.041 | 0.037 | 0.027 | 0.041 | 0.042 | |
| | 1600 | 0.023 | -0.035 | -0.035 | -0.041 | 0.040 | 0.030 | 0.041 | 0.042 | |
| | 1800 | 0.022 | -0.043 | -0.034 | -0.037 | 0.038 | 0.027 | 0.038 | 0.041 | |
| | 2000 | 0.147 | -0.042 | -0.025 | -0.025 | 0.039 | 0.027 | 0.039 | 0.041 | |
| | 2200 | 0.539 | -0.038 | -0.023 | -0.025 | 0.042 | 0.028 | 0.039 | 0.041 | |
| | 2400 | 0.345 | -0.042 | -0.020 | -0.020 | 0.041 | 0.028 | 0.037 | 0.042 | |
| | 2600 | 0.460 | -0.037 | -0.003 | 0.066 | 0.043 | 0.028 | 0.038 | 0.041 | |
| | 2800 | 0.296 | -0.047 | 0.117 | 1.216 | 0.043 | 0.028 | 0.038 | 0.037 | |
| | 3000 | 0.059 | -0.045 | 0.430 | 0.398 | 0.038 | 0.028 | 0.037 | 0.039 | |
| | 3200 | 0.005 | -0.036 | 0.298 | 0.285 | 0.037 | 0.029 | 0.037 | 0.041 | |
| | 3400 | -0.001 | -0.043 | 0.295 | 0.126 | 0.037 | 0.028 | 0.040 | 0.041 | |
| | 3600 | 0.000 | -0.044 | 0.021 | -0.001 | 0.037 | 0.029 | 0.035 | 0.038 | |
| | 3800 | 0.001 | -0.048 | -0.020 | -0.010 | 0.038 | 0.027 | 0.034 | 0.037 | |
| | 4000 | 0.072 | -0.041 | -0.008 | -0.004 | 0.037 | 0.030 | 0.033 | 0.038 | |
| | 4200 | 0.209 | -0.049 | 0.056 | -0.011 | 0.035 | 0.033 | 0.031 | 0.037 | |
| | 4400 | 0.037 | -0.043 | 0.077 | -0.034 | 0.039 | 0.029 | 0.032 | 0.036 | |
| | 4600 | -0.001 | -0.046 | 0.023 | -0.046 | 0.038 | 0.029 | 0.034 | 0.035 | |
| | 4800 | -0.007 | -0.070 | -0.028 | -0.049 | 0.038 | 0.032 | 0.033 | 0.034 | |
| | 5000 | -0.009 | -0.047 | -0.044 | -0.052 | 0.037 | 0.029 | 0.032 | 0.034 | |

### Example 6.

Four sections of a GUR 1050 compression molded bar were treated as follows:
Sample 6.1.1: No coating.
Sample 6.1.2: Coated with a solution of 10% vitamin E (VE) dissolved in isopropanol.
Sample 6.1.3: Coated with a solution of 10% vitamin E-phosphite (VEP) dissolved in dichloromethane.
Sample 6.1.4: Coated with a solution of 10% recovered vitamin E-phosphite (VEP-R) dissolved in dichloromethane.

The recovered VEP was obtained by a secondary cleaning of the glassware used for synthesis of the VEP, which had some increased exposure to oxygen during recovery as compared to the VEP which was maintained under an inert environment until application to the UHMWPE section.

All sections were individually placed in a metallized mylar bag under nitrogen purge to prevent oxygen infiltration. The bagged sections were then melted at 150 °C for 14 hours in air, followed by slow cooling (16 hours) to ambient temperature. Following the initial melting treatment, the bagged sections were irradiated with 100 kGy using a 10 MeV electron beam. After cooling in the mylar bags purged with nitrogen, the sections were removed from the inert atmosphere and were melted at 150 °C for 14 hours in air, followed by slow cooling (16 hours) to ambient temperature, in air.

The Samples were sectioned, then films were prepared with a microtome from top to bottom of each sectioned form, and from side to side of each sectioned form, and analyzed for trans-vinyl index and oxidation index per ASTM F2381-10 and ASTM F2102-13, respectively.

Table 6 illustrates oxidation index to a depth of 5 mm as measured from the top, bottom, and both sides of Samples 6.1.1-6.1.4.

**Table 6. Oxidation index for Samples 6.1.1-6.1.4.**

| **Sample** | **Depth, A** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 6.1.1 | 0 | 5.614 | 4.718 | 2.477 | 0.769 | 0.037 | 0.063 | 0.097 | 0.037 | None |
| | 200 | 1.277 | 2.597 | 2.026 | 0.365 | 0.020 | 0.026 | 0.035 | 0.035 | |
| | 400 | 0.531 | 0.847 | 0.747 | 0.159 | 0.029 | 0.029 | 0.038 | 0.039 | |
| | 600 | 0.201 | 0.377 | 0.331 | 0.112 | 0.036 | 0.035 | 0.043 | 0.039 | |
| | 800 | 0.162 | 0.150 | 0.163 | 0.095 | 0.037 | 0.039 | 0.047 | 0.039 | |
| | 1000 | 0.118 | 0.097 | 0.113 | 0.083 | 0.036 | 0.039 | 0.046 | 0.039 | |
| | 1200 | 0.127 | 0.082 | 0.090 | 0.078 | 0.037 | 0.041 | 0.045 | 0.038 | |
| | 1400 | 0.099 | 0.066 | 0.090 | 0.071 | 0.035 | 0.039 | 0.045 | 0.037 | |
| | 1600 | 0.099 | 0.064 | 0.081 | 0.066 | 0.038 | 0.039 | 0.045 | 0.037 | |
| | 1800 | 0.089 | 0.045 | 0.075 | 0.059 | 0.039 | 0.039 | 0.044 | 0.037 | |
| | 2000 | 0.077 | 0.057 | 0.073 | 0.050 | 0.037 | 0.038 | 0.044 | 0.037 | |
| | 2200 | 0.071 | 0.048 | 0.063 | 0.040 | 0.037 | 0.039 | 0.042 | 0.036 | |
| | 2400 | 0.069 | 0.044 | 0.053 | 0.031 | 0.036 | 0.040 | 0.042 | 0.037 | |
| | 2600 | 0.054 | 0.035 | 0.047 | 0.027 | 0.037 | 0.040 | 0.042 | 0.036 | |
| | 2800 | 0.062 | 0.030 | 0.041 | 0.017 | 0.037 | 0.039 | 0.042 | 0.036 | |
| | 3000 | 0.047 | 0.022 | 0.037 | 0.021 | 0.037 | 0.039 | 0.040 | 0.035 | |
| | 3200 | 0.037 | 0.023 | 0.035 | 0.016 | 0.037 | 0.046 | 0.040 | 0.036 | |
| | 3400 | 0.039 | 0.010 | 0.028 | 0.009 | 0.038 | 0.039 | 0.040 | 0.036 | |
| | 3600 | 0.036 | 0.010 | 0.020 | 0.007 | 0.038 | 0.041 | 0.040 | 0.036 | |
| | 3800 | 0.037 | 0.000 | 0.018 | 0.002 | 0.038 | 0.041 | 0.040 | 0.035 | |
| | 4000 | 0.034 | 0.006 | 0.009 | -0.002 | 0.039 | 0.041 | 0.039 | 0.034 | |
| | 4200 | 0.029 | -0.001 | 0.010 | -0.003 | 0.038 | 0.039 | 0.039 | 0.035 | |
| | 4400 | 0.014 | -0.004 | 0.003 | -0.003 | 0.038 | 0.039 | 0.039 | 0.034 | |
| | 4600 | 0.023 | -0.013 | 0.002 | -0.010 | 0.038 | 0.039 | 0.039 | 0.035 | |
| | 4800 | 0.021 | -0.004 | -0.001 | -0.015 | 0.039 | 0.040 | 0.039 | 0.034 | |
| | 5000 | 0.016 | -0.008 | -0.004 | -0.009 | 0.038 | 0.040 | 0.039 | 0.035 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 6.1.2 | 0 | 11.339 | 3.188 | 0.039 | 0.067 | 0.061 | 0.111 | 0.041 | 0.057 | Vitamin E |
| | 200 | 6.656 | 1.836 | 0.038 | 0.059 | 0.065 | 0.045 | 0.042 | 0.054 | |
| | 400 | 4.394 | 0.275 | 0.014 | 0.044 | 0.033 | 0.042 | 0.040 | 0.052 | |
| | 600 | 0.711 | 0.162 | 0.004 | 0.030 | 0.031 | 0.042 | 0.039 | 0.050 | |
| | 800 | 0.279 | 0.128 | -0.006 | 0.019 | 0.038 | 0.042 | 0.038 | 0.048 | |
| | 1000 | 0.189 | 0.103 | -0.010 | 0.012 | 0.039 | 0.043 | 0.036 | 0.048 | |
| | 1200 | 0.158 | 0.077 | -0.013 | 0.006 | 0.039 | 0.041 | 0.036 | 0.046 | |
| | 1400 | 0.120 | 0.061 | -0.015 | 0.010 | 0.039 | 0.042 | 0.035 | 0.046 | |
| | 1600 | 0.104 | 0.052 | -0.022 | 0.004 | 0.038 | 0.041 | 0.034 | 0.044 | |
| | 1800 | 0.090 | 0.044 | -0.017 | 0.004 | 0.039 | 0.042 | 0.034 | 0.043 | |
| | 2000 | 0.077 | 0.018 | -0.019 | 0.004 | 0.038 | 0.041 | 0.034 | 0.044 | |
| | 2200 | 0.072 | 0.023 | -0.041 | 0.002 | 0.039 | 0.040 | 0.033 | 0.044 | |
| | 2400 | 0.067 | 0.024 | -0.022 | 0.002 | 0.037 | 0.041 | 0.034 | 0.042 | |
| | 2600 | 0.058 | 0.017 | -0.022 | 0.001 | 0.038 | 0.041 | 0.033 | 0.042 | |
| | 2800 | 0.054 | 0.012 | -0.019 | 0.008 | 0.038 | 0.042 | 0.032 | 0.042 | |
| | 3000 | 0.047 | 0.011 | -0.017 | 0.014 | 0.037 | 0.042 | 0.032 | 0.041 | |
| | 3200 | 0.048 | 0.007 | -0.020 | 0.041 | 0.038 | 0.041 | 0.032 | 0.041 | |
| | 3400 | 0.034 | 0.004 | -0.019 | 0.091 | 0.037 | 0.041 | 0.032 | 0.039 | |
| | 3600 | 0.034 | 0.000 | -0.014 | 0.101 | 0.037 | 0.041 | 0.032 | 0.039 | |
| | 3800 | 0.033 | -0.002 | -0.005 | 0.086 | 0.038 | 0.041 | 0.032 | 0.041 | |
| | 4000 | 0.016 | -0.009 | 0.047 | 0.086 | 0.038 | 0.042 | 0.031 | 0.042 | |
| | 4200 | 0.027 | -0.005 | 0.057 | 0.028 | 0.038 | 0.042 | 0.032 | 0.041 | |
| | 4400 | 0.002 | -0.009 | 0.095 | 0.011 | 0.040 | 0.042 | 0.034 | 0.041 | |
| | 4600 | 0.016 | -0.010 | -0.008 | 0.003 | 0.038 | 0.042 | 0.033 | 0.039 | |
| | 4800 | 0.007 | -0.014 | -0.023 | -0.008 | 0.037 | 0.043 | 0.031 | 0.039 | |
| | 5000 | -0.001 | -0.009 | -0.032 | -0.004 | 0.039 | 0.043 | 0.032 | 0.038 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 6.1.3 | 0 | 0.066 | 0.029 | 2.329 | 2.905 | 0.044 | 0.040 | 0.146 | 0.028 | VE-Phosphite |
| | 200 | 0.046 | 0.010 | 5.314 | 0.510 | 0.034 | 0.032 | 0.037 | 0.040 | |
| | 400 | 0.046 | 0.041 | 0.911 | 0.261 | 0.038 | 0.038 | 0.034 | 0.049 | |
| | 600 | 0.036 | 0.030 | 0.274 | 0.148 | 0.036 | 0.039 | 0.044 | 0.051 | |
| | 800 | 0.028 | 0.026 | 0.159 | 0.128 | 0.038 | 0.040 | 0.044 | 0.050 | |
| | 1000 | 0.033 | 0.023 | 0.113 | 0.110 | 0.038 | 0.038 | 0.044 | 0.050 | |
| | 1200 | 0.032 | 0.021 | 0.098 | 0.095 | 0.036 | 0.040 | 0.043 | 0.049 | |
| | 1400 | 0.027 | 0.016 | 0.081 | 0.078 | 0.037 | 0.038 | 0.043 | 0.048 | |
| | 1600 | 0.027 | 0.011 | 0.074 | 0.072 | 0.036 | 0.038 | 0.044 | 0.048 | |
| | 1800 | 0.025 | 0.012 | 0.058 | 0.067 | 0.036 | 0.038 | 0.042 | 0.048 | |
| | 2000 | 0.023 | 0.010 | 0.057 | 0.056 | 0.035 | 0.041 | 0.042 | 0.047 | |
| | 2200 | 0.020 | 0.018 | 0.042 | 0.043 | 0.037 | 0.040 | 0.041 | 0.047 | |
| | 2400 | 0.018 | 0.021 | 0.034 | 0.021 | 0.037 | 0.040 | 0.040 | 0.046 | |
| | 2600 | 0.018 | 0.052 | 0.031 | 0.029 | 0.037 | 0.039 | 0.041 | 0.047 | |
| | 2800 | 0.011 | 0.056 | 0.026 | 0.029 | 0.038 | 0.041 | 0.040 | 0.046 | |
| | 3000 | 0.012 | 0.051 | 0.018 | 0.017 | 0.038 | 0.042 | 0.040 | 0.045 | |
| | 3200 | 0.014 | 0.037 | 0.014 | 0.017 | 0.037 | 0.044 | 0.040 | 0.044 | |
| | 3400 | 0.019 | 0.101 | 0.009 | 0.009 | 0.038 | 0.043 | 0.039 | 0.045 | |
| | 3600 | 0.038 | 0.040 | 0.012 | 0.005 | 0.038 | 0.043 | 0.038 | 0.044 | |
| | 3800 | 0.076 | 0.009 | 0.011 | 0.005 | 0.036 | 0.043 | 0.039 | 0.045 | |
| | 4000 | 0.066 | -0.001 | 0.007 | -0.002 | 0.039 | 0.040 | 0.039 | 0.045 | |
| | 4200 | 0.101 | -0.006 | 0.015 | 0.001 | 0.038 | 0.042 | 0.038 | 0.044 | |
| | 4400 | 0.054 | -0.006 | 0.014 | -0.007 | 0.040 | 0.040 | 0.039 | 0.044 | |
| | 4600 | 0.016 | -0.015 | 0.016 | -0.004 | 0.039 | 0.041 | 0.038 | 0.044 | |
| | 4800 | 0.012 | -0.012 | 0.008 | -0.008 | 0.040 | 0.042 | 0.039 | 0.044 | |
| | 5000 | 0.007 | -0.011 | -0.002 | -0.009 | 0.039 | 0.041 | 0.038 | 0.043 | |

| **Sample** | **Depth, µ** | **Top OI** | **Btm OI** | **Side 1 OI** | **Side 2 OI** | **Top TVI** | **Btm TVI** | **Side 1 TVI** | **Side 2 TVI** | **AO Treatment** |
|---|---|---|---|---|---|---|---|---|---|---|
| 6.1.4 | 0 | 0.114 | 0.023 | 0.087 | 0.029 | 0.043 | 0.036 | 0.057 | 0.051 | VE-Phosphite-R |
| | 200 | 0.098 | 0.051 | 0.042 | 0.021 | 0.035 | 0.036 | 0.049 | 0.048 | |
| | 400 | 0.064 | 0.045 | 0.035 | 0.004 | 0.037 | 0.038 | 0.048 | 0.046 | |
| | 600 | 0.050 | 0.037 | 0.024 | -0.003 | 0.038 | 0.036 | 0.048 | 0.045 | |
| | 800 | 0.045 | 0.036 | 0.017 | -0.013 | 0.037 | 0.036 | 0.049 | 0.047 | |
| | 1000 | 0.044 | 0.035 | 0.002 | -0.032 | 0.036 | 0.038 | 0.048 | 0.043 | |
| | 1200 | 0.031 | 0.024 | -0.003 | -0.015 | 0.036 | 0.037 | 0.046 | 0.044 | |
| | 1400 | 0.031 | 0.025 | 0.008 | -0.029 | 0.036 | 0.037 | 0.046 | 0.044 | |
| | 1600 | 0.030 | 0.023 | -0.001 | -0.027 | 0.036 | 0.037 | 0.044 | 0.043 | |
| | 1800 | 0.028 | 0.023 | 0.003 | -0.023 | 0.036 | 0.037 | 0.045 | 0.041 | |
| | 2000 | 0.023 | 0.018 | -0.006 | -0.021 | 0.036 | 0.038 | 0.044 | 0.042 | |
| | 2200 | 0.027 | 0.012 | -0.010 | -0.027 | 0.035 | 0.038 | 0.044 | 0.041 | |
| | 2400 | 0.023 | 0.013 | -0.012 | -0.022 | 0.034 | 0.048 | 0.042 | 0.040 | |
| | 2600 | 0.021 | 0.015 | -0.012 | -0.023 | 0.036 | 0.039 | 0.042 | 0.042 | |
| | 2800 | 0.017 | 0.014 | -0.015 | -0.019 | 0.038 | 0.040 | 0.042 | 0.040 | |
| | 3000 | 0.018 | 0.016 | -0.010 | -0.023 | 0.038 | 0.038 | 0.042 | 0.040 | |
| | 3200 | 0.019 | 0.013 | -0.012 | -0.017 | 0.037 | 0.039 | 0.041 | 0.039 | |
| | 3400 | 0.018 | 0.028 | -0.009 | -0.016 | 0.037 | 0.040 | 0.041 | 0.039 | |
| | 3600 | 0.016 | 0.105 | -0.008 | -0.016 | 0.038 | 0.038 | 0.041 | 0.039 | |
| | 3800 | 0.012 | 0.319 | -0.012 | -0.014 | 0.038 | 0.036 | 0.041 | 0.039 | |
| | 4000 | 0.015 | 0.218 | -0.013 | -0.010 | 0.038 | 0.042 | 0.041 | 0.039 | |
| | 4200 | 0.009 | 0.036 | -0.013 | 0.001 | 0.039 | 0.042 | 0.041 | 0.039 | |
| | 4400 | 0.019 | 0.001 | -0.023 | 0.317 | 0.040 | 0.040 | 0.039 | 0.035 | |
| | 4600 | 0.027 | -0.003 | -0.020 | 0.328 | 0.038 | 0.039 | 0.040 | 0.038 | |
| | 4800 | 0.188 | -0.007 | -0.012 | 0.027 | 0.035 | 0.040 | 0.041 | 0.040 | |
| | 5000 | 0.201 | -0.009 | -0.011 | 0.000 | 0.038 | 0.040 | 0.041 | 0.040 | |

Table 7 illustrates average oxidation index and average trans vinyl index, along with standard deviations, for all of the samples in Examples 1-6.

**Table 7. Average oxidation index and average trans vinyl index for Example 1-6, where "TB" indicates top to bottom, and where "SS" indicates side to side.**

| **Sample** | **Area** | **N** | **ASTM OI** *(Peak Ratio: 1765-1680*/ *1392-1330)* | | **ASTM TVI:** (Peak Ratio: 980-947/ 1392-1330) | |
|---|---|---|---|---|---|---|
| | | | **All Data** | | **All Data** | |
| | | | **Avg OI** | **SD OI** | **Avg TVI** | **SD TVI** |
| 1.1.1 | TB | 253 | 0.0479 | 0.3551 | 0.0336 | 0.0067 |
| | SS | 201 | 0.1198 | 0.7170 | 0.0367 | 0.0035 |
| 1.1.2 | TB | 262 | 0.0087 | 0.0216 | 0.0346 | 0.0080 |
| | SS | 420 | 0.0012 | 0.0186 | 0.0391 | 0.0024 |
| 1.1.3 | TB | 260 | 0.0054 | 0.0186 | 0.0356 | 0.0069 |
| | SS | 312 | 0.0055 | 0.0153 | 0.0400 | 0.0022 |
| 1.1.4 | TB | 258 | 0.0100 | 0.0251 | 0.0358 | 0.0074 |
| | SS | 293 | 0.0085 | 0.0262 | 0.0406 | 0.0027 |
| 2.1.1 | TB | 253 | 0.0769 | 0.5209 | 0.0335 | 0.0076 |
| | SS | 237 | 0.0951 | 0.6158 | 0.0369 | 0.0037 |
| 2.1.2 | TB | 257 | 0.0031 | 0.0129 | 0.0345 | 0.0077 |
| | SS | 375 | 0.0046 | 0.0210 | 0.0387 | 0.0031 |
| 2.1.3 | TB | 257 | 0.0042 | 0.0147 | 0.0336 | 0.0074 |
| | SS | 314 | 0.0138 | 0.0584 | 0.0389 | 0.0032 |
| 2.1.4 | TB | 259 | 0.0053 | 0.0152 | 0.0334 | 0.0065 |
| | SS | 297 | 0.0050 | 0.0215 | 0.0386 | 0.0030 |
| 3.1.1 | TB | 265 | 0.1380 | 0.9340 | 0.0306 | 0.0104 |
| | SS | 364 | 0.0995 | 0.6657 | 0.0381 | 0.0044 |
| 3.1.2 | TB | 268 | 0.0387 | 0.3842 | 0.0321 | 0.0100 |
| | SS | 253 | 0.0550 | 0.3690 | 0.0373 | 0.0045 |
| 3.1.3 | TB | 267 | 0.0737 | 0.4757 | 0.0301 | 0.0082 |
| | SS | 287 | 0.1270 | 0.6694 | 0.0331 | 0.0027 |
| 3.1.4 | TB | 270 | 0.0250 | 0.1176 | 0.0331 | 0.0093 |
| | SS | 329 | 0.0206 | 0.0736 | 0.0390 | 0.0030 |
| 4.1.1 | TB | 265 | 0.0530 | 0.3308 | 0.0275 | 0.0084 |
| | SS | 420 | 0.0391 | 0.4183 | 0.0359 | 0.0034 |
| 4.1.2 | TB | 271 | -0.0034 | 0.0193 | 0.0307 | 0.0103 |
| | SS | 361 | -0.0246 | 0.2580 | 0.0318 | 0.0029 |
| 4.1.3 | TB | 269 | -0.0007 | 0.0131 | 0.0329 | 0.0084 |
| | SS | 317 | 0.0081 | 0.0292 | 0.0378 | 0.0032 |
| 4.1.4 | TB | 268 | 0.0038 | 0.0166 | 0.0334 | 0.0082 |
| | SS | 297 | 0.0005 | 0.0194 | 0.0379 | 0.0028 |
| 5.1.1 | TB | 382 | 0.0423 | 0.5516 | 0.0366 | 0.0037 |
| | SS | 295 | 0.1289 | 0.7927 | 0.0339 | 0.0034 |
| 5.1.2 | TB | 381 | 0.0374 | 0.4960 | 0.0375 | 0.0051 |
| | SS | 381 | 0.0333 | 0.4529 | 0.0371 | 0.0044 |
| 5.1.3 | TB | 386 | 0.0204 | 0.3141 | 0.0386 | 0.0038 |
| | SS | 282 | -0.0081 | 0.0700 | 0.0354 | 0.0044 |
| 5.1.4 | TB | 386 | -0.0135 | 0.0739 | 0.0363 | 0.0046 |
| | SS | 312 | -0.0397 | 0.0910 | 0.0313 | 0.0046 |
| 6.1.1 | TB | 384 | 0.0410 | 0.4054 | 0.0380 | 0.0038 |
| | SS | 304 | 0.0082 | 0.1985 | 0.0346 | 0.0046 |
| 6.1.2 | TB | 387 | 0.0701 | 0.7283 | 0.0385 | 0.0062 |
| | SS | 313 | -0.0216 | 0.0218 | 0.0335 | 0.0040 |
| 6.1.3 | TB | 388 | 0.0016 | 0.0146 | 0.0375 | 0.0043 |
| | SS | 270 | 0.0384 | 0.4007 | 0.0390 | 0.0074 |
| 6.1.4 | TB | 385 | 0.0055 | 0.0279 | 0.0369 | 0.0044 |
| | SS | 293 | 0.0436 | 0.0635 | 0.0380 | 0.0032 |

## Claims

1. A method of melt-stabilizing ultra high molecular weight polyethylene (UHMWPE), the method comprising:
coating a solid, consolidated material comprising UHMWPE with an antioxidant, so that the antioxidant does not penetrate past a depth of 0 mm of the surface of application, to provide an antioxidant-coated solid material;
pre-irradiatively heating the antioxidant-coated solid material to diffuse the antioxidant therein, to provide an antioxidant-diffused solid material;
irradiating the antioxidant-diffused solid material with an electron beam, to provide an irradiated solid material;
post-irradiatively heating the irradiated solid material in an oxygen-containing environment to melt at least part of the UHMWPE, to provide a heated material; and
solidifying the heated material, to provide a melt-stabilized material.

2. The method of claim 1, wherein the diffusion of the antioxidant in the antioxidant-coated solid material allows the antioxidant to penetrate to a depth of at least 1 mm from the surface of the antioxidant-diffused solid material.

3. The method of claim 1, comprising cooling the antioxidant-diffused solid material prior to the irradiating.

4. The method of claim 1, wherein the pre-irradiative heating comprises heating to melt at least part of the UHMWPE.

5. The method of claim 1, wherein the pre-irradiative heating comprises preheating to equal to or greater than a preheat temperature to provide a preheated antioxidant-diffused solid material, wherein irradiating the antioxidant-diffused solid material comprises irradiating the preheated antioxidant-diffused solid material.

6. The method of claim 1, wherein after the pre-irradiative heating, further comprising preheating the antioxidant-diffused solid material equal to or greater than a preheat temperature to provide a preheated antioxidant-diffused solid material, wherein irradiating the antioxidant-diffused solid material comprises irradiating the preheated antioxidant-diffused solid material.

7. The method of claim 1, wherein the irradiating comprises irradiating with a dose rate of 0.001 mGy/h to 500 MGy/h.

8. The method of claim 1, wherein the antioxidant is at least one of a tocopherol, a tocopherol phosphite, a tocotrienol, vitamin E, vitamin E acetate, a protected vitamin E, a rosemary oil, pentaerythritol tetrakis(3-(3,5-di-tert-butyl-4-hydroxyphenyl)propionate), butanedioic acid dimethyl ester/4-hydroxy-2,2,6,6-tetramethyl-1-piperidine ethanol copolymer, tannic acid, bilberry extract, vitamin C, a carotene, a flavonoid, an isoflavonoid, a neoflavonoid, a lignin, quinine, ubiquinone, vitamin K1, a metal, glutathione, propyl gallate, octyl gallate, lauryl gallate, resveratrol, rosmarinic acid, rutin, 5-aminosalicylic acid, butylated hydroxy anisole, butylated hydroxy toluene, a phenolic compound, and a monomeric or polymeric hindered amine stabilizer.

9. The method of claim 1, wherein the antioxidant is a hindered amine stabilizer or a hindered phenol stabilizer.

10. The method of claim 9, wherein the antioxidant is a hindered amine stabilizer and the hindered amine stabilizer is at least one of a 2,2,6,6-tetra((C₁-C₅₀)hydrocarbyl)-4-piperidyl diester of HOC(O)-(C₁-C₅₀)hydrocarbyl-C(O)OH, a 2,2,6,6-tetramethyl-4-piperidyl diester of HOC(O)-(C₁-C₅₀)hydrocarbyl-C(O)OH, bis(2,2,6,6-tetramethyl-4-piperidyl) sebacate, 1,2,2,6,6-penta((C₁-C₅₀)hydrocarbyl)-4-piperidyl diester of HOC(O)-(C₁-C₅₀)hydrocarbyl-C(O)OH, a 1,2,2,6,6-pentamethyl-4-piperidyl diester of HOC(O)-(C₁-C₅₀)hydrocarbyl-C(O)OH, 2,2,6,6-tetramethylpiperidine, wherein each (C₁-C₅₀)hydrocarbyl is independently selected, is substituted or unsubstituted, and is interrupted by 0, 1, 2, or 3 -O- groups.

11. The method of claim 1, wherein the antioxidant is a protected tocopherol or tocotrienol having the structure: 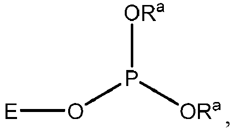 or a salt thereof, or 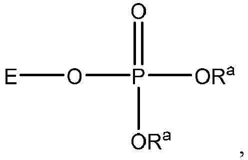 or a salt thereof,
wherein
at each occurrence, R^{a} is independently chosen from -H, -E, and substituted or unsubstituted (Ci-Cio)hydrocarbyl,
E has the structure: 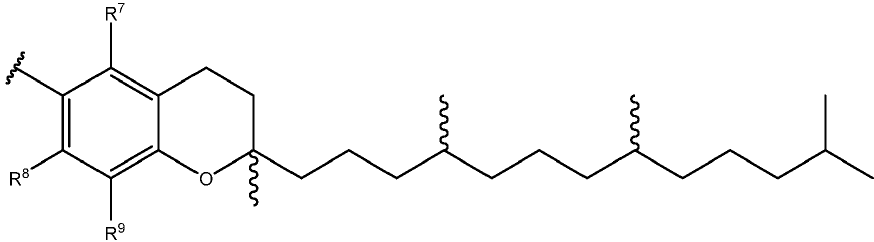 or 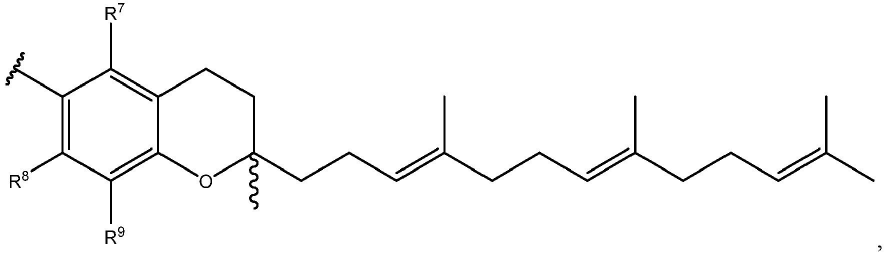 and
R⁷, R⁸, and R⁹ are each independently chosen from -H, substituted or unsubstituted (Ci-Cio)alkyl, and substituted or unsubstituted (Ci-Cio)alkenyl.

12. The method of claim 1, wherein the antioxidant is a hindered amine stabilizer-protected tocopherol or tocotrienol of formula (I): 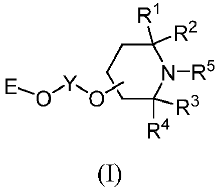 or a salt thereof,
wherein
R¹, R², R³, and R⁴ are each, independently, hydrogen or (Ci-Cio)alkyl, R⁵ is chosen from hydrogen, (Ci-Cio)alkyl, -O·, and -OR¹¹ wherein R¹¹ is hydrogen or (Ci-Cio)alkyl,
E has the structure: 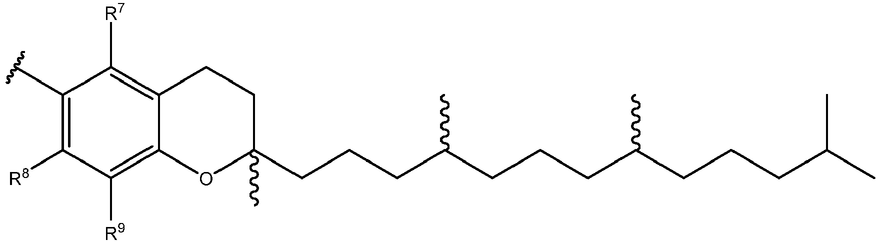 or 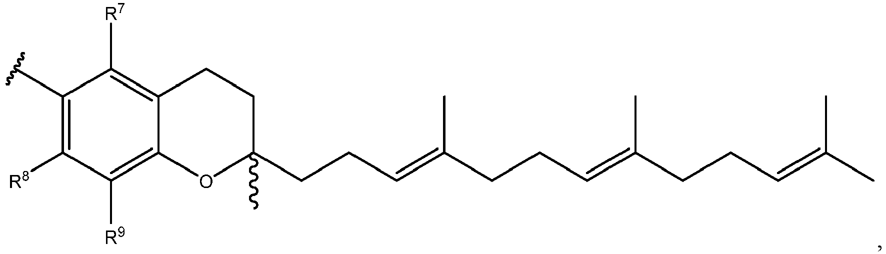 wherein R⁷, R⁸, and R⁹ are each independently chosen from -H, substituted or unsubstituted (Ci-Cio)alkyl, and substituted or unsubstituted (Ci-Cio)alkenyl, and
Y represents the group: 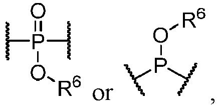 wherein R⁶ is hydrogen, (Ci-Cio)alkyl, -E, or a radical of the formula:

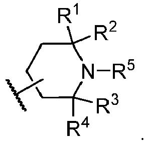

13. A method of melt-stabilizing ultra high molecular weight polyethylene (UHMWPE), the method comprising:
coating a solid, consolidated material comprising UHMWPE with a protected vitamin E antioxidant so that the antioxidant does not penetrate past a depth of 0mm of the surface of application, to provide an antioxidant-coated solid material, wherein the protected vitamin E antioxidant is at least one of at least one of a protected tocopherol or tocotrienol having the structure: 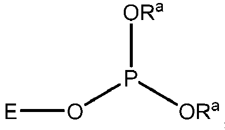 , or a salt thereof, or 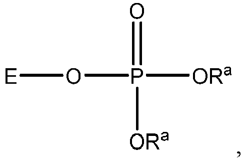 or a salt thereof,
wherein
at each occurrence, R^{a} is independently chosen from -H, -E, and substituted or unsubstituted (Ci-Cio)hydrocarbyl,
E has the structure: 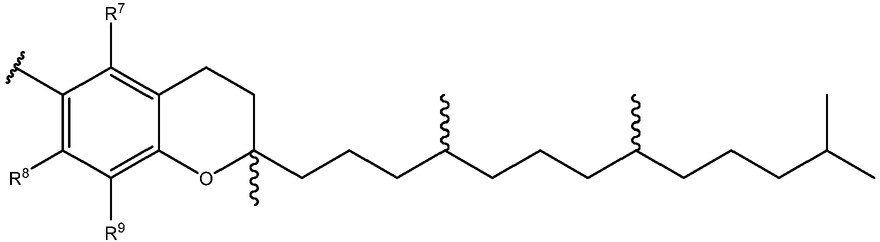 or 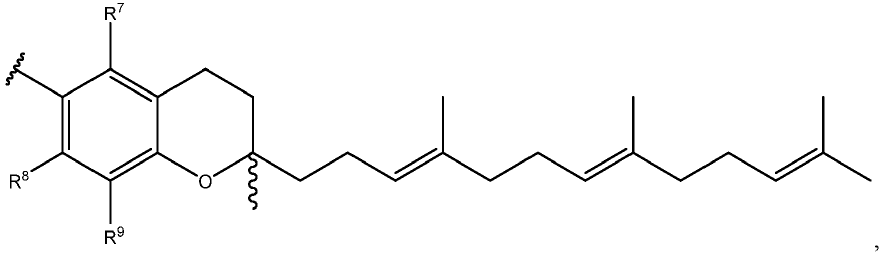 and
R⁷, R⁸, and R⁹ are each independently chosen from -H, substituted or unsubstituted (Ci-Cio)alkyl, and substituted or unsubstituted (Ci-Cio)alkenyl, and
a hindered amine stabilizer-protected tocopherol or tocotrienol of formula (I): 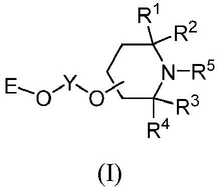 or a salt thereof,
wherein
R¹, R², R³, and R⁴ are each, independently, hydrogen or (Ci-Cio)alkyl,
R⁵ is chosen from hydrogen, (Ci-Cio)alkyl, -O·, and -OR¹¹ wherein R¹¹ is hydrogen or (Ci-Cio)alkyl, and
Y represents the group: 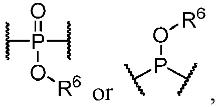 wherein R⁶ is hydrogen, (Ci-Cio)alkyl, -E, or a radical of the formula: 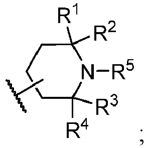
pre-irradiatively heating the antioxidant-coated solid material to diffuse the antioxidant therein, to provide an antioxidant-diffused solid material;
irradiating the antioxidant-diffused solid material with an electron beam, to provide an irradiated solid material;
post-irradiatively heating the irradiated solid material in an oxygen-containing environment to melt at least part of the UHMWPE, to provide a heated material; and
solidifying the heated material, to provide a melt-stabilized material.

## Patentansprüche

1. Verfahren zum Schmelzstabilisieren von ultrahochmolekularem Polyethylen (UHMWPE), wobei das Verfahren Folgendes umfasst:
Beschichten eines festen, verdichteten Materials, das UHMWPE umfasst, mit einem Antioxidans, sodass das Antioxidans nicht über eine Tiefe von 0 mm der Anwendungsoberfläche hinaus eindringt, um ein mit Antioxidans beschichtetes festes Material bereitzustellen;
Vorbestrahlungserwärmen des mit Antioxidans beschichteten festen Materials, um das Antioxidans darin zu diffundieren, um ein festes Material mit diffundiertem Antioxidans bereitzustellen;
Bestrahlen des festen Materials mit diffundiertem Antioxidans mit einem Elektronenstrahl, um ein bestrahltes festes Material bereitzustellen;
Nachbestrahlenserwärmen des bestrahlten festen Materials in einer sauerstoffhaltigen Umgebung, um mindestens einen Teil des UHMWPE zu schmelzen, um ein erwärmtes Material bereitzustellen; und
Verfestigen des erwärmten Materials, um ein schmelzstabilisiertes Material bereitzustellen.

2. Verfahren nach Anspruch 1, wobei es die Diffusion des Antioxidans in dem mit Antioxidans beschichteten festen Material dem Antioxidans ermöglicht, bis zu einer Tiefe von mindestens 1 mm von der Oberfläche des festen Materials mit diffundiertem Antioxidans einzudringen.

3. Verfahren nach Anspruch 1, umfassend Kühlen des festen Materials mit diffundiertem Antioxidans vor dem Bestrahlen.

4. Verfahren nach Anspruch 1, wobei das Vorbestrahlungserwärmen Erwärmen, um mindestens einen Teil des UHMWPE zu schmelzen, umfasst.

5. Verfahren nach Anspruch 1, wobei das Vorbestrahlungserwärmen Vorwärmen auf eine Vorwärmtemperatur oder mehr umfasst, um ein vorgewärmtes festes Material mit diffundiertem Antioxidans bereitzustellen, wobei das Bestrahlen des festen Materials mit diffundiertem Antioxidans Bestrahlen des vorgewärmten festen Materials mit diffundiertem Antioxidans umfasst.

6. Verfahren nach Anspruch 1, wobei nach dem Vorbestrahlungserwärmen ferner umfassend Vorwärmen des festen Materials mit diffundiertem Antioxidans auf eine Vorwärmtemperatur oder mehr, um ein vorgewärmtes festes Material mit diffundiertem Antioxidans bereitzustellen, wobei das Bestrahlen des festen Materials mit diffundiertem Antioxidans Bestrahlen des vorgewärmten festen Materials mit diffundiertem Antioxidans umfasst.

7. Verfahren nach Anspruch 1, wobei das Bestrahlen Bestrahlen mit einer Dosisrate von 0,001 mGy/h bis 500 MGy/h umfasst.

8. Verfahren nach Anspruch 1, wobei das Antioxidans mindestens eines von einem Tocopherol, einem Tocopherolphosphit, einem Tocotrienol, Vitamin E, Vitamin-E-Acetat, einem geschützten Vitamin E, einem Rosmarinöl, Pentaerythritoltetrakis(3-(3,5-di-tert-butyl-4-hydroxyphenyl)propionat), Butandisäure-Dimethylester/4-Hydroxy-2,2,6,6-tetramethyl-1-piperidin-Ethanol-Copolymer, Gerbsäure, Heidelbeerextrakt, Vitamin C, einem Carotin, einem Flavonoid, einem Isoflavonoid, einem Neoflavonoid, einem Lignin, Chinin, Ubichinon, Vitamin K1, einem Metall, Glutathion, Propylgallat, Octylgallat, Laurylgallat, Resveratrol, Rosmarinsäure, Rutin, 5-Aminosalicylsäure, butyliertem Hydroxyanisol, butyliertem Hydroxytoluol, einer Phenolverbindung und einem Hindered-Amine-Monomer- oder Polymerstabilisator ist.

9. Verfahren nach Anspruch 1, wobei das Antioxidans ein Hindered-Amine-Stabilisator oder Hindered-Phenol-Stabilisator ist.

10. Verfahren nach Anspruch 9, wobei das Antioxidans ein Hindered-Amine-Stabilisator ist und der Hindered-Amine-Stabilisator mindestens eines von einem 2,2,6,6-Tetra((C₁-C₅₀)hydrocarbyl)-4-piperidyldiester von HOC(O)-(C₁-C₅₀)hydrocarbyl-C(O)OH, einem 2,2,6,6-Tetramethyl-4-piperidyldiester von HOC(O)-(C₁-C₅₀)hydrocarbyl-C(O)OH, Bis(2,2,6,6-tetramethyl-4-piperidyl)sebacat, 1,2,2,6,6-Penta((C₁-C₅₀)hydrocarbyl)-4-piperidyldiester von HOC(O)-(C₁-C₅₀)hydrocarbyl-C(O)OH, einem 1,2,2,6,6-Pentamethyl-4-piperidyldiester von HOC(O)-(C₁-C₅₀)hydrocarbyl-C(O)OH, 2,2,6,6-Tetramethylpiperidin ist, wobei jedes (C₁-C₅₀)-Hydrocarbyl unabhängig ausgewählt ist, substituiert oder unsubstituiert ist und durch 0, 1, 2 oder 3 -O-Gruppen unterbrochen ist.

11. Verfahren nach Anspruch 1, wobei das Antioxidans ein geschütztes Tocopherol oder Tocotrienol ist, das die folgende Struktur aufweist: 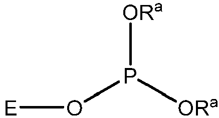 oder ein Salz davon oder 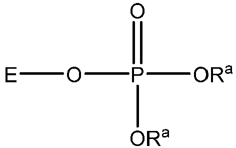 oder ein Salz davon,
wobei
bei jedem Vorkommen R^{a} unabhängig aus -H, -E und substituiertem oder unsubstituiertem (C₁-C₁₀)-Hydrocarbyl gewählt ist,
E die folgende Struktur aufweist: 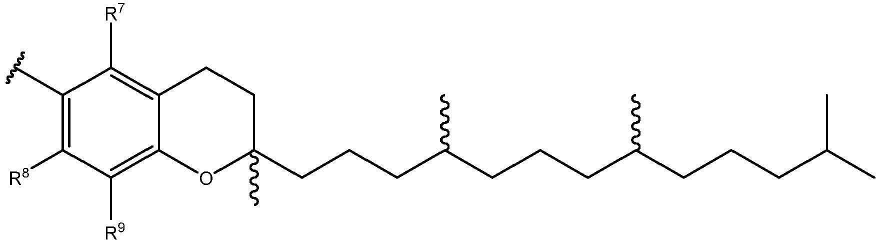 oder 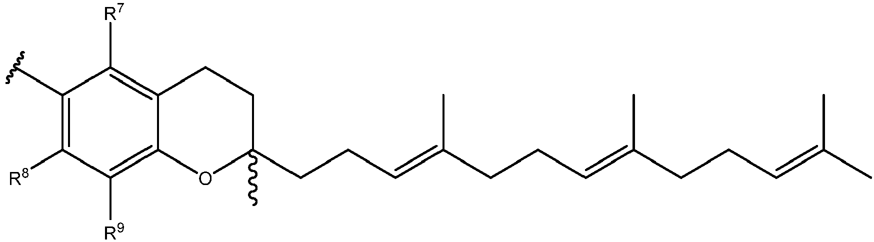 und
R⁷, R⁸ und R⁹ jeweils unabhängig aus -H, substituiertem oder unsubstituiertem (C₁-C₁₀)-Alkyl und substituiertem oder unsubstituiertem(C₁-C₁₀)-Alkenyl gewählt sind.

12. Verfahren nach Anspruch 1, wobei das Antioxidans ein durch einen Hindered-Amine-Stabilisator geschütztes Tocopherol oder Tocotrienol der folgenden Formel (I) ist: 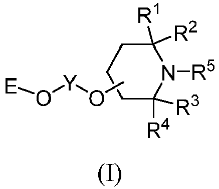
oder ein Salz davon,
wobei
R¹, R², R³ und R⁴ jeweils unabhängig Wasserstoff oder (C₁-C₁₀)-Alkyl sind,
R⁵ aus Wasserstoff, (C₁-C₁₀)-Alkyl, -O· und -OR¹¹ gewählt ist, wobei R¹¹ Wasserstoff oder (C₁-C₁₀)-Alkyl ist,
E die folgende Struktur aufweist: 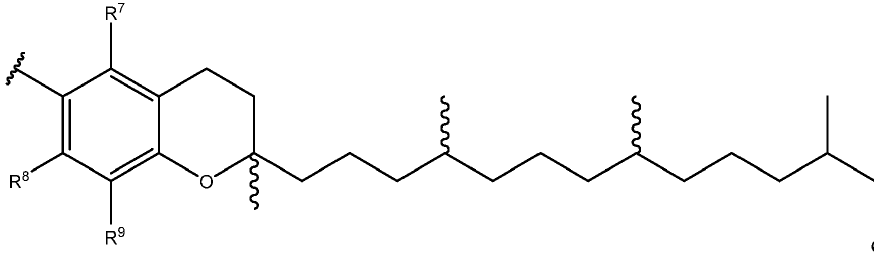 oder 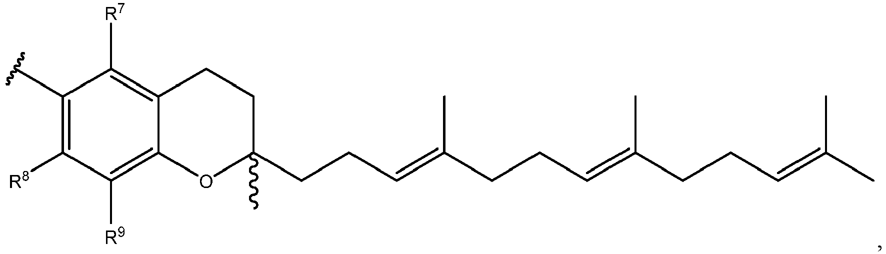
wobei R⁷, R⁸ und R⁹ jeweils unabhängig aus -H, substituiertem oder unsubstituiertem (C₁-C₁₀)-Alkyl und substituiertem oder unsubstituiertem (C₁-C₁₀)-Alkenyl gewählt sind, und
Y die folgende Gruppe darstellt: 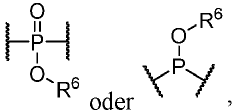
wobei R⁶ Wasserstoff, (C₁-C₁₀)Alkyl, -E oder ein Radikal der folgenden Formel ist:

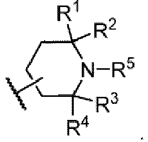

13. Verfahren zum Schmelzstabilisieren von ultrahochmolekularem Polyethylen (UHMWPE), wobei das Verfahren Folgendes umfasst:
Beschichten eines festen, verdichteten Materials, das UHMWPE umfasst, mit einem geschützten Vitamin-E-Antioxidans, sodass das Antioxidans nicht über eine Tiefe von 0 mm der Anwendungsoberfläche hinaus eindringt, um ein mit Antioxidans beschichtetes festes Material bereitzustellen, wobei das geschützte Vitamin-E-Antioxidans mindestens eines von mindestens einem von einem geschützten Tocopherol oder Tocotrienol, das die folgende Struktur aufweist: 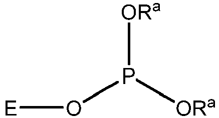 oder ein Salz davon oder 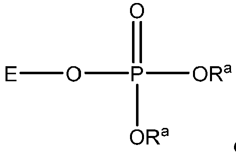 oder ein Salz davon,
wobei
bei jedem Vorkommen R^{a} unabhängig aus -H, -E und substituiertem oder unsubstituiertem (C₁-C₁₀)-Hydrocarbyl gewählt ist,
E die folgende Struktur aufweist: 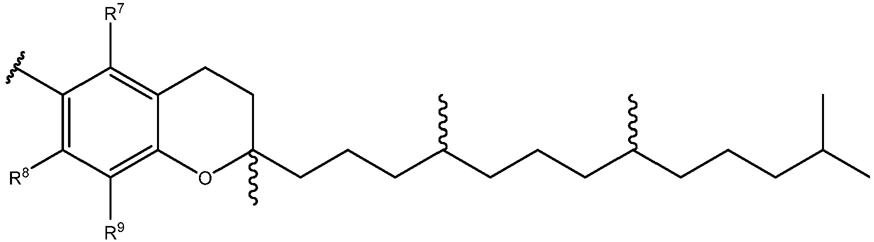 oder 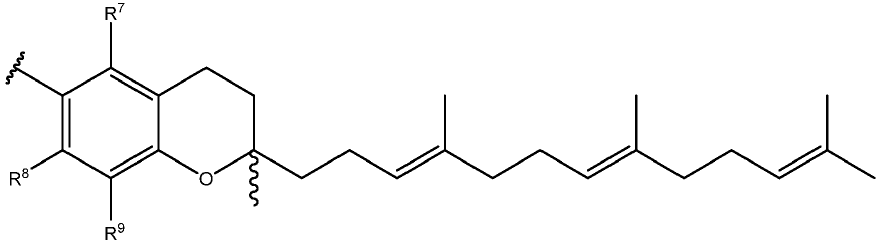 und
R⁷, R⁸ und R⁹ jeweils unabhängig aus -H, substituiertem oder unsubstituiertem (C₁-C₁₀)-Alkyl und substituiertem oder unsubstituiertem(C₁-C₁₀)-Alkenyl gewählt sind, und
einem durch einen Hindered-Amine-Stabilisator geschützten Tocopherol oder Tocotrienol der folgenden Formel (I) ist: 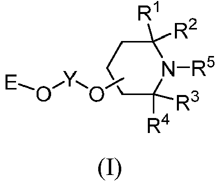
oder ein Salz davon,
wobei
R¹, R², R³ und R⁴ jeweils unabhängig Wasserstoff oder (C₁-C₁₀)-Alkyl sind,
R⁵ aus Wasserstoff, (C₁-C₁₀)-Alkyl, -O- und -OR¹¹ gewählt ist, wobei R¹¹ Wasserstoff oder (C₁-C₁₀)-Alkyl ist, und
Y die folgende Gruppe darstellt: 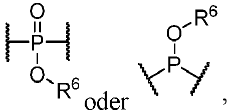
wobei R⁶ Wasserstoff, (C₁-C₁₀)Alkyl, -E oder ein Radikal der folgenden Formel ist: 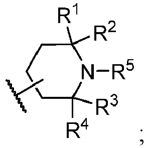
Vorbestrahlungserwärmen des mit Antioxidans beschichteten festen Materials, um das Antioxidans darin zu diffundieren, um ein festes Material mit diffundiertem Antioxidans bereitzustellen;
Bestrahlen des festen Materials mit diffundiertem Antioxidans mit einem Elektronenstrahl, um ein bestrahltes festes Material bereitzustellen;
Nachbestrahlenserwärmen des bestrahlten festen Materials in einer sauerstoffhaltigen Umgebung, um mindestens einen Teil des UHMWPE zu schmelzen, um ein erwärmtes Material bereitzustellen; und
Verfestigen des erwärmten Materials, um ein schmelzstabilisiertes Material bereitzustellen.

## Revendications

1. Procédé de stabilisation à l'état fondu d'un polyéthylène à ultra haut poids moléculaire (PEUHPM), le procédé comprenant :
l'enduction d'un matériau consolidé solide comprenant un PEUHPM avec un antioxydant, de sorte que l'antioxydant ne pénètre pas au-delà d'une profondeur de 0 mm de la surface d'application, de manière à fournir un matériau solide enduit d'antioxydant ;
le préchauffage par irradiation du matériau solide enduit d'antioxydant pour diffuser l'antioxydant en son sein, de manière à fournir un matériau solide ayant subi une diffusion d'antioxydant ;
l'irradiation du matériau solide ayant subi une diffusion d'antioxydant avec un faisceau d'électrons, de manière à fournir un matériau solide irradié ;
l'après-chauffage par irradiation du matériau solide irradié dans un environnement contenant de l'oxygène pour fondre au moins une partie du PEUHPM, de manière à fournir un matériau chauffé ; et
la solidification du matériau chauffé, de manière à fournir un matériau stabilisé à l'état fondu.

2. Procédé selon la revendication 1, ladite diffusion de l'antioxydant dans le matériau solide enduit d'antioxydant permettant la pénétration de l'antioxydant jusqu'à une profondeur d'au moins 1 mm de la surface du matériau solide ayant subi une diffusion d'antioxydant.

3. Procédé selon la revendication 1, comprenant le refroidissement du matériau solide ayant subi une diffusion d'antioxydant avant l'irradiation.

4. Procédé selon la revendication 1, ledit préchauffage par irradiation comprenant le chauffage jusqu'à fondre d'au moins une partie du PEUHPM.

5. Procédé selon la revendication 1, ledit préchauffage par irradiation comprenant le préchauffage jusqu'à une température supérieure ou égale à une température de préchauffage de manière à fournir un matériau solide ayant subi une diffusion d'antioxydant préchauffé, ladite irradiation du matériau solide ayant subi une diffusion d'antioxydant comprenant l'irradiation du matériau solide ayant subi une diffusion d'antioxydant préchauffé.

6. Procédé selon la revendication 1, après ledit préchauffage par irradiation, comprenant en outre le préchauffage du matériau solide ayant subi une diffusion d'antioxydant jusqu'à une température supérieure ou égale à une température de préchauffage de manière à fournir un matériau solide ayant subi une diffusion d'antioxydant préchauffé, ladite irradiation du matériau solide ayant subi une diffusion d'antioxydant comprenant l'irradiation du matériau solide ayant subi une diffusion d'antioxydant préchauffé.

7. Procédé selon la revendication 1, ladite irradiation comprenant l'irradiation avec un débit de dose de 0,001 mGy/h à 500 MGy/h.

8. Procédé selon la revendication 1, ledit antioxydant étant au moins l'un parmi un tocophérol, un phosphite de tocophérol, un tocotriénol, la vitamine E, l'acétate de vitamine E, une vitamine E protégée, une huile de romarin, le pentaérythritol tétrakis(3-(3,5-di-tert-butyl-4-hydroxyphényl)propionate), le copolymère ester de diméthyle de l'acide butanedioïque/4-hydroxy-2,2,6,6-tétraméthyl-1-pipéridine éthanol, l'acide tannique, un extrait de myrtille, la vitamine C, un carotène, un flavonoïde, un isoflavonoïde, un néoflavonoïde, une lignine, la quinine, l'ubiquinone, la vitamine K1, un métal, le glutathion, le gallate de propyle, le gallate d'octyle, le gallate de lauryle, le resvératrol, l'acide rosmarinique, la rutine, l'acide 5-aminosalicylique, l'hydroxyanisole butylé, l'hydroxytoluène butylé, un composé phénolique, et un stabilisant d'amine monomère ou polymère encombrée.

9. Procédé selon la revendication 1, ledit antioxydant étant un stabilisant d'amine encombrée ou un stabilisant de phénol encombré.

10. Procédé selon la revendication 9, ledit antioxydant étant un stabilisant d'amine encombrée et ledit stabilisant d'amine encombrée étant au moins l'un parmi un diester de 2,2,6,6-tétra((C₁-C₅₀)hydrocarbyl)-4-pipéridyle de HOC(O)-(C₁-C₅₀)hydrocarbyl-C(O)OH, un diester de 2,2,6,6-tétraméthyl-4-pipéridyle de HOC(O)-(C₁-C₅₀)hydrocarbyl-C(O)OH, le sébacate de bis(2,2,6,6-tétraméthyl-4-pipéridyle), un diester de 1,2,2,6,6-penta((C₁-C₅₀)hydrocarbyl)-4-pipéridyle de HOC(O)-(C₁-C₅₀)hydrocarbyl-C(O)OH, un diester de l,2,2,6,6-pentaméthyl-4-pipéridyle de HOC(O)-(C₁-C₅₀)hydrocarbyl-C(O)OH, la 2,2,6,6-tétraméthylpipéridine, chaque groupe (C₁-Cso)hydrocarbyle étant indépendamment choisi, étant substitué ou non substitué, et étant interrompu par 0, 1, 2 ou 3 groupes -O-.

11. Procédé selon la revendication 1, ledit antioxydant étant un tocophérol or tocotriénol protégé comportant la structure : 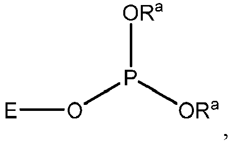 ou un sel de celui-ci, ou 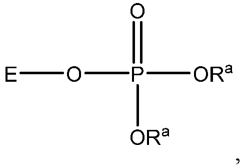 ou un sel de celui-ci,
dans laquelle
à chaque occurrence, R^{a} est indépendamment choisi parmi les groupes -H, -E et (C₁-C₁₀)hydrocarbyle substitué ou non substitué,
E comporte la structure : 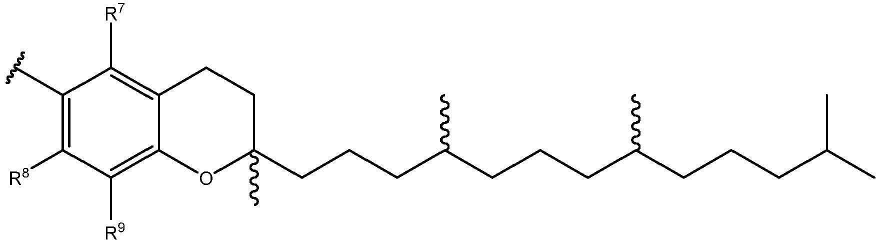 ou 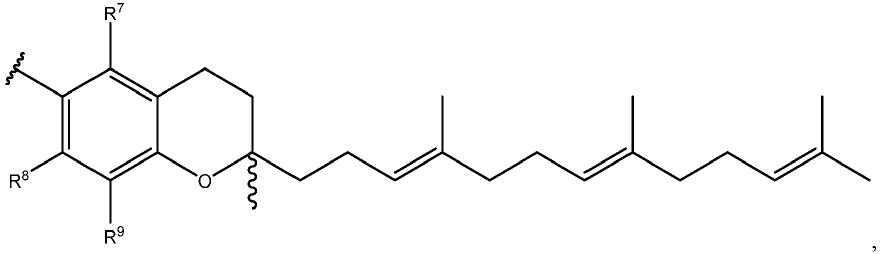 et
R⁷, R⁸, et R⁹ sont chacun indépendamment choisis parmi les groupes -H, (C₁-C₁₀)alkyle substitué ou non substitué, et (C₁-C₁₀)alcényle substitué ou non substitué.

12. Procédé selon la revendication 1, ledit antioxydant étant un tocophérol ou tocotriénol protégé par stabilisant d'amine encombrée de formule (I) : 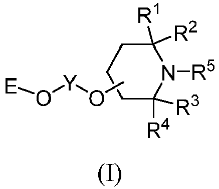
ou un sel de celui-ci ;
dans laquelle
R¹, R², R³ et R⁴ représentent chacun, indépendamment, un atome d'hydrogène ou un groupe (C₁-C₁₀)alkyle,
R⁵ est choisi parmi un atome d'hydrogène, les groupes (C₁-C₁₀)alkyle,-O· et -OR¹¹ dans lequel R¹¹ représente un atome d'hydrogène ou un groupe (C₁-C₁₀)alkyle,
E comporte la structure : 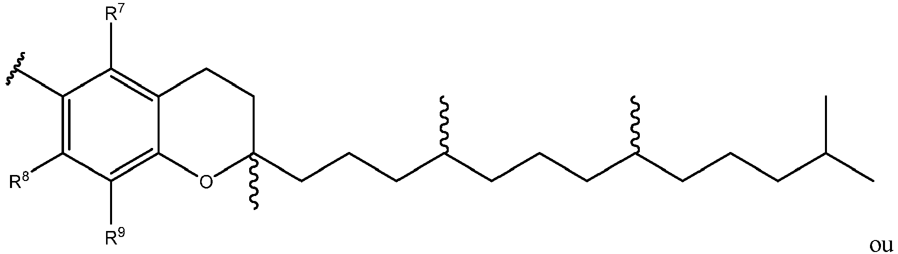 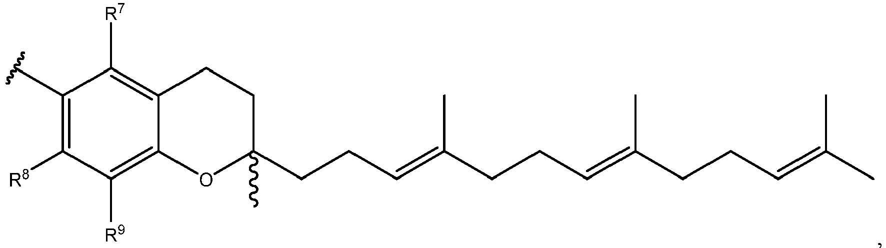
dans laquelle R⁷, R⁸ et R⁹ sont chacun indépendamment choisis parmi les groupes -H, (C₁-C₁₀)alkyle substitué ou non substitué, et (C₁-C₁₀)alcényle substitué ou non substitué, et
Y représente le groupe : 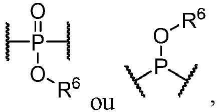
dans lequel R⁶ représente un atome d'hydrogène, un groupe (C₁-C₁₀)alkyle,-E, ou un radical de la formule :

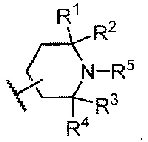

13. Procédé de stabilisation à l'état fondu d'un polyéthylène à ultra haut poids moléculaire (PEUHPM), le procédé comprenant :
l'enduction d'un matériau consolidé solide comprenant un PEUHPM avec un antioxydant de vitamine E protégée de sorte que l'antioxydant ne pénètre pas au-delà d'une profondeur de 0 mm de la surface d'application, de manière à fournir un matériau solide enduit d'antioxydant, ledit antioxydant de vitamine E protégée étant au moins l'un parmi au moins un tocophérol ou tocotriénol protégé comportant la structure : 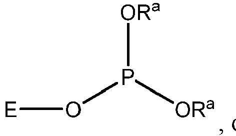 ou un sel de celui-ci, ou 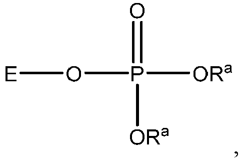 ou un sel de celui-ci,
dans laquelle
à chaque occurrence, R^{a} est indépendamment choisi parmi les groupes -H, -E et (C₁-C₁₀)hydrocarbyle substitué ou non substitué,
E comporte la structure : 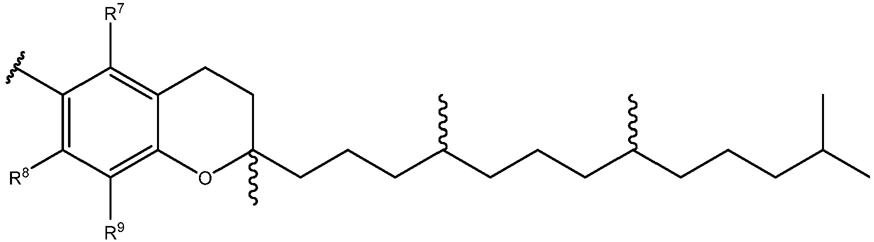 ou 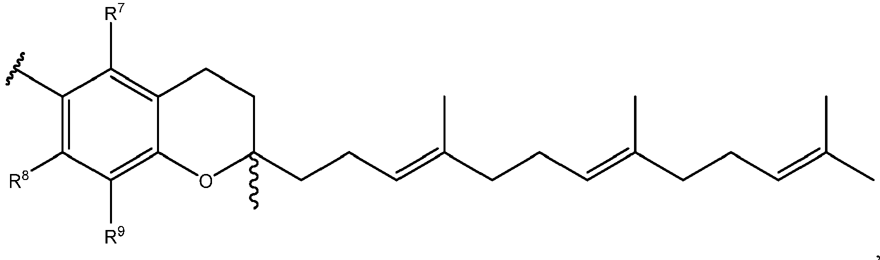 et
R⁷, R⁸ et R⁹ sont chacun indépendamment choisis parmi les groupes -H, (C₁-C₁₀)alkyle substitué ou non substitué, et (C₁-C₁₀)alcényle substitué ou non substitué, et
un tocophérol ou tocotriénol protégé par stabilisant d'amine encombrée de formule (I) : 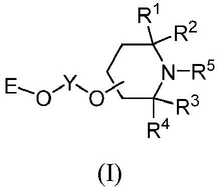
ou un sel de celui-ci ;
dans laquelle
R¹, R², R³ et R⁴ représentent chacun, indépendamment, un atome d'hydrogène ou un groupe (C₁-C₁₀)alkyle,
R⁵ est choisi parmi un atome d'hydrogène, les groupes (C₁-C₁₀)alkyle,-O· et -OR¹¹ dans lequel R¹¹ représente un atome d'hydrogène ou un groupe (C₁-C₁₀)alkyle,et
Y représente le groupe : 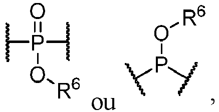
dans lequel R⁶ représente un atome d'hydrogène, un groupe (C₁-C₁₀)alkyle,-E, ou un radical de la formule : 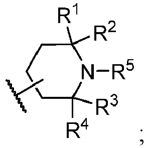
le préchauffage par irradiation du matériau solide enduit d'antioxydant pour diffuser l'antioxydant en son sein, de manière à fournir un matériau solide ayant subi une diffusion d'antioxydant ;
l'irradiation du matériau solide ayant subi une diffusion d'antioxydant avec un faisceau d'électrons, de manière à fournir un matériau solide irradié ;
l'après-chauffage par irradiation du matériau solide irradié dans un environnement contenant de l'oxygène pour fondre au moins une partie du PEUHPM, de manière à fournir un matériau chauffé ; et
la solidification du matériau chauffé, de manière à fournir un matériau stabilisé à l'état fondu.
